# EUROPEAN PATENT SPECIFICATION

(11) **EP 3 356 826 B1**
(45) Date of publication and mention of the grant of the patent: **06.12.2023**
(21) Application number: 16778307.5
(22) Date of filing: 30.09.2016
(51) Int. Cl.: G01N 33/574

(54) **GDF-15 AS A DIAGNOSTIC MARKER FOR MELANOMA**
GDF-15 ALS DIAGNOSTISCHER MARKER FÜR MELANOME
GDF-15 COMME MARQUEUR DIAGNOSTIQUE DU MÉLANOME

(30) Priority: 02.10.2015 GB 201517528
(43) Date of publication of application: 08.08.2018
(73) Proprietor: Julius-Maximilians-Universität Würzburg, 97070 Würzburg (DE)
(72) Inventor: WISCHHUSEN, Jörg, 97082 Würzburg (DE); SCHÄFER, Tina, 97204 Höchberg (DE); WEIDE, Benjamin, 72074 Tübingen (DE)
(74) Representative: Hoffmann Eitle
(86) International application number: PCT/EP2016/073521
(87) International publication number: WO 2017/055614

(56) References cited:
- EP-A1- 2 694 973
- WO-A1-2014/049087
- WO-A1-2015/144855
- GLEN M. BOYLE ET AL: "Macrophage Inhibitory Cytokine-1 Is Overexpressed in Malignant Melanoma and Is Associated with Tumorigenicity", JOURNAL OF INVESTIGATIVE DERMATOLOGY, vol. 129, no. 2, 28 August 2008 (2008-08-28), pages 383-391, XP055330204, US ISSN: 0022-202X, DOI: 10.1038/jid.2008.270
- B WEIDE ET AL: "Serum markers lactate dehydrogenase and S100B predict independently disease outcome in melanoma patients with distant metastasis", BRITISH JOURNAL OF CANCER, vol. 107, no. 3, 10 July 2012 (2012-07-10) , pages 422-428, XP055330215, GB ISSN: 0007-0920, DOI: 10.1038/bjc.2012.306
- KONSTANTINOS G. LASITHIOTAKIS ET AL: "The incidence and mortality of cutaneous melanoma in southern Germany", CANCER., vol. 107, no. 6, 1 January 2006 (2006-01-01), pages 1331-1339, XP055330221, US ISSN: 0008-543X, DOI: 10.1002/cncr.22126
- SUNG JIN HUH ET AL: "Tumorigenesis and Neoplastic Progression Macrophage Inhibitory Cytokine-1 Regulates Melanoma Vascular Development", AM J PATHOL, vol. 176, 1 June 2010 (2010-06-01), pages 2948-2957, XP055330224,
- MURIELLE MIMEAULT ET AL: "Divergent molecular mechanisms underlying the pleiotropic functions of macrophage inhibitory cytokine-1 in cancer", JOURNAL OF CELLULAR PHYSIOLOGY, vol. 224, no. 3, 12 May 2010 (2010-05-12), pages 626-635, XP055330227, US ISSN: 0021-9541, DOI: 10.1002/jcp.22196
- ANDREAS MAUERER ET AL: "Identification of new genes associated with melanoma", EXPERIMENTAL DERMATOLOGY, vol. 20, no. 6, 16 March 2011 (2011-03-16) , pages 502-507, XP055122228, ISSN: 0906-6705, DOI: 10.1111/j.1600-0625.2011.01254.x
- J. CORRE ET AL: "Concise Review: Growth Differentiation Factor 15 in Pathology: A Clinical Role?", STEM CELLS TRANSLATIONAL MEDICINE : SCTM, vol. 2, no. 12, 1 December 2013 (2013-12-01), pages 946-952, XP055330730, Durham ISSN: 2157-6564, DOI: 10.5966/sctm.2013-0055
- ZHANG L ET AL: "Expression of growth differentiation factor 15 is positively correlated with histopathological malignant grade and in vitro cell proliferation in oral squamous cell carcinoma", ORAL ONCOLOGY, ELSEVIER SCIENCE, OXFORD, GB, vol. 45, no. 7, 1 July 2009 (2009-07-01), pages 627-632, XP026285229, ISSN: 1368-8375, DOI: 10.1016/J.ORALONCOLOGY.2008.07.017 [retrieved on 2008-09-19]
- ANNETTE PFLUGFELDER ET AL: "Malignant Melanoma S3-Guideline "Diagnosis, Therapy and Follow-up of Melanoma"", JDDG. JOURNAL DER DEUTSCHEN DERMATOLOGISCHEN GESELLSCHAFT, vol. 11, no. s6, 1 August 2013 (2013-08-01), pages 1-116, XP055330231, DE ISSN: 1610-0379, DOI: 10.1111/ddg.12113_suppl
- MATTHEW N. SIROTT ET AL: "Prognostic factors in patients with metastatic malignant melanoma: A multivariate analysis", CANCER., vol. 72, no. 10, 15 November 1993 (1993-11-15), pages 3091-3098, XP055330232, US ISSN: 0008-543X, DOI: 10.1002/1097-0142(19931115)72:10<3091::AID -CNCR2820721034>3.0.CO;2-V
- SARAH L. LAKE ET AL: "Comparison of Formalin-Fixed and Snap-Frozen Samples Analyzed by Multiplex Ligation-Dependent Probe Amplification for Prognostic Testing in Uveal Melanoma", INVESTIGATIVE OPTHALMOLOGY & VISUAL SCIENCE, vol. 53, no. 6, 4 May 2012 (2012-05-04), page 2647, XP055296795, US ISSN: 1552-5783, DOI: 10.1167/iovs.12-9584
- Hargita Hegyesi ET AL: "Validation of Growth Differentiation Factor (GDF-15) as a Radiation Response Gene and Radiosensitizing Target in Mammary Adenocarcinoma Model", , 1 January 2012 (2012-01-01), XP055313890, Retrieved from the Internet: URL:http://cdn.intechopen.com/pdfs/24965.p df [retrieved on 2016-10-25]
- DE WIT N J W ET AL: "Analysis of differential gene expression in human melanocytic tumour lesions by custom made oligonucleotide arrays", BRITISH JOURNAL OF CANCER, NATURE PUBLISHING GROUP, GB, vol. 92, no. 12, 1 June 2005 (2005-06-01), pages 2249-2261, XP008121830, ISSN: 0007-0920, DOI: 10.1038/SJ.BJC.6602612 [retrieved on 2005-06-14]
- M MIMEAULT ET AL: "Marked improvement of cytotoxic effects induced by docetaxel on highly metastatic and androgen-independent prostate cancer cells by downregulating macrophage inhibitory cytokine-1", BRITISH JOURNAL OF CANCER, vol. 108, no. 5, 28 February 2013 (2013-02-28), pages 1079-1091, XP055330293, GB ISSN: 0007-0920, DOI: 10.1038/bjc.2012.484
- DANIELA SUESSKIND ET AL: "GDF-15: a novel serum marker for metastases in uveal melanoma patients", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 250, no. 6, 1 September 2011 (2011-09-01), pages 887-895, XP035058124, ISSN: 1435-702X, DOI: 10.1007/S00417-011-1786-6
- Betül Ünal ET AL: "The divergent roles of growth differentiation factor-15 (GDF-15) in benign and malignant skin pathologies", ARCHIVES OF DERMATOLOGICAL RESEARCH., vol. 307, no. 7, 18 February 2015 (2015-02-18), pages 551-557, XP055677903, DE ISSN: 0340-3696, DOI: 10.1007/s00403-015-1546-2

## Description

### FIELD OF THE INVENTION

The present invention relates to methods for predicting the probability of survival of a human melanoma cancer patient as defined in the appended claims 1 to 12, and to apparatuses configured to perform said methods, and to uses of kits in these methods.

### BACKGROUND

The serum level of lactate dehydrogenase (sLDH) is the most widely used prognostic biomarker in melanoma and has been incorporated in the AJCC staging system for melanoma patients with distant metastases since 2001 (Balch, CM et al., J Clin Oncol / 19 / 3635-48. 2001). sLDH had been identified as an independent prognostic marker for patients with unresectable disease by different research groups (Sirott, MN et al., Cancer / 72 / 3091-8. 1993; Eton, O et al., J Clin Oncol /16 / 1103-11. 1998; Manola, J et al., J Clin Oncol / 18 / 3782-93. 2000). Results from a comprehensive meta-analysis based on a large pool of clinical studies (31,857 patients with various solid tumors) confirmed the consistent effect of elevated LDH on OS (HR = 1.48, 95%CI = 1.43 to 1.53) across all disease subgroups and stages, with particular relevance for metastatic tumors. While the exact mechanism underlying tumor promotion by LDH remains unknown and may also be related to hypoxia and metabolic reprogramming via a Warburg effect, LDH also reflects high tumor burden (Zhang, J., Yao, Y.-H., Li, B.-G., Yang, Q., Zhang, P.-Y., and Wang, H.-T. (2015). Prognostic value of pretreatment serum lactate dehydrogenase level in patients with solid tumors: a systematic review and meta-analysis. Scientific Reports 5, 9800). Still, there is a need for improved prognostic biomarkers for melanoma patients.

Serum concentrations of S100B (sS100B) are widely used mainly in Europe to screen patients without evidence of disease to detect recurrences early (Pflugfelder, A et al., J Dtsch Dermatol Ges / 11 Suppl 6 / 1-116, 1-26. 2013). A meta-analysis by Mocellin et al. summarized the evidence on the suitability of sS100B to predict patients' survival. Twenty-two series enrolling 3393 patients comprising all stages were included in this analysis. Serum S100B positivity was associated with significantly poorer survival in melanoma patients of all stages especially in the subgroup of stage I to III patients independent from other prognostic factors (Mocellin, S et al., Int J Cancer / 123 / 2370-6. 2008). In prior studies, it was demonstrated that sS100B and sLDH had independent impact on prognosis of patients with distant metastases and the combined analysis of both markers might be used to select patients for complete metastasectomy (Weide, B et al., PLoS One / 8 / e81624. 2013; Weide, B et al., Br J Cancer / 107 / 422-8. 2012). However, despite this large evidence, no worldwide consensus exists on its implementation in the routine clinical setting in melanoma patients.

Growth and Differentiation Factor-15 (GDF-15, also known as Macrophage Inhibitor Cytokine-1 (MIC-1), Placental TGF-β (PTGFβ), Placental Bone Morphogenetic Protein (PLAB), Nonsteroidal Anti-inflammatory Drug-Activated Gene (NAG1) or Prostate-Derived Factor (PDF) is over-expressed in tumor cells of several types of solid cancers (Mimeault, M et al., Br J Cancer / 108 / 1079-91. 2013; Bock, AJ et al., Int J Gynecol Cancer / 20 / 1448-55. 2010; Zhang, L et al., Oral Oncol / 45 / 627-32. 2009). GDF-15 is induced by a number of tumor suppressor pathways including p53, GSK-3β, and EGR-1 (Wang, X et al., Biochem Pharmacol 1851 597-606. 2013) and there is also evidence that GDF-15 itself can exert tumor suppressive effects, as shown in nude mouse xenograft models (Martinez, JM et al., J Pharmacol Exp Ther / 318 / 899-906. 2006; Eling, TE et al., J Biochem Mol Biol / 39 / 649-55. 2006) and in transgenic mice (Baek, SJ et al., Mol Pharmacol / 59 / 901-8. 2001). With regard to tumor cells both pro- and anti-apoptotic effects have been described for GDF-15 (Mimeault, M et al., Br J Cancer / 108 / 1079-91. 2013; Baek, SJ et al., Mol Pharmacol / 59 / 901-8. 2001; Zimmers, TA et al., J Cancer Res Clin Oncol / 136 / 571-6. 2010; Jones, MF et al., Cell Death Differ / 2015) and a multitude of possible signaling pathways has been suggested (Mimeault, M and Batra SK, J Cell Physiol / 224 / 626-35. 2010). Further complexity was added recently when the unprocessed pro-protein was shown to go into the nucleus where it altered TGF-beta dependent SMAD signaling and thereby transcription patterns (Min, KW et al., Oncogene / 2015). In vivo, constitutive GDF-15 overexpression reduced tumor formation but increased metastasis in an animal model for prostate cancer (Husaini, Y et al., PLoS One / 7 / e43833. 2012). GDF-15 was further shown to induce cancer cachexia (Johnen, H et al., Nat Med / 13 / 1333-40. 2007). Similarly, patent applications WO 2005/099746 and WO 2009/021293 relate to an anti-human-GDF-15 antibody (Mab26) capable of antagonizing effects of human GDF-15 (hGDF-15) on tumor-induced weight loss *in vivo* in mice. WO 2014/049087 and PCT/EP2015/056654 relate to monoclonal antibodies to hGDF-15 and medical uses thereof.

Clinically, a high GDF-15 serum level (sGDF-15) was found to correlate with the presence of bone metastases and poor prognosis in prostate cancer (Selander, KS et al., Cancer Epidemiol Biomarkers Prev / 16 / 532-7. 2007). In colorectal cancer, patients with high plasma levels showed shorter time to recurrence and reduced overall survival (Wallin, U et al., Br J Cancer / 104 / 1619-27. 2011). The allelic H6D polymorphism in the GDF-15 gene was further identified as independent predictor of metastasis at the time of diagnosis (Brown, DA, Clin Cancer Res / 9 / 2642-50. 2003). The association between high sGDF-15 and poor outcome was further shown for thyroid, pancreatic, gastric, ovarian and other cancers (Mimeault, M and Batra SK, J Cell Physiol / 224 / 626-35. 2010; Bauskin, AR et al., Cancer Res / 66 / 4983-6. 2006; Brown, DA et al., Clin Cancer Res / 15 / 6658-64. 2009; Blanco-Calvo, M et al., Future Oncol / 10 / 1187-202. 2014; Staff, AC et al., Gynecol Oncol / 118 / 237-43. 2010). Similar findings have also been reported for the level of GDF-15 tissue expression as assessed by immunohistochemistry (Wallin, U et al., Br J Cancer / 104 / 1619-27. 2011). In melanoma, GDF-15 expression was found to increase from benign nevi over primary melanoma to melanoma metastases (Mauerer, A et al., Exp Dermatol / 20 / 502-7. 2011; Boyle, GM et al., J Invest Dermatol / 129 / 383-91. 2009). Serum concentrations of GDF-15 were indicative for metastasis in patients with uveal melanoma (Suesskind, D et al., Graefes Arch Clin Exp Ophthalmol / 250 / 887-95. 2012) and correlated with stage in patients with cutaneous melanoma (Kluger, HM et al., ClinCancer Res / 17 / 2417-25. 2011). Riker et al. compared gene expression in melanoma metastasis and primary tumor, and identified GDF-15 as the only soluble factor among the top 5 genes correlating with metastasis (Riker, Al et al., BMC Med Genomics 11 / 13. 2008). Boyle et al. (Boyle, GM et al., J Invest Dermatol / 129 / 383-91. 2009) found by immunohistochemistry that 15 of 22 primary melanomas expressed low levels of GDF-15 whereas 16 of 16 melanoma metastasis showed strong expression. Furthermore, knock-down of GDF-15 in three melanoma cell lines results in decreased tumorigenicity in the same study. Before that, Talantov et al. (Talantov, D et al., Clin Cancer Res / 11 / 7234-42. 2005) had already identified GDF-15 in melanoma metastases, but not in nevi and normal lymph nodes. Similar findings were reported in a study of Mauerer et al. who found GDF-15 to be preferentially expressed in metastatic tumors and in some primary melanomas, but not in melanocytic nevi (Mauerer, A et al., Exp Dermatol / 20 / 502-7. 2011). However, a direct role of GDF-15 in metastasis has only been shown in prostate cancer where constitutive overexpression of GDF-15 slowed cancer development but increased metastases (Husaini, Y et al., PLoS One / 7 / e43833. 2012). Clinical relevance of GDF-15 serum levels in melanoma patients was reported in two studies. GDF-15 serum concentrations were associated with metastasis in a cohort of 188 patients with metastatic (n=170) or non-metastatic (n=18) uveal melanoma (Suesskind, D et al., Graefes Arch Clin Exp Ophthalmol / 250 / 887-95. 2012). Finally, Kluger et al. reported a correlation between plasma levels of GDF-15 and stage in 216 patients with cutaneous melanoma (Kluger, HM et al., ClinCancer Res / 17 / 2417-25. 2011). In contrast to these findings, however, some studies have suggested an anti-tumorigenic role of GDF-15 (see, for instance, Liu T et al: "Macrophage inhibitory cytokine 1 reduces cell adhesion and induces apoptosis in prostate cancer cells." Cancer Res., vol. 63, no. 16, 1 August 2003, pp.5034-5040). Huh et al. (Am J Pathol / 176 / 2948-57. 2010) reports that macrophage inhibitory cytokine-1 regulates melanoma vascular development. Lasithiotakis et al. (Cancer / 107 / 1331-9. 2006) relates to the incidence and mortality of cutaneous melanoma in southern Germany. Corre et al. (Stem Cells Transl Med / 2 / 946-952. 2013) relates to the Growth Differentiation Factor 15 in Pathology: A Clinical Role?. Ünal et al. (Arch Dermatol Res / 307 / 551-7. 2015) relates to the divergent roles of growth differentiation factor-15 (GDF-15) in benign and malignant skin pathologies.

Thus, from the above-mentioned studies, and in view of the complex functional role of human GDF-15 (hGDF-15) in various cancers and its different effects on primary tumors and metastases in prostate cancer, it remained, however, unknown whether hGDF-15 could be used as a prognostic clinical marker for patient survival in melanoma.

Thus, there is a need in the art for prognostic biomarkers for melanoma, and in particular for improved prognostic biomarkers in melanoma, and for methods which allow to predict patient survival in melanoma more reliably.

### DESCRIPTION OF THE INVENTION

The present invention is as defined in the appended claims. The present invention meets the above needs and solves the above problems in the art by providing the embodiments described below:
In particular, in order to solve the above problems, the present inventors set out to investigate the impact of serum GDF-15 levels on overall survival (OS) of melanoma patients. In the course of these studies, the present inventors have surprisingly found that the probability of survival in melanoma patients significantly decreases with increasing hGDF-15 levels in the patient sera and vice versa. The inventors have shown that a high serum level of hGDF-15 is a potent biomarker for poor overall survival in tumor-free stage III and unresectable stage IV melanoma patients.

Thus, according to the invention, the probability of survival in melanoma patients inversely correlates with hGDF-15 levels.

Moreover, in the studies of the inventors, Cox regression analysis revealed that the knowledge of hGDF-15 serum levels adds independent prognostic information, e.g. if considered in combination with the M-category, and is superior to the established biomarker LDH in patients with distant metastasis.

Therefore, according to the invention, hGDF-15 levels can be used as a biomarker for the prediction of survival. This biomarker is advantageous, e.g. because it has a prognostic value that is independent of known biomarkers such as LDH. This means that if hGDF-15 levels are used for the prediction of melanoma patient survival according to the invention, they may, in a preferred aspect of the invention, be combined with additional biomarkers.

According to the invention, the combination of hGDF-15 levels as a biomarker with additional biomarkers such as LDH or S100B provides an improved prediction of survival, which is improved even when compared to the use of hGDF-15 levels alone.

Additionally, the use of hGDF-15 level as a biomarker is also advantageous because it allows to provide a prediction of survival that includes sub-groups of melanoma patients such as macroscopically tumor-free stage III patients, for which S100B represents the only available predictive and diagnostic marker.

The present invention is as defined in the appended claims. Thus, the present invention provides improved means to predict survival of melanoma patients as detailed below:
1. A method for predicting the probability of survival of a human melanoma patient, wherein the method comprises the steps of:
   a) determining the level of hGDF-15 in a human blood sample obtained from said patient; and
   b) predicting said probability of survival based on the determined level of hGDF-15 in said human blood sample; wherein a decreased level of hGDF-15 in said human blood sample indicates an increased probability of survival,

   wherein the human melanoma patient is a tumor-free stage III melanoma patient or an unresectable stage IV melanoma patient,
   wherein step b) comprises comparing said level of hGDF-15 determined in step a) with a hGDF-15 threshold level, wherein said probability is predicted based on the comparison of said level of hGDF-15 determined in step a) with said hGDF-15 threshold level; and wherein a level of hGDF-15 in said human blood sample which is decreased compared to said hGDF-15 threshold level indicates that the probability of survival is increased compared to a probability of survival at or above said hGDF-15 threshold level,
   wherein the human blood sample is a human serum sample, and
   wherein the hGDF-15 threshold level is a level selected from the range of between 1.1 ng/ml and 2.2 ng/ml.
2. The method of item 1, wherein the hGDF-15 threshold level is a hGDF-15 level selected from the range of between 1.2 ng/ml and 2.0 ng/ml.
3. The method of item 1, wherein the hGDF-15 threshold level is a hGDF-15 level selected from the range of between 1.3 ng/ml and 1.8 ng/ml.
4. The method of item 1, wherein the hGDF-15 threshold level is a hGDF-15 level selected from the range of between 1.4 ng/ml and 1.6 ng/ml.
5. The method of item 1, wherein the hGDF-15 threshold level is 1.5 ng/ml.
6. The method according to any one of the preceding items,
   wherein step a) further comprises determining the level of lactate dehydrogenase in said human blood sample, and
   wherein in step b), said probability of survival is also predicted based on the determined level of lactate dehydrogenase in said human blood sample; and wherein a decreased level of lactate dehydrogenase in said human blood sample indicates an increased probability of survival;
   wherein optionally, step b) comprises comparing said level of lactate dehydrogenase determined in step a) with a lactate dehydrogenase threshold level, wherein said probability is also predicted based on the comparison of said level of lactate dehydrogenase determined in step a) with said lactate dehydrogenase threshold level; and wherein a level of lactate dehydrogenase in said human blood sample which is decreased compared to said lactate dehydrogenase threshold level or is at said lactate dehydrogenase threshold level indicates that the probability of survival is increased compared to a probability of survival above said lactate dehydrogenase threshold level;
   wherein optionally, step a) further comprises determining the level of S100B in said human blood sample, and wherein in step b), said probability of survival is also predicted based on the determined level of S100B in said human blood sample; and wherein a decreased level of S100B in said human blood sample indicates an increased probability of survival,
      preferably wherein step b) comprises comparing said level of S100B determined in step a) with a S100B threshold level, wherein said probability is predicted based on the comparison of said level of S100B determined in step a) with said S100B threshold level; and wherein a level of S100B in said human blood sample which is decreased compared to said S100B threshold level or is at said S100B threshold level indicates that the probability of survival is increased compared to a probability of survival above said S100B threshold level.
7. The method according to any of the preceding items, wherein in step b), said probability of survival is also predicted based on the age of said patient; and wherein an increased age indicates a decreased probability of survival,
   preferably wherein step b) comprises comparing said age of said patient to a threshold age, wherein said probability is predicted based on the comparison of said age of said patient with said threshold age; and wherein an age of said patient which is equal to or increased compared to said threshold age indicates that the probability of survival is decreased compared to a probability of survival below said threshold age,
   more preferably wherein said threshold age is selected from the range of 60 to 65 years,
   most preferably wherein said threshold age is 63 years.
8. The method according to any one of the preceding items, wherein in step b), said probability of survival is also predicted based on metastasis; and wherein the presence of metastases in visceral organs other than lung indicates that the probability of survival is decreased as compared to the probability of survival when metastases are absent from visceral organs other than lung.
9. The method according to any of the preceding items, wherein step a) comprises determining the level of hGDF-15 by using one or more antibodies capable of binding to hGDF-15 or an antigen-binding portion thereof,
   preferably wherein the one or more antibodies capable of binding to hGDF-15 or the antigen-binding portion thereof form a complex with hGDF-15,
   and/or wherein the one or more antibodies comprise at least one polyclonal antibody,
   and/or wherein the one or more antibodies or the antigen-binding portion comprise at least one monoclonal antibody or an antigen-binding portion thereof, and wherein the binding is preferably binding to a conformational or discontinuous epitope on hGDF-15, and wherein the conformational or discontinuous epitope is comprised by the amino acid sequences of SEQ ID No: 25 and SEQ ID No: 26, and wherein preferably, the antibody or antigen-binding portion thereof comprises a heavy chain variable domain which comprises a CDR1 region comprising the amino acid sequence of SEQ ID NO: 3, a CDR2 region comprising the amino acid sequence of SEQ ID NO: 4 and a CDR3 region comprising the amino acid sequence of SEQ ID NO: 5, and the antibody or antigen-binding portion thereof comprises a light chain variable domain which comprises a CDR1 region comprising the amino acid sequence of SEQ ID NO: 6, a CDR2 region comprising the amino acid sequence ser-ala-ser and a CDR3 region comprising the amino acid sequence of SEQ ID NO: 7.
10. The method according to any one of items 1 to 9, wherein in step a), the level of hGDF-15 is determined by capturing hGDF-15 with a monoclonal antibody or antigen-binding fragment thereof according to item 9 and by detecting hGDF-15 with a polyclonal antibody, or by detecting hGDF-15 with a monoclonal antibody or antigen-binding fragment thereof which binds to a different epitope than the antibody which captures hGDF-15.
11. The method according to any one of the preceding items, wherein the method is an *in vitro* method.
12. The method according to any one of the preceding items,
   wherein
   (i) in step a), the level of hGDF-15 in the human blood sample is determined by an enzyme linked immunosorbent assay, or
   (ii) in step a), the level of hGDF-15 in the human blood sample is determined by an electrochemiluminescence assay, and wherein the electrochemiluminescence assay is preferably a sandwich detection method comprising a step of forming a detection complex between
      (A) streptavidin-coated beads;
      (B) a biotinylated first antibody or antigen-binding portion thereof capable of binding to hGDF-15;
      (C) hGDF-15 from the sample; and
      (D) a ruthenium complex-labelled second antibody or antigen-binding portion thereof capable of binding to hGDF-15;

      wherein said detection complex has the structure (A)-(B)-(C)-(D), and wherein the biotinylated first antibody or antigen-binding portion thereof binds to a first hGDF-15 epitope and the ruthenium complex-labelled second antibody or antigen-binding portion thereof binds to a second hGDF-15 epitope which is different from said first hGDF-15 epitope,
      wherein the method further comprises a step of detecting the detection complex by measuring electrochemiluminescence,
      and wherein the level of hGDF-15 in the human blood sample is determined based on the electrochemiluminescence measurement.
13. An apparatus configured to perform the method according to any one of items 1-12, wherein the apparatus is an electrochemiluminescence analyzer configured to perform the method according to item 12 wherein in step a), the level of hGDF-15 in the human blood sample is determined by an electrochemiluminescence assay.
14. Use of a detection kit in a method according to any one of items 1-12, the detection kit comprising:
   (i) streptavidin-coated beads;
   (ii) a biotinylated first antibody or antigen-binding portion thereof capable of binding to hGDF-15;
   (iii) recombinant hGDF-15, preferably in form of a buffered solution comprising recombinant hGDF-15, the buffered solution having a pH in the range of 4 to 7;
   (iv) a ruthenium complex-labelled second antibody or antigen-binding portion thereof capable of binding to hGDF-15; and optionally
   (v) instructions for use, preferably instructions for use in a method according to items 1-12; and preferably
   (vi) a container containing said recombinant hGDF-15, wherein the surface of the container which is in contact with recombinant hGDF-15 is coated with a non-adhesive material,

   wherein the biotinylated first antibody or antigen-binding portion thereof is capable of binding to a first hGDF-15 epitope and the ruthenium complex-labelled second antibody or antigen-binding portion thereof is capable of binding to a second hGDF-15 epitope which is different from said first hGDF-15 epitope,
   preferably wherein one of the first antibody or antigen-binding portion thereof capable of binding to hGDF-15 and second antibody or antigen-binding portion thereof capable of binding to hGDF-15 is a monoclonal antibody or antigen-binding portion thereof, wherein the binding is binding to a conformational or discontinuous epitope on hGDF-15 which is comprised by the amino acid sequences of SEQ ID No: 25 and SEQ ID No: 26, and wherein preferably,
   the antibody or antigen-binding portion thereof comprises a heavy chain variable domain which comprises a CDR1 region comprising the amino acid sequence of SEQ ID NO: 3, a CDR2 region comprising the amino acid sequence of SEQ ID NO: 4 and a CDR3 region comprising the amino acid sequence of SEQ ID NO: 5, and the antibody or antigen-binding portion thereof comprises a light chain variable domain which comprises a CDR1 region comprising the amino acid sequence of SEQ ID NO: 6, a CDR2 region comprising the amino acid sequence ser-ala-ser and a CDR3 region comprising the amino acid sequence of SEQ ID NO: 7.

### BRIEF DESCRIPTION OF THE DRAWINGS

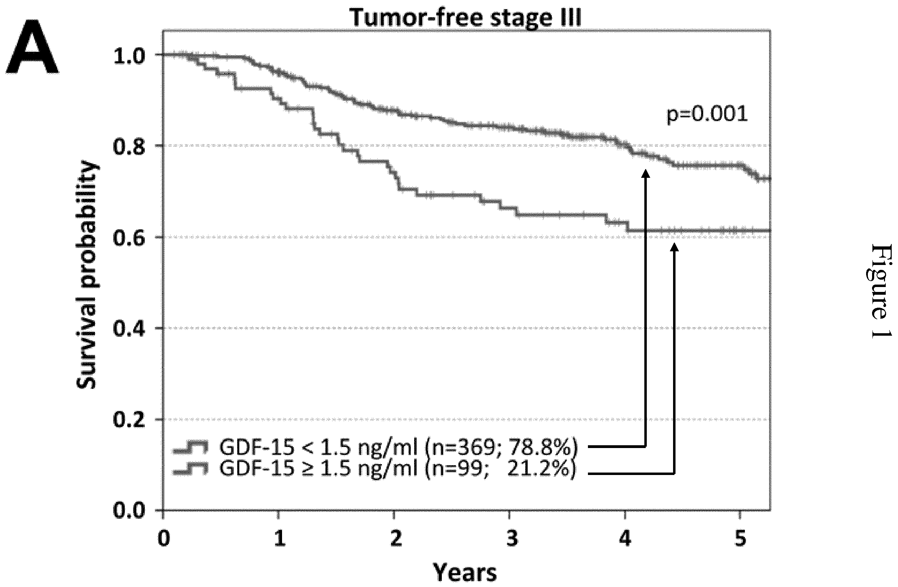

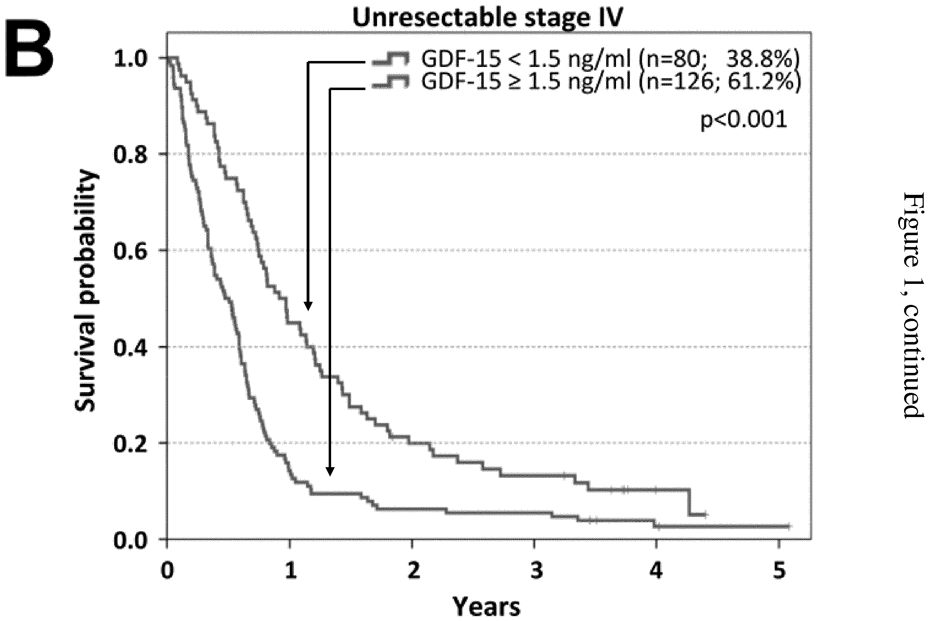

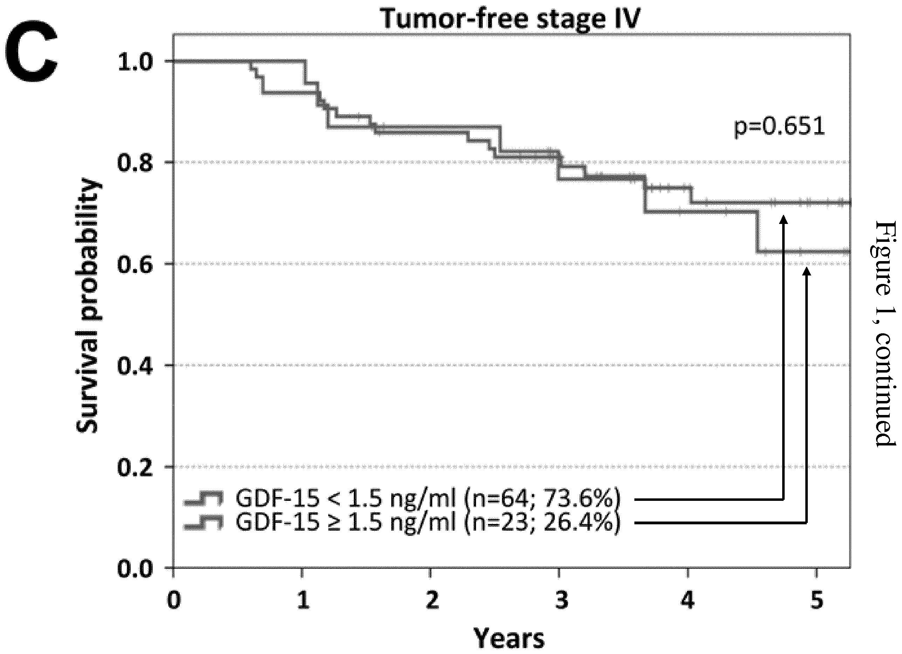

**Figure 1****:** Overall survival of distinct melanoma patient populations according to GDF-15 serum levels. Kaplan-Meier curves are shown for overall survival of 468 tumor-free stage III (A), 206 unresectable stage IV (B) and 87 tumor-free stage IV (C) patients with either normal (<1.5 ng/mL) or elevated (≥1.5 ng/mL) GDF-15 levels. Censoring is indicated by vertical lines; p-values were calculated by log rank statistics. In Figures 1A and 1B, the upper curves are those for normal hGDF-15 levels, and the lower curves are those for elevated hGDF-15 levels.

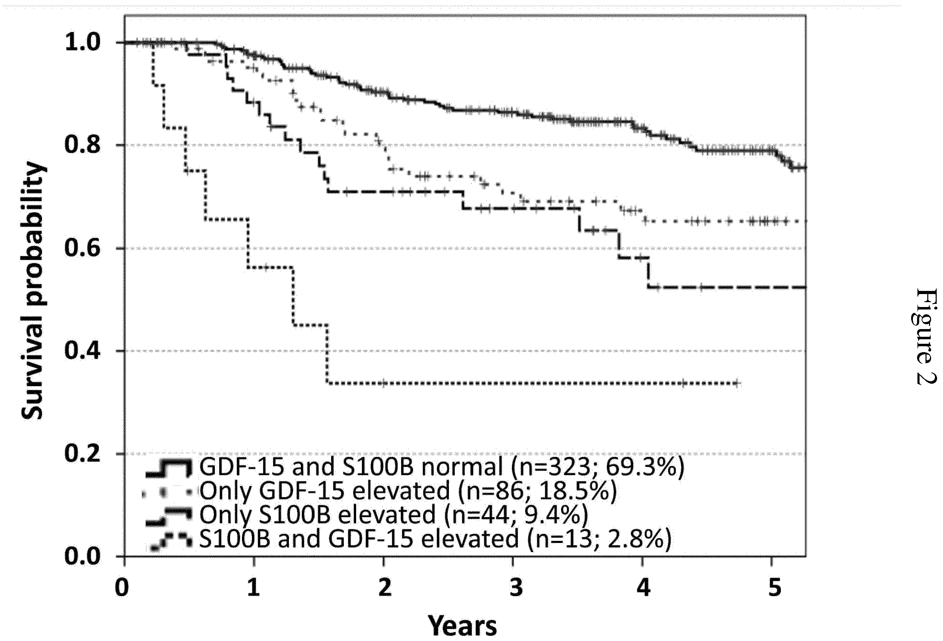

**Figure 2****:** Combinations of S100B and GDF-15 serum levels and their correlation with overall survival of stage III patients. Using a Cox regression model, S100B and hGDF-15 levels were shown to independently predict prognosis of tumor-free stage III patients. Kaplan-Meier curves of overall survival for patients with different biomarker combinations are presented for 466 patients. Censoring is indicated by vertical lines. In Figure 2, the highest curve is the curve for normal hGDF-15 levels and normal S100B levels, the 2^{nd} highest curve is the curve for elevated hGDF-15 levels, the 2^{nd} lowest curve is the curve for elevated S100B levels, and the lowest curve is the one for elevated hGDF-15 levels and elevated S100B levels.

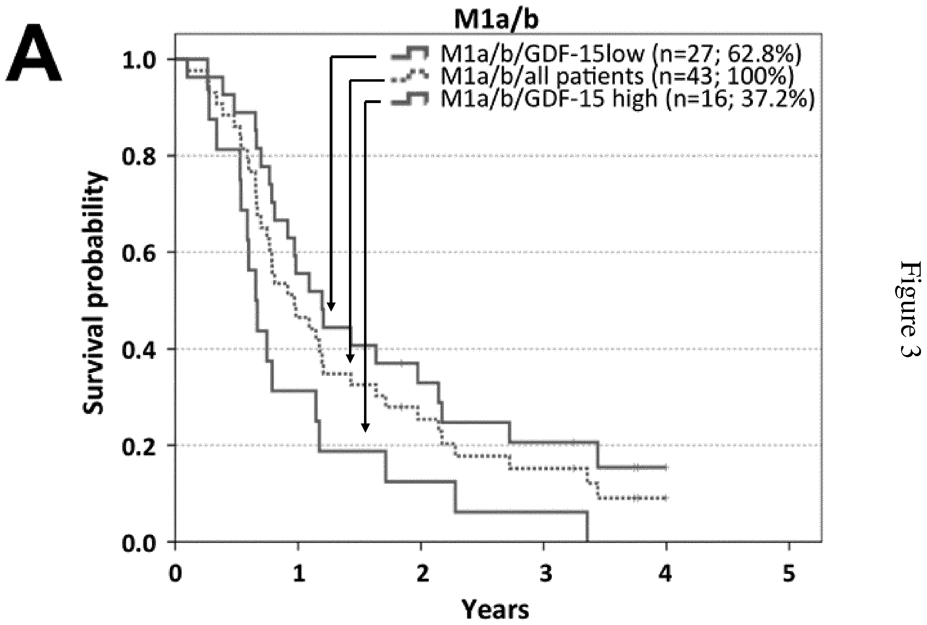

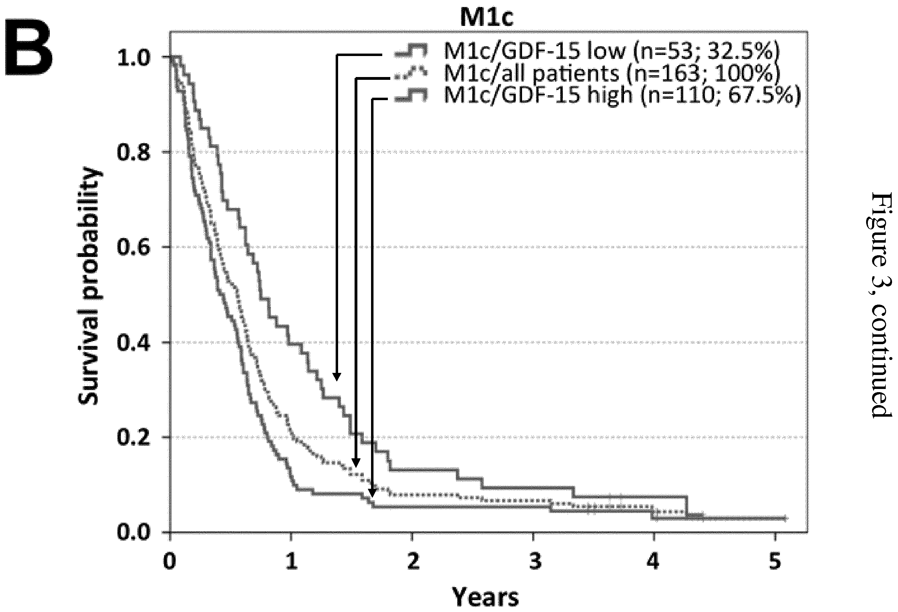

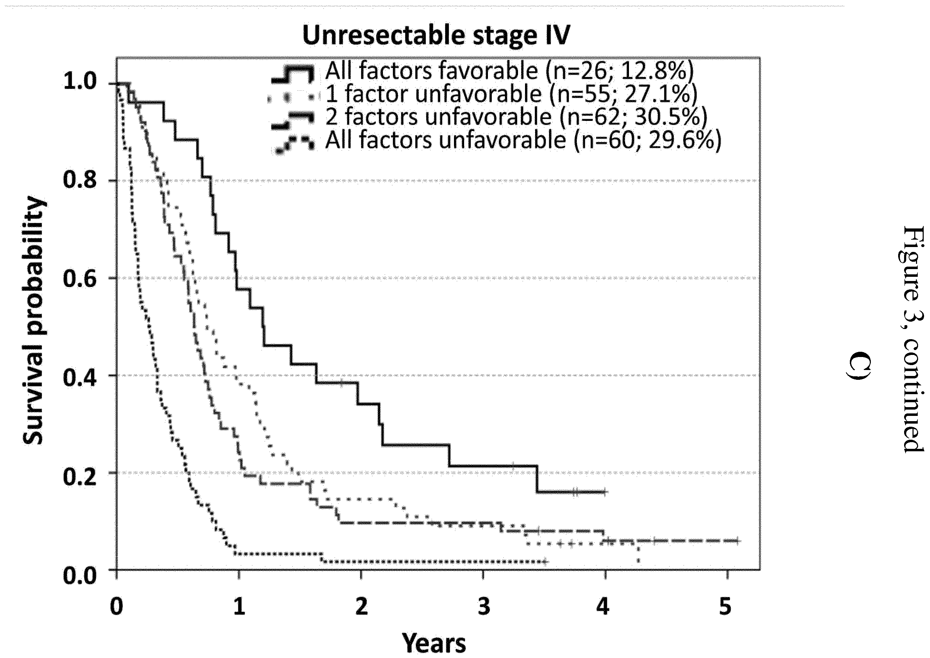

**Figure 3****:** Overall survival of unresectable stage IV patients according to combinations of serum levels of LDH and GDF-15, and the pattern of distant metastasis. The independent prognostic impact of GDF-15 serum levels on overall survival is illustrated for M-categories M1a/b (A) as well as for M1c patients (B). Broken lines indicate all patients of the given M-category. Continuous lines represent sub-groups with low (blue) or high (red) GDF-15 levels, respectively. Differences in OS between patients with high or low GDF-15 levels were significant for M1a/b and for M1c patients (log-rank p-values 0.047 and 0.003, respectively). In (C), overall survival is displayed according to the number of unfavorable values considering all 3 independent prognostic factors according to model 1 of Cox regression analysis (LDH levels, pattern of visceral metastasis, and GDF-15 levels). The order of curves (i.e. the order from the highest curve to the lowest curve) in the legend contained in panels (A) to (C) of the figure reflects the order of curves in the respective panels.

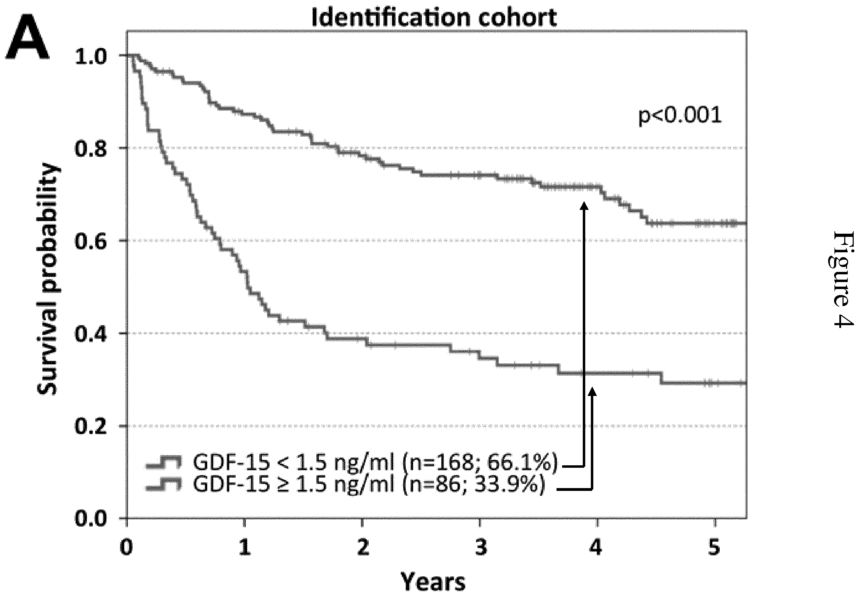

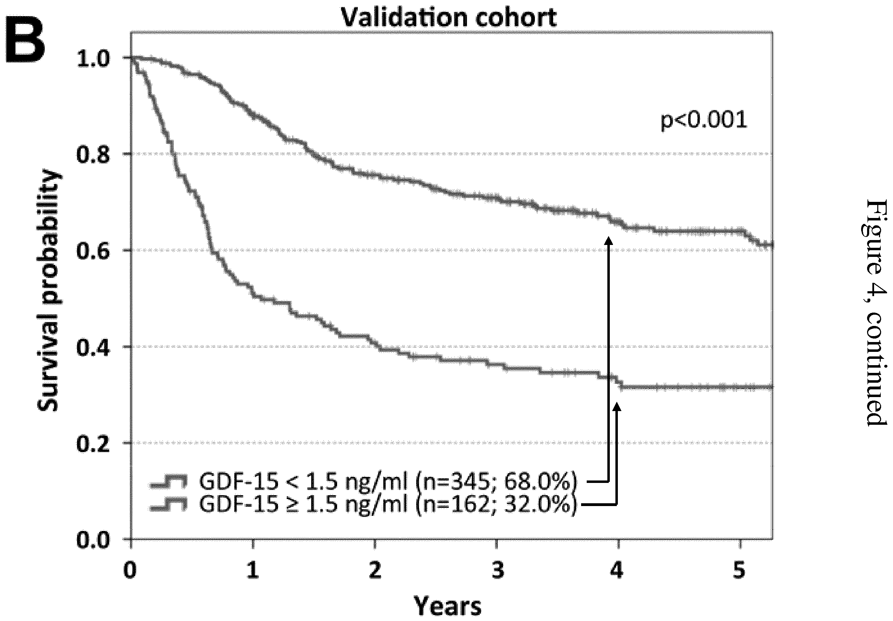

**Figure 4****:** Overall survival correlates with GDF-15 serum levels in melanoma patients. 762 patients were randomly assigned to two cohorts. In the identification cohort (254 patients), different cut-off values were tested by Kaplan-Meier analysis and log rank tests to obtain the best possible discrimination between patients with high and low GDF-15 serum levels. Overall survival of patients of the identification cohort according to the optimized cut-off point (<1.5 ng/mL vs. ≥1.5 ng/mL) is shown in (A). Differences in overall survival were confirmed in 508 patients of the validation cohort (B). Censoring is indicated by vertical lines; p-values were calculated by log rank statistics. In Figures 4A and 4B, the upper curves are those for normal hGDF-15 levels, and the lower curves are those for elevated hGDF-15 levels.

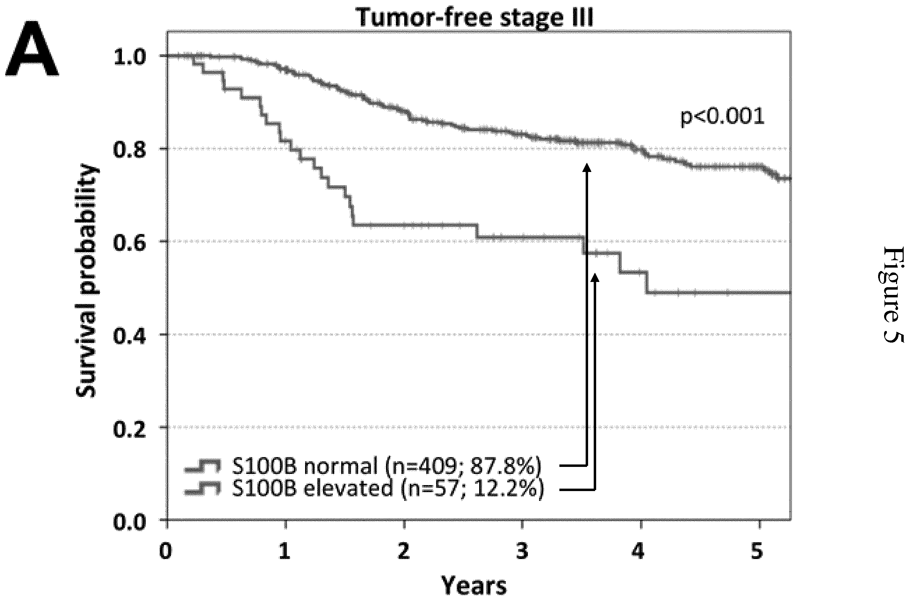

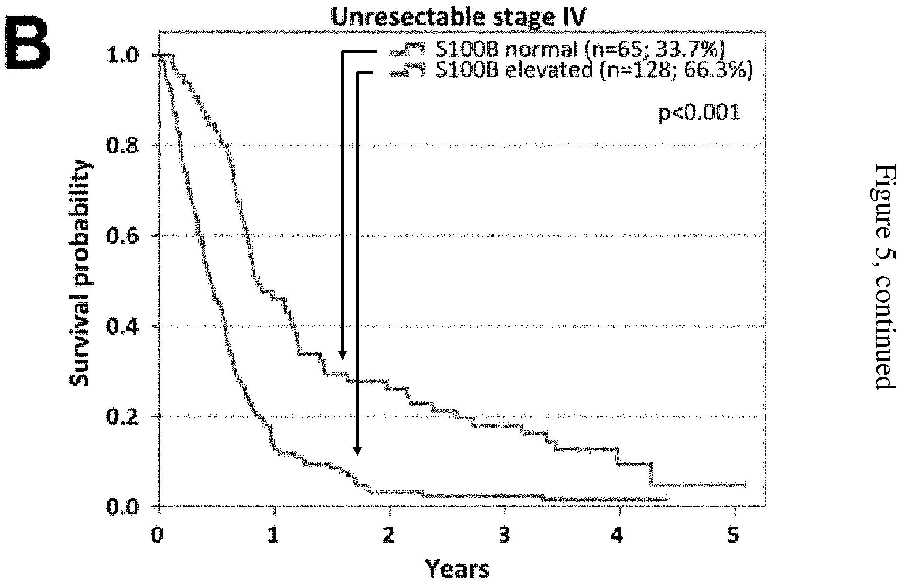

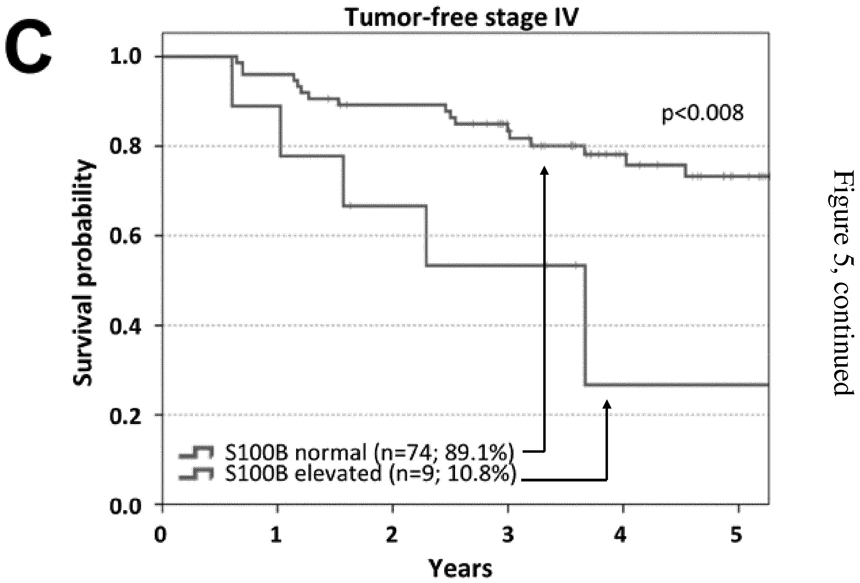

**Figure 5****:** Overall survival according to S100B serum levels. Kaplan-Meier curves are shown for overall survival of 466 tumor-free stage III (A), 193 unresectable stage IV (B) and 83 tumor-free stage IV (C) patients. Patients were categorized based on S100B serum levels (normal vs. elevated). Censoring is indicated by vertical lines; p-values were calculated by log rank statistics. In Figures 5A to 5C, the upper curves are those for normal S100B levels, and the lower curves are those for elevated S100B levels.

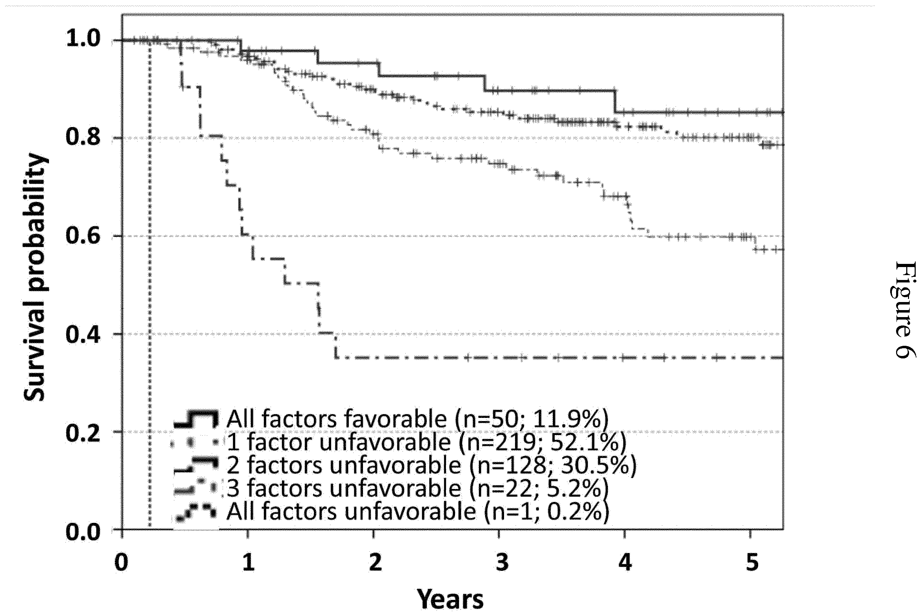

**Figure 6****:** Overall survival of stage III patients according to the number of unfavorable values considering serum levels of GDF-15, S100B, age, and sub-stage. Model 2 of Cox regression analysis (Table 2) revealed an independent negative prognostic impact for GDF-15 levels >1.5 ng/mL, for elevated S100B levels, for age <63 years, and for sub-stage IIIC. Patients were now stratified according to the number of unfavorable values among those four factors. The resulting Kaplan-Meier curves of overall survival are presented and censoring is indicated by vertical lines. The highest curve is the curve, wherein all factors are favorable. The 2^{nd} highest curve is the curve, wherein one factor is unfavorable. The 3^{rd} highest curve is the curve, wherein two factors are unfavorable. The 2^{nd} lowest curve is the curve, wherein three factors are unfavorable. The lowest curve is the curve, wherein all factors are unfavorable.

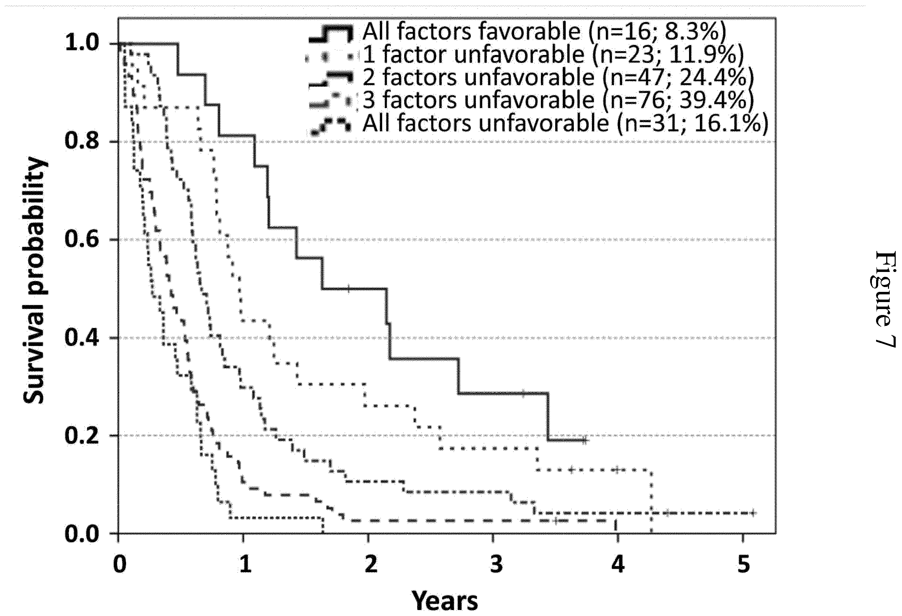

**Figure 7****:** Overall survival of unresectable stage IV patients according to the number of unfavorable values considering serum levels of GDF-15, S100B, the pattern of distant metastasis, and age. Model 2 of Cox regression analysis (Table 3) revealed an independent negative prognostic impact for GDF-15 levels >1.5 ng/mL, for elevated S100B levels, for the metastatic involvement of visceral organs other than lung, and for age of 63 years or older. Patients were thus stratified according to the number of unfavorable factors. The resulting Kaplan-Meier curves for overall survival are shown and censoring is indicated by vertical lines. The highest curve is the curve, wherein all factors are favorable. The 2^{nd} highest curve is the curve, wherein one factor is unfavorable. The 3^{rd} highest curve is the curve, wherein two factors are unfavorable. The 2^{nd} lowest curve is the curve, wherein three factors are unfavorable. The lowest curve is the curve, wherein all factors are unfavorable.

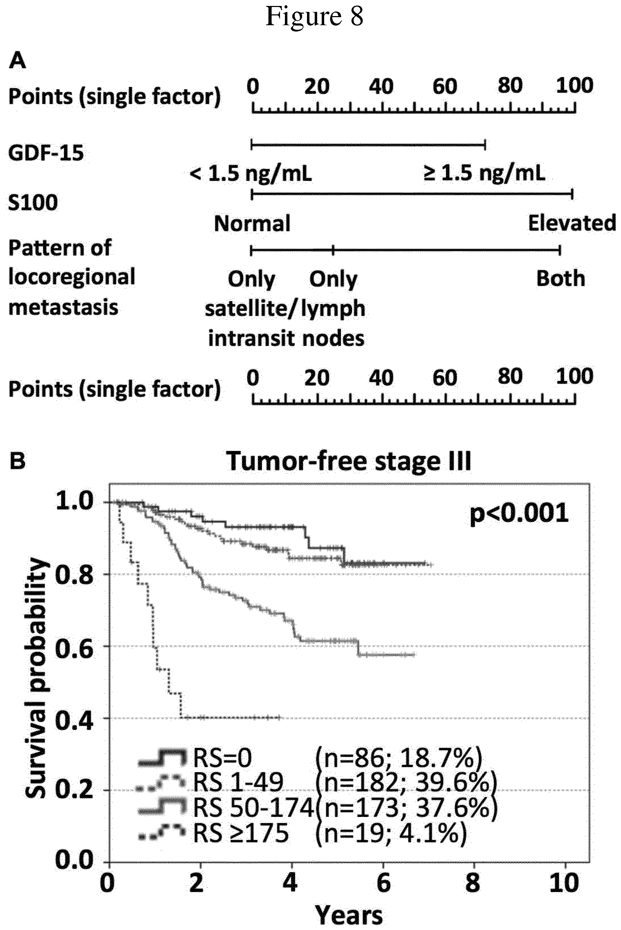

**Figure 8****:** Overall survival subsequent to serum sampling of stage III patients according to combinations of different factors. A nomogram (a) was developed for tumor-free stage III patients using the nomogram function of R considering the relative impact of single independent factors according to multivariate analysis (sGDF-15, sS100B, pattern of loco-regional metastasis). A risk score ranging between 0 and 266 points was calculated for 466 stage III patients with complete data. In (b), Kaplan-Meier curves of overall survival subsequent to serum sampling is displayed for different risk score categories. Censoring is indicated by vertical lines.

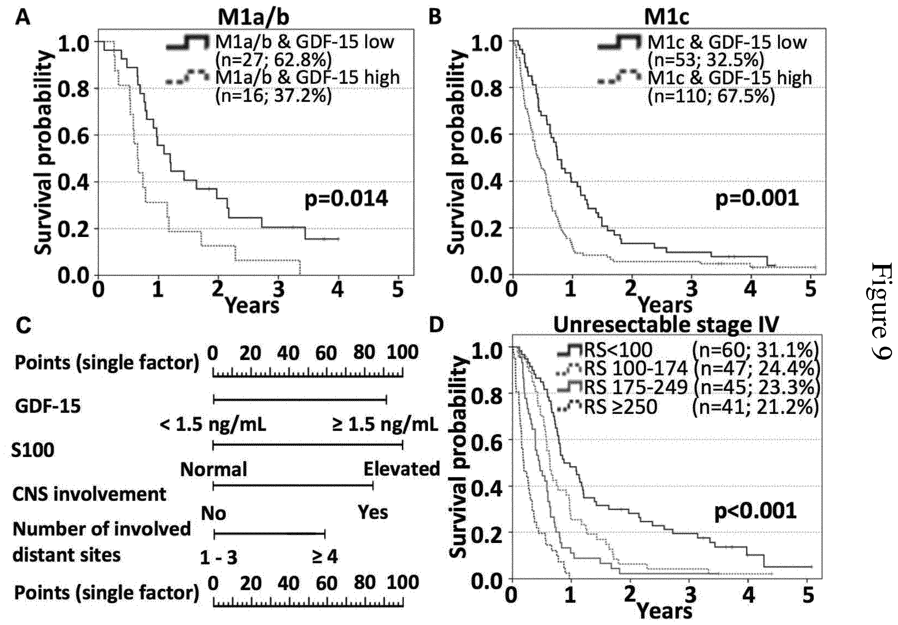

**Figure 9****:** Overall survival subsequent to serum sampling of unresectable stage IV patients according to combinations of different factors. GDF-15 serum levels have independent impact on overall survival of unresectable stage IV patients in addition to the M category. This is illustrated by the significant differences in OS according to sGDF-15 in both, M1a/b patients (a), and M1c patients (b). A nomogram (c) was developed for unresectable stage IV patients using the nomogram function of R considering the relative impact of single independent factors according to multivariate analysis (sGDF-15, sS100B, CNS involvement, and number of involved distant sites). A risk score ranging between 0 and 334 points was calculated for 193 unresectable stage IV patients with complete data. In (d), Kaplan-Meier curves of overall survival subsequent to serum sampling is displayed for different risk score categories. Censoring is indicated by vertical lines.

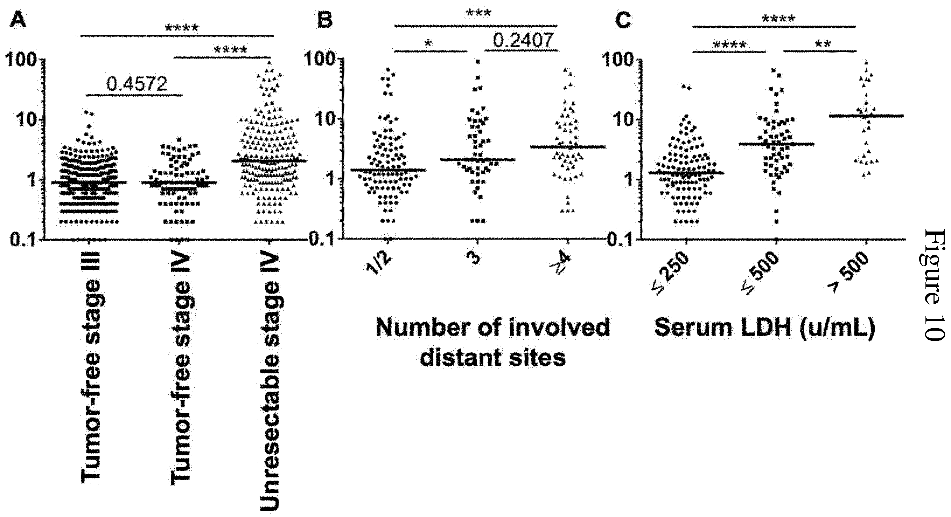

**Figure 10****:** Correlations of sGDF-15 with stage/disease status and sLDH. Serum GDF-15 levels are shown for tumor-free stage III (n=468), tumor-free stage IV patients (n=87) and unresectable stage IV patients (n=206) (a). In unresectable stage IV patients, sGDF-15 is presented for according to the number of distant metastases (b) or stratified according to sLDH (c). Red bars indicate median levels of GDF-15; ** p < 0.01; *** p < 0.001 using Mann Whitney tests.

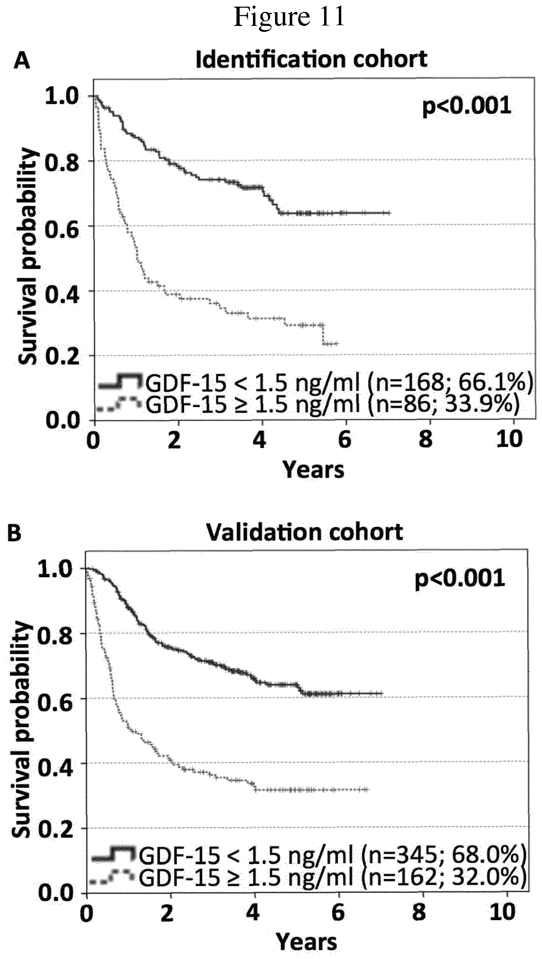

**Figure 11****:** Overall survival subsequent to serum sampling correlates with GDF-15 serum levels in melanoma patients. 761 patients were randomly assigned to two cohorts. In the identification cohort (254 patients), different cut-off values were tested by Kaplan-Meier analysis and log rank tests to obtain the best possible discrimination between patients with high and low GDF-15 serum levels. Overall survival subsequent to serum sampling of patients of the identification cohort according to the optimized cut-off point (<1.5ng/mL vs. ≥1.5ng/mL) is shown in (a). Differences in overall survival subsequent to serum sampling were confirmed in 507 patients of the validation cohort (b). Censoring is indicated by vertical lines; p-values were calculated by log rank statistics.

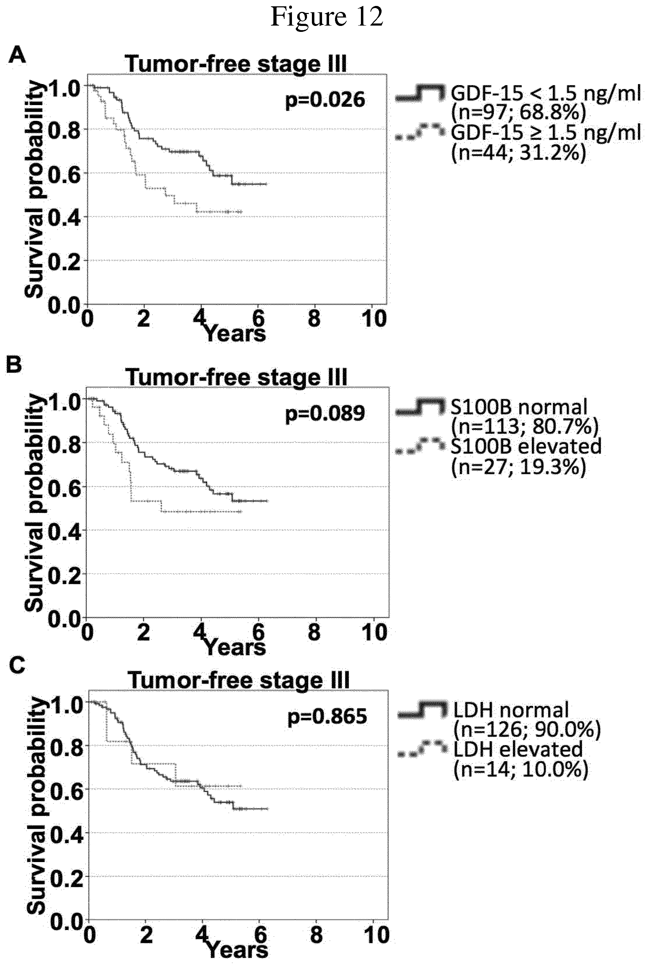

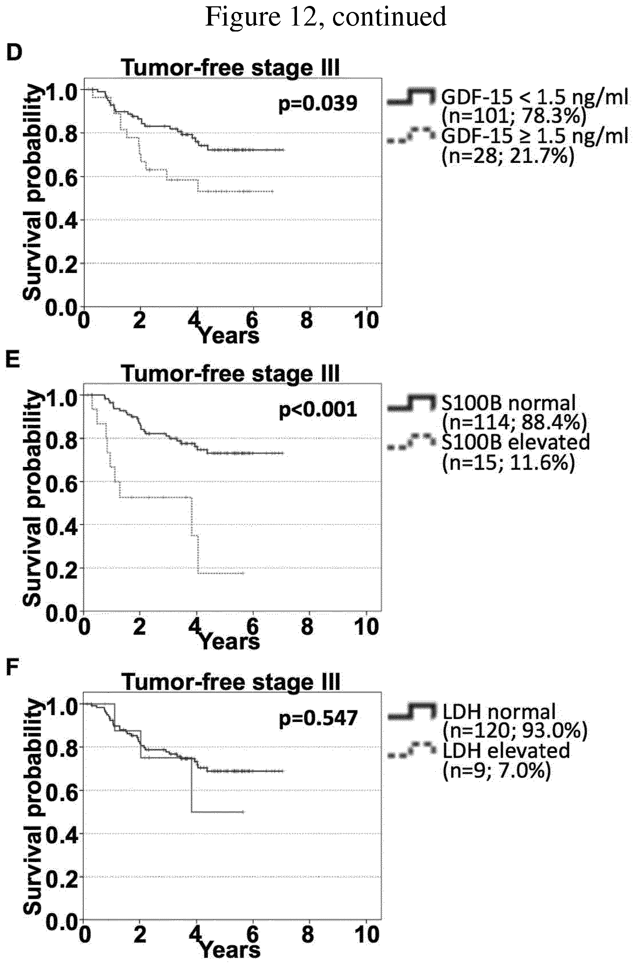

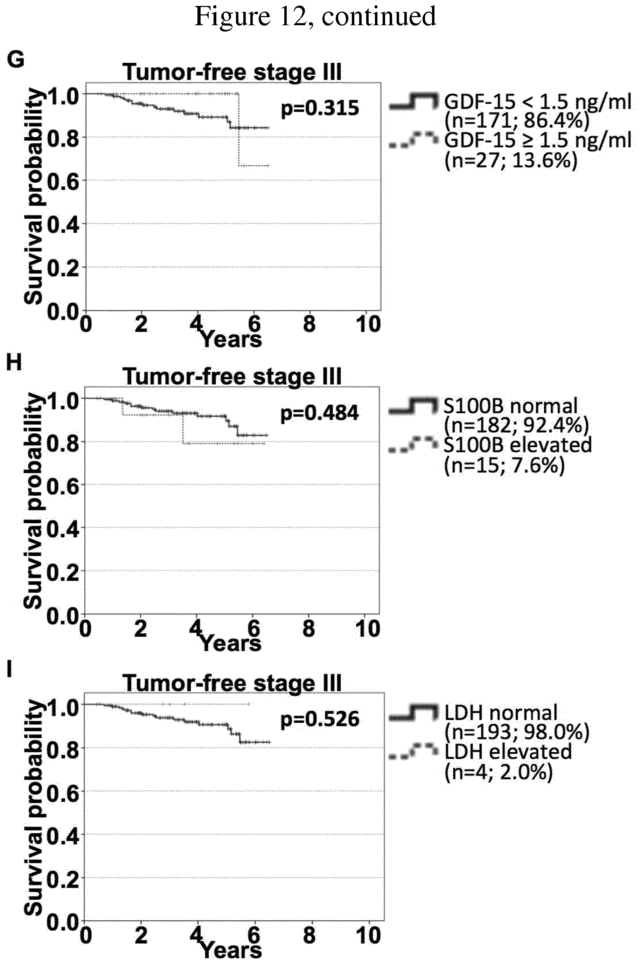

**Figure 12****:** Association of sGDF-15, sS100B and sLDH with OS according to time-point of serum sampling in tumor-free stage III patients. Overall survival of tumor-free stage III patients according to sGDF-15 (left), sS100B (middle) and sLDH (right) according to the time point of last recurrence before serum sampling. Patients were categorized as being tumor-free for less than 6 months (a-c), for between 6 months and 2 years (d-f) or for more than 2 years (g-i) since detection of last metastasis. Censoring is indicated by vertical lines; p-values were calculated by log rank statistics.

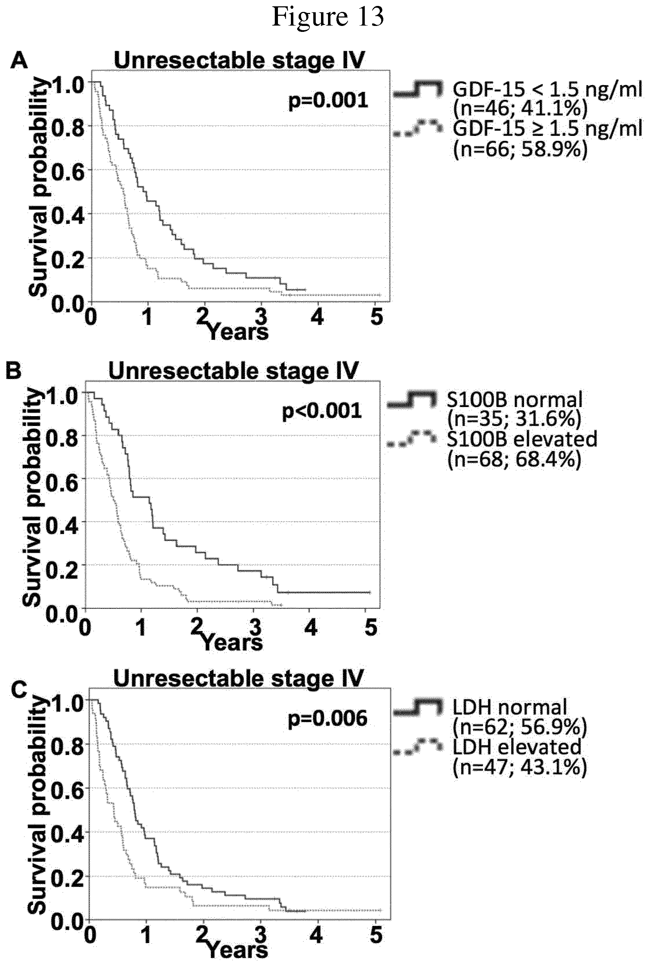

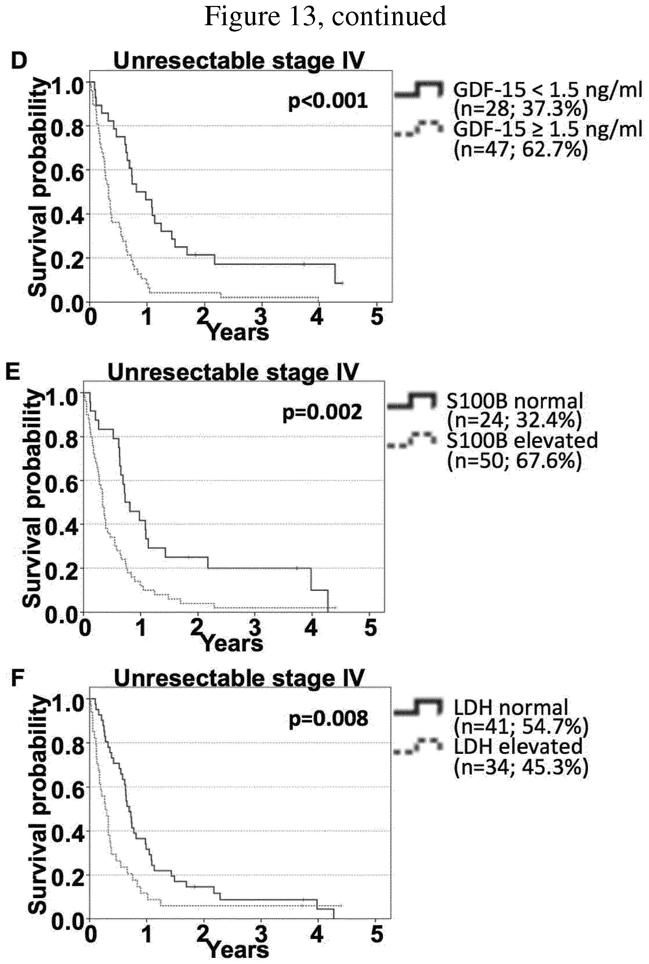

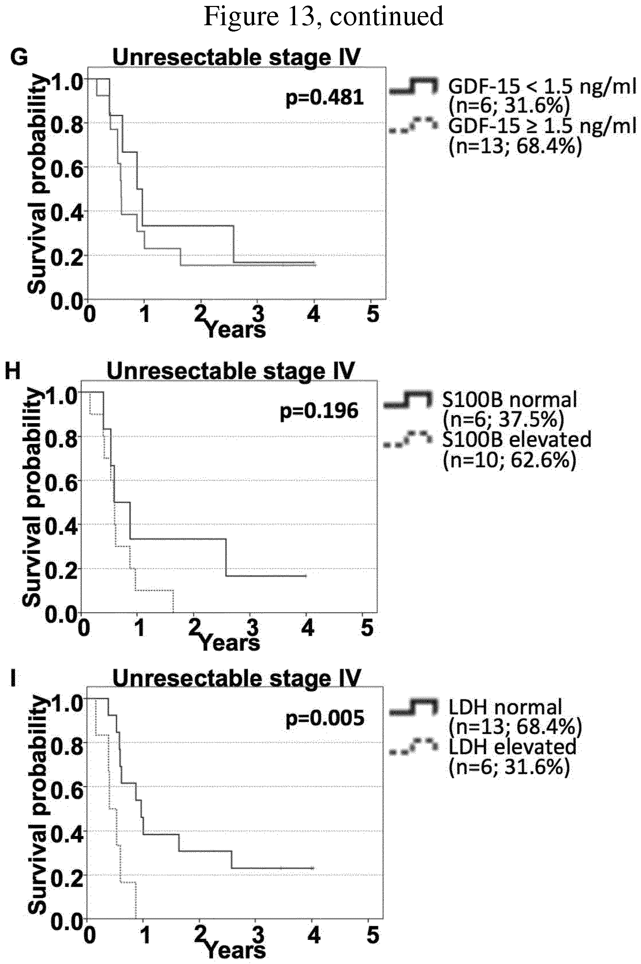

**Figure 13****:** Association of sGDF-15, sS100B and sLDH with OS according to time-point of serum sampling in unresectable stage IV patients. Overall survival of unresectable stage IV patients according to sGDF-15 (left), S100B (middle) and LDH (right) according to the time span since diagnosis of stage IV disease. The first distant metastasis was detected within 6 months (a-c), between 6 months and 2 years (d-f) and more than 2 years (g-i) before serum sampling. Censoring is indicated by vertical lines; p-values were calculated by log rank statistics.

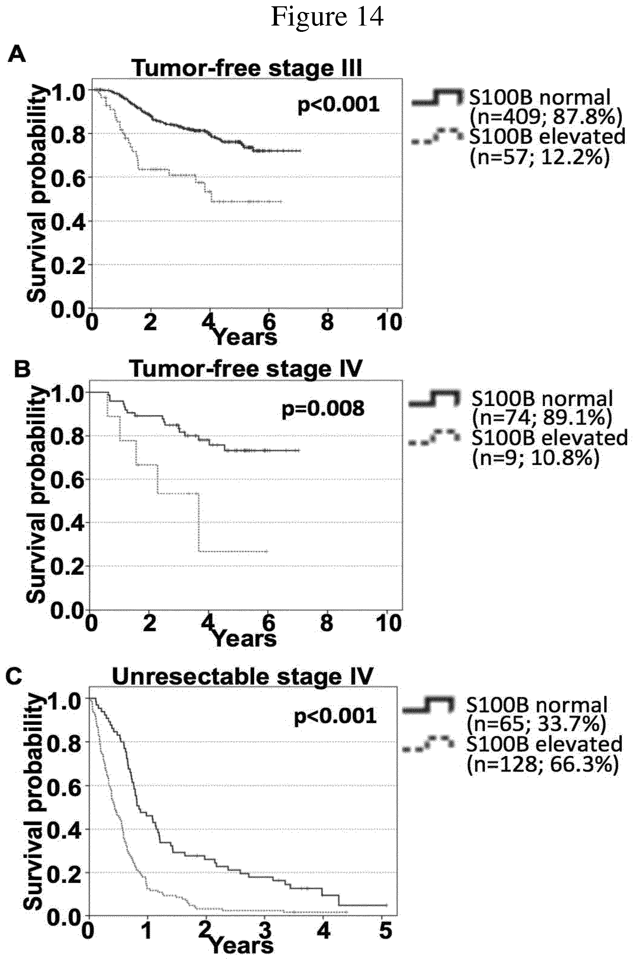

**Figure 14****:** Overall survival subsequent to serum sampling according to S100B serum levels. Kaplan-Meier curves are shown for overall survival subsequent to serum sampling of 466 tumor-free stage III (a), 83 tumor-free stage IV (b) and 193 unresectable stage IV (c) patients. Patients were categorized based on S100B serum levels (normal vs. elevated). Censoring is indicated by vertical lines; p-values were calculated by log rank statistics.

### DETAILED DESCRIPTION OF THE INVENTION

### Definitions and General Techniques

Unless otherwise defined below, the terms used in the present invention shall be understood in accordance with their common meaning known to the person skilled in the art.

The term "antibody" as used herein refers to any functional antibody that is capable of specific binding to the antigen of interest, as generally outlined in chapter 7 of Paul, W.E. (Ed.).: Fundamental Immunology 2nd Ed. Raven Press, Ltd., New York 1989. Without particular limitation, the term "antibody" encompasses antibodies from any appropriate source species, including chicken and mammalian such as mouse, goat, non-human primate and human. Preferably, the antibody is a humanized antibody. The antibody is preferably a monoclonal antibody which can be prepared by methods well-known in the art. The term "antibody" encompasses an IgG-1, -2, -3, or -4, IgE, IgA, IgM, or IgD isotype antibody. The term "antibody" encompasses monomeric antibodies (such as IgD, IgE, IgG) or oligomeric antibodies (such as IgA or IgM). The term "antibody" also encompasses - without particular limitations - isolated antibodies and modified antibodies such as genetically engineered antibodies, e.g. chimeric antibodies.

The nomenclature of the domains of antibodies follows the terms as known in the art. Each monomer of an antibody comprises two heavy chains and two light chains, as generally known in the art. Of these, each heavy and light chain comprises a variable domain (termed V_{H} for the heavy chain and V_{L} for the light chain) which is important for antigen binding. These heavy and light chain variable domains comprise (in an N-terminal to C-terminal order) the regions FR1, CDR1, FR2, CDR2, FR3, CDR3, and FR4 (FR, framework region; CDR, complementarity determining region which is also known as hypervariable region). The identification and assignment of the above-mentioned antibody regions within the antibody sequence is generally in accordance with Kabat et al. (Sequences of proteins of immunological interest, U.S. Dept. of Health and Human Services, Public Health Service, National Institutes of Health, Bethesda, Md. 1983), or Chothia et al. (Conformations of immunoglobulin hypervariable regions. Nature. 1989 Dec 21-28;342(6252):877-83.), or may be performed by using the IMGT/V-QUEST software described in Giudicelli et al. (IMGT/V-QUEST, an integrated software program for immunoglobulin and T cell receptor V-J and V-D-J rearrangement analysis. Nucleic Acids Res. 2004 Jul 1;32(Web Server issue): W435-40). Preferably, the antibody regions indicated above are identified and assigned by using the IMGT/V-QUEST software.

A "monoclonal antibody" is an antibody from an essentially homogenous population of antibodies, wherein the antibodies are substantially identical in sequence (i.e. identical except for minor fraction of antibodies containing naturally occurring sequence modifications such as amino acid modifications at their N- and C-termini). Unlike polyclonal antibodies which contain a mixture of different antibodies directed to either a single epitope or to numerous different epitopes, monoclonal antibodies are directed to the same epitope and are therefore highly specific. The term "monoclonal antibody" includes (but is not limited to) antibodies which are obtained from a monoclonal cell population derived from a single cell clone, as for instance the antibodies generated by the hybridoma method described in Kohler and Milstein (Nature, 1975 Aug 7;256(5517):495-7) or Harlow and Lane ("Antibodies: A Laboratory Manual" Cold Spring Harbor Laboratory Press, Cold Spring Harbor, New York 1988). A monoclonal antibody may also be obtained from other suitable methods, including phage display techniques such as those described in Clackson et al. (Nature. 1991 Aug 15;352(6336):624-8) or Marks et al. (J Mol Biol. 1991 Dec 5;222(3):581-97). A monoclonal antibody may be an antibody that has been optimized for antigen-binding properties such as decreased Kd values, optimized association and dissociation kinetics by methods known in the art. For instance, Kd values may be optimized by display methods including phage display, resulting in affinity-matured monoclonal antibodies. The term "monoclonal antibody" is not limited to antibody sequences from particular species of origin or from one single species of origin. Thus, the meaning of the term "monoclonal antibody" encompasses chimeric monoclonal antibodies such as humanized monoclonal antibodies.

"Humanized antibodies" are antibodies which contain human sequences and a minor portion of non-human sequences which confer binding specificity to an antigen of interest (e.g. human GDF-15). Typically, humanized antibodies are generated by replacing hypervariable region sequences from a human acceptor antibody by hypervariable region sequences from a non-human donor antibody (e.g. a mouse, rabbit, rat donor antibody) that binds to an antigen of interest (e.g. human GDF-15). In some cases, framework region sequences of the acceptor antibody may also be replaced by the corresponding sequences of the donor antibody. In addition to the sequences derived from the donor and acceptor antibodies, a "humanized antibody" may either contain other (additional or substitute) residues or sequences or not. Such other residues or sequences may serve to further improve antibody properties such as binding properties (e.g. to decrease Kd values) and/or immunogenic properties (e.g. to decrease antigenicity in humans). Non-limiting examples for methods to generate humanized antibodies are known in the art, e.g. from Riechmann et al. (Nature. 1988 Mar 24; 332(6162):323-7) or Jones et al. (Nature. 1986 May 29-Jun 4; 321 (6069):522-5).

The term "human antibody" relates to an antibody containing human variable and constant domain sequences. This definition encompasses antibodies having human sequences bearing single amino acid substitutions or modifications which may serve to further improve antibody properties such as binding properties (e.g. to decrease Kd values) and/or immunogenic properties (e.g. to decrease antigenicity in humans). The term "human antibody" excludes humanized antibodies where a portion of non-human sequences confers binding specificity to an antigen of interest.

An "antigen-binding portion" of an antibody as used herein refers to a portion of an antibody that retains the capability of the antibody to specifically bind to the antigen (e.g. hGDF-15, PD-1, PD-L1 or CTLA4). This capability can, for instance, be determined by determining the capability of the antigen-binding portion to compete with the antibody for specific binding to the antigen by methods known in the art. The antigen-binding portion may contain one or more fragments of the antibody. Without particular limitation, the antigen-binding portion can be produced by any suitable method known in the art, including recombinant DNA methods and preparation by chemical or enzymatic fragmentation of antibodies. Antigen-binding portions may be Fab fragments, F(ab') fragments, F(ab')₂ fragments, single chain antibodies (scFv), single-domain antibodies, diabodies or any other portion(s) of the antibody that retain the capability of the antibody to specifically bind to the antigen.

An "antibody" (e.g. a monoclonal antibody) or an "antigen-binding portion" may have been derivatized or be linked to a different molecule. For example, molecules that may be linked to the antibody are other proteins (e.g. other antibodies), a molecular label (e.g. a fluorescent, luminescent, colored or radioactive molecule), a pharmaceutical and/or a toxic agent. The antibody or antigen-binding portion may be linked directly (e.g. in form of a fusion between two proteins), or via a linker molecule (e.g. any suitable type of chemical linker known in the art).

As used herein, the terms "binding" or "bind" refer to specific binding to the antigen of interest (e.g. human GDF-15). Preferably, the Kd value is less than 100 nM, more preferably less than 50 nM, still more preferably less than 10 nM, still more preferably less than 5 nM and most preferably less than 2 nM.

The term "epitope" as used herein refers to a small portion of an antigen that forms the binding site for an antibody.

For the purposes of characterizing the binding properties of antibodies, binding or competitive binding of antibodies or their antigen-binding portions to the antigen of interest (e.g. human GDF-15) is preferably measured by using surface plasmon resonance measurements as a reference standard assay, as described below.

The terms "K_{D}" or "K_{D} value" relate to the equilibrium dissociation constant as known in the art. In the context of the present disclosure, these terms relate to the equilibrium dissociation constant of an antibody with respect to a particular antigen of interest (e.g. human GDF-15) The equilibrium dissociation constant is a measure of the propensity of a complex (e.g. an antigen-antibody complex) to reversibly dissociate into its components (e.g. the antigen and the antibody). For the antibodies according to the disclosure, K_{D} values (such as those for the antigen human GDF-15) are preferably determined by using surface plasmon resonance measurements as described below.

An "isolated antibody" as used herein is an antibody that has been identified and separated from the majority of components (by weight) of its source environment, e.g. from the components of a hybridoma cell culture or a different cell culture that was used for its production (e.g. producer cells such as CHO cells that recombinantly express the antibody). The separation is performed such that it sufficiently removes components that may otherwise interfere with the suitability of the antibody for the desired applications (e.g. with a therapeutic use of the anti-human GDF-15 antibody). Methods for preparing isolated antibodies are known in the art and include Protein A chromatography, anion exchange chromatography, cation exchange chromatography, virus retentive filtration and ultrafiltration. Preferably, the isolated antibody preparation is at least 70 % pure (w/w), more preferably at least 80 % pure (w/w), still more preferably at least 90 % pure (w/w), still more preferably at least 95 % pure (w/w), and most preferably at least 99 % pure (w/w), as measured by using the Lowry protein assay.

A "diabody" as used herein is a small bivalent antigen-binding antibody portion which comprises a heavy chain variable domain linked to a light chain variable domain on the same polypeptide chain linked by a peptide linker that is too short to allow pairing between the two domains on the same chain. This results in pairing with the complementary domains of another chain and in the assembly of a dimeric molecule with two antigen binding sites. Diabodies may be bivalent and monospecific (such as diabodies with two antigen binding sites for human GDF-15), or may be bivalent and bispecific (e.g. diabodies with two antigen binding sites, one being a binding site for human GDF-15, and the other one being a binding site for a different antigen). A detailed description of diabodies can be found in Holliger P et al. (""Diabodies": small bivalent and bispecific antibody fragments." Proc Natl Acad Sci USA. 1993 Jul 15;90(14):6444-8.).

A "single-domain antibody" (which is also referred to as "Nanobody^{™}") as used herein is an antibody fragment consisting of a single monomeric variable antibody domain. Structures of and methods for producing single-domain antibodies are known from the art, e.g. from Holt LJ et al. ("Domain antibodies: proteins for therapy." Trends Biotechnol. 2003 Nov;21(11):484-90.), Saerens D et al. ("Single-domain antibodies as building blocks for novel therapeutics." Curr Opin Pharmacol. 2008 Oct;8(5):600-8. Epub 2008 Aug 22.), and Arbabi Ghahroudi M et al. ("Selection and identification of single domain antibody fragments from camel heavy-chain antibodies." FEBS Lett. 1997 Sep 15;414(3):521-6.).

The terms "significant", "significantly", etc. as used herein refer to a statistically significant difference between values as assessed by appropriate methods known in the art, and as assessed by the methods referred to herein.

In accordance with the present invention, each occurrence of the term "comprising" may optionally be substituted with the term "consisting of".

The terms "cancer" and "cancer cell" is used herein in accordance with their common meaning in the art (see for instance Weinberg R. et al.: The Biology of Cancer. Garland Science: New York 2006. 850p).

The cancers, for which a prediction of a clinical outcome, in particular a prediction of patient survival according to the present invention is provided, is a melanoma as defined in the appended claims. As used herein, the term "melanoma" is used in accordance with its general meaning known in the art. Melanomas are classified according to the AJCC staging system for melanoma patients with distant metastases since 2001 (Balch, CM et al., J Clin Oncol / 19 / 3635-48. 2001). The melanoma stages referred to herein refer to this staging system. In a preferred aspect of the present invention in accordance with all of the embodiments of the present invention, the melanoma is not a uveal melanoma.

The melanoma patients, for which a prediction of survival according to the invention is provided, may be subject to a treatment of the melanoma. As used herein, terms such as "treatment of cancer" or "treating cancer" or "treatment of melanoma" or "treating melanoma" refer to a therapeutic treatment. As used herein, such treatments do not only include treatments of the melanoma itself but also palliative treatments. Such palliative treatments are known in the art and include, for instance, treatments which only improve the symptoms of the melanoma disease.

As referred to herein, a treatment of cancer can be a first-line therapy, a second-line therapy or a third-line therapy or a therapy that is beyond third-line therapy. The meaning of these terms is known in the art and in accordance with the terminology that is commonly used by the US National Cancer Institute.

A treatment of cancer does not exclude that additional or secondary therapeutic benefits also occur in patients. For example, an additional or secondary benefit may be an influence on cancer-induced weight loss.

As referred to herein, a "tumor-free" melanoma patient is a patient in which no primary tumor and no metastasis can be detected according to clinical standard methods known in the art. This, however, does not exclude that tumors (or micrometastases) exist in the patient, which are below the detection limit, or that tumor cells exist, which may form a new tumor.

### Blood samples:

As referred to herein, the term "blood sample" includes, without limitation, whole blood, serum and plasma samples. It also includes other sample types such as blood fractions other than serum and plasma. Such samples and fractions are known in the art.

Blood samples which are used for the methods according to the disclosure can be any types of blood samples which contain hGDF-15. Suitable types of blood samples containing hGDF-15 are known in the art and include serum and plasma samples. Alternatively, further types of blood samples which contain hGDF-15 can also be readily identified by the skilled person, e.g. by measuring whether hGDF-15 is contained in these samples, and which levels of hGDF-15 are contained in these samples, by using the methods disclosed herein.

### Clinical outcomes:

Levels of hGDF-15 in human blood samples can be used to predict the probability of survival of a human melanoma patient.

Survival of patient groups can be analysed by methods known in the art, e.g. by Kaplan-Meier curves.

Appropriate time periods for the assessment of survival are known in the art and will be chosen by the skilled person with due regard to factors such as the respective stage of the melanoma.

For example, survival, preferably short-term survival, may, for instance, be predicted for time points of 6 months, 12 months and/or 18 months after the time point when the prediction was made. Alternatively, survival, preferably long-term survival, may, for instance, be assessed at a time point of 2 years, 3 years, 5 years and/or 10 years after the time point when the prediction was made.

### Predicting the probability of a positive clinical outcome according to the invention

For predicting the probability of a positive clinical outcome (i.e. survival) according to the invention, i.e. based on hGDF-15 levels, the methods for predicting, which are defined above in the preferred embodiments, are preferably used.

In order to practice the methods, statistical methods known in the art can be employed.

For instance, overall survival can be analyzed by Cox regression analysis.

Preferred statistical methods, which can be used to generate statistical models of patient data from clinical studies, are disclosed in Example 1. It is understood that the statistical methods disclosed in Example 1 are not limited to the particular features of Example 1 such as the melanoma stage, the particular threshold levels chosen and the particular statistical values obtained in the Example. Rather, these methods disclosed in Example 1 can generally be used in connection with any aspect of the disclosure.

### hGDF-15 levels

In an advantageous aspect of the invention, there is an inverse relationship between hGDF-15 levels and the probability of a positive clinical outcome, i.e. the probability of survival, in human melanoma patients. Thus, according to the invention, a decreased level of hGDF-15 indicates an increased probability of survival in human melanoma patients.

Thus, as used herein, terms such as "wherein a decreased level of hGDF-15 in said human blood sample indicates an increased probability of survival" mean that the level of hGDF-15 in said human blood sample and the probability of survival follow an inverse relationship. Thus, the higher the level of hGDF-15 in said human blood sample is, the lower is the probability of survival.

In connection with the methods for predicting according to the invention defined herein, hGDF-15 threshold levels are used.

The inverse relationship between hGDF-15 levels and the probability of survival applies to a threshold value as detailed in the appended claims.

Preferable hGDF-15 threshold levels are hGDF-15 serum levels as defined above in the preferred embodiments.

Alternatively, hGDF-15 threshold levels according to the present disclosure can be obtained, and/or further adjusted, by using the above-mentioned statistical methods, e.g. the methods of Example 1.

An hGDF-15 threshold level may be a single hGDF-15 threshold level. The disclosure also encompasses the use of more than one hGDF-15 threshold level, e.g. 2, 3, 4, 5, 6, 7, 8, 9, 10 or more hGDF-15 threshold levels.

For each single hGDF-15 threshold level of the one or more hGDF-15 threshold levels, a corresponding probability of survival can be predicted at a given time point.

hGDF-15 levels in blood samples can be measured by any methods known in the art. For instance, a preferred method of measuring hGDF-15 levels in blood samples including serum levels is a measurement of hGDF-15 levels by Enzyme-Linked Immunosorbent Assay (ELISA) by using antibodies to hGDF-15. Such ELISA methods are exemplified in Example 1, but can also include bead-based methods like the Luminex technology and others. Alternatively, hGDF-15 levels in blood samples including serum levels may be determined by known electrochemiluminesence immunoassays using antibodies to hGDF-15. For instance, the Roche Elecsys^{®} technology can be used for such electrochemiluminesence immunoassays. Other possible methods would include antibody-based detection from bodily fluids after separation of proteins in an electrical field.

The median hGDF-15 serum level of healthy human control individuals is < 0.8 ng/ml. The expected range is between 0.2 ng/ml and 1.2 ng/ml in healthy human controls (Reference: Tanno T et al.: "Growth differentiation factor 15 in erythroid health and disease." Curr Opin Hematol. 2010 May; 17(3): 184-190.).

According to the invention, hGDF-15 threshold levels are hGDF-15 serum levels as defined in the appended claims.

It is understood that for these hGDF-15 serum levels, and based on the disclosure of the invention provided herein, corresponding hGDF-15 levels in other blood samples can be routinely obtained by the skilled person (e.g. by comparing the relative level of hGDF-15 in serum with the respective level in other blood samples). Thus, the present disclosure also encompasses preferred hGDF-15 levels in plasma, whole blood and other blood samples, which correspond to each of the preferred hGDF-15 serum levels and ranges indicated above.

### Lactate dehydrogenase levels

Lactate dehydrogenase levels in blood samples can be measured by any methods known in the art Lactate dehydrogenase (LDH) levels are typically measured in enzymatic units (U). One unit will reduce 1.0 µmole of pyruvate to L-lactate per minute at pH 7.5 at 37°C.

### [L-Lactic Dehydrogenase]

Pyruvate + β-NADH → L-Lactate + β-NAD⁺

Lactate and NAD+ are converted to pyruvate and NADH by the action of LDH. NADH strongly absorbs light at 340 nm, whereas NAD+ does not. The rate of increase in absorbance at 340 nm is directly proportional to the LDH activity in the sample. Thus, LDH units are preferably determined by measuring absorbance at 340 nm.

Various clinically accepted diagnostic tests are available for the measurement of LDH levels. In accordance with the present embodiments, tests which can be applied to melanoma will be selected based on known clinical standards. Isoform-specific tests for LDH can be performed according to methods known in the art.

In a further advantageous aspect, there is also an inverse relationship between lactate dehydrogenase (LDH) levels and the probability of a positive clinical outcome, in particular the probability of survival, in human melanoma patients. Thus, in an embodiment according to the invention, a decreased level of lactate dehydrogenase indicates an increased probability of survival in melanoma patients.

Thus, as used herein, terms such as "wherein a decreased level of lactate dehydrogenase in said human blood sample indicates an increased probability of survival" mean that the level of lactate dehydrogenase in said human blood sample and the probability of survival follow an inverse relationship. Thus, the higher the level of lactate dehydrogenase in said human blood sample is, the lower is the probability of survival.

For instance, in connection with the methods for predicting according to the embodiments defined herein, lactate dehydrogenase threshold levels can be used.

According to the embodiments, the inverse relationship between lactate dehydrogenase levels and the probability of survival applies to any threshold value, and hence the embodiment is not limited to particular threshold values.

Alternatively, lactate dehydrogenase threshold levels according to the present disclosure can be obtained, and/or further adjusted, by using the above-mentioned statistical methods, e.g. the methods of Example 1.

A lactate dehydrogenase threshold level may be a single lactate dehydrogenase threshold level. The embodiment also encompasses the use of more than one lactate dehydrogenase threshold level, e.g. 2, 3, 4, 5, 6, 7, 8, 9, 10 or more lactate dehydrogenase threshold levels.

For each single lactate dehydrogenase threshold level of the one or more lactate dehydrogenase threshold levels, a corresponding probability of survival can be predicted.

According to the embodiment, preferable lactate dehydrogenase threshold levels are lactate dehydrogenase serum levels as defined above.

In a very preferred embodiment, the lactate dehydrogenase threshold level is a clinically accepted threshold level which distinguishes between normal and elevated LDH levels in patients. Such very preferred clinically accepted threshold levels are known in the art, and will be chosen by the skilled person with regard to the particular specifications of the LDH test.

It is understood that for these lactate dehydrogenase serum levels, and based on the disclosure provided herein, corresponding lactate dehydrogenase levels in other blood samples can be routinely obtained by the skilled person (e.g. by comparing the relative level of lactate dehydrogenase in serum with the respective level in other blood samples). Thus, the present embodiment also encompasses preferred lactate dehydrogenase levels in plasma, whole blood and other blood samples, which correspond to each of the preferred lactate dehydrogenase serum levels and ranges indicated above.

### S100B levels

In a further advantageous aspect, there is also an inverse relationship between S100B levels and the probability of a positive clinical outcome, in particular the probability of survival, in human melanoma patients. Thus, in an embodiment according to the invention, a decreased level of S100B indicates an increased probability of survival in melanoma patients.

Thus, as used herein, terms such as "wherein a decreased level of S100B in said human blood sample indicates an increased probability of survival" mean that the level of S100B in said human blood sample and the probability of survival follow an inverse relationship. Thus, the higher the level of S100B in said human blood sample is, the lower is the probability of survival.

For instance, in connection with the methods for predicting according to the embodiment defined herein, S100B threshold levels can be used.

According to the embodiment, the inverse relationship between S100B levels and the probability of survival applies to any threshold value, and hence the embodiment is not limited to particular threshold values.

S100B threshold levels according to the present disclosure can, for instance, be obtained, and/or further adjusted, by using the above-mentioned statistical methods, e.g. the methods of Example 1.

An S100B threshold level may be a single S100B threshold level. The embodiment also encompasses the use of more than one S100B threshold level, e.g. 2, 3, 4, 5, 6, 7, 8, 9, 10 or more S100B threshold levels.

In a very preferred embodiment, the S100B threshold level is a clinically accepted threshold level which distinguishes between normal and elevated S100B levels in patients. Such very preferred clinically accepted threshold levels are known in the art, and will be chosen by the skilled person with regard to the particular specifications of the S100B test.

For each single S100B threshold level of the one or more S100B threshold levels, a corresponding probability of survival can be predicted.

S100B levels in blood samples can be measured by any methods known in the art. Such methods include antibody-based assays. A preferred method of measuring S100B levels in blood samples a measurement of S100B serum levels by electrochemoluminescence assays, e.g. by using an Elecsys S100 electrochemiluminescence immunoassay. Further non-limiting examples of methods to measure S100B levels are given in Gongalves et al.: "Biological and methodological features of the measurement of S100B, a putative marker of brain injury." Clinical Biochemistry 41 (2008) 755-763).

### Antibodies capable of binding to hGDF-15 which can be used in accordance with the invention

The methods, and kits used in the methods of the invention may use one or more antibodies capable of binding to hGDF-15 or an antigen-binding portion thereof, as defined above.

It was previously shown that human GDF-15 protein can be advantageously targeted by a monoclonal antibody (WO2014/049087), and that such antibody has advantageous properties including a high binding affinity to human GDF-15, as demonstrated by an equilibrium dissociation constant of about 790pM for recombinant human GDF-15 (see Reference Example 1). Thus, in a preferred embodiment, the invention uses an antibody capable of binding to hGDF-15, or an antigen-binding portion thereof. Preferably, the antibody is a monoclonal antibody capable of binding to hGDF-15, or an antigen-binding portion thereof.

Thus, in a more preferred embodiment, the antibody capable of binding to hGDF-15 or antigen-binding portion thereof used in the methods of the invention is a monoclonal antibody capable of binding to human GDF-15, or an antigen-binding portion thereof, wherein the heavy chain variable domain comprises a CDR3 region comprising the amino acid sequence of SEQ ID NO: 5 or an amino acid sequence at least 90% identical thereto, and wherein the light chain variable domain comprises a CDR3 region comprising the amino acid sequence of SEQ ID NO: 7 or an amino acid sequence at least 85% identical thereto. In this aspect, preferably, the antibody or antigen-binding portion thereof comprises a heavy chain variable domain which comprises a CDR1 region comprising the amino acid sequence of SEQ ID NO: 3 and a CDR2 region comprising the amino acid sequence of SEQ ID NO: 4, and the antibody or antigen-binding portion thereof comprises a light chain variable domain which comprises a CDR1 region comprising the amino acid sequence of SEQ ID NO: 6, and a CDR2 region comprising the amino acid sequence ser-ala-ser.

Thus, preferably, the antibody capable of binding to hGDF-15 or antigen-bindinc portion thereof used in the methods of the invention is a monoclonal antibody capable of binding to human GDF-15, or an antigen-binding portion thereof, wherein the antibody or antigen-binding portion thereof comprises a heavy chain variable domain which comprises a CDR1 region comprising the amino acid sequence of SEQ ID NO: 3, a CDR2 region comprising the amino acid sequence of SEQ ID NO: 4 and a CDR3 region comprising the amino acid sequence of SEQ ID NO: 5, and wherein the antibody or antigen-binding portion thereof comprises a light chain variable domain which comprises a CDR1 region comprising the amino acid sequence of SEQ ID NO: 6, a CDR2 region comprising the amino acid sequence ser-ala-ser and a CDR3 region comprising the amino acid sequence of SEQ ID NO: 7.

In another embodiment in accordance with the above embodiments of the monoclonal antibody capable of binding to human GDF-15, or an antigen-binding portion thereof, the heavy chain variable domain comprises a region comprising an FR1, a CDR1, an FR2, a CDR2 and an FR3 region and comprising the amino acid sequence of SEQ ID NO: 1 or a sequence 85%, 90%, 91%, 92%, 93%, 94%, 95%, 96%, 97%, 98% or 99% identical thereto, and the light chain variable domain comprises a region comprising an FR1, a CDR1, an FR2, a CDR2 and an FR3 region and comprising the amino acid sequence of SEQ ID NO: 2 or a sequence 85%, 90%, 91%, 92%, 93%, 94%, 95%, 96%, 97%, 98% or 99% identical thereto.

In another embodiment in accordance with the above embodiments of the monoclonal antibody capable of binding to human GDF-15, or an antigen-binding portion thereof, the heavy chain variable domain comprises a CDR1 region comprising the amino acid sequence of SEQ ID NO: 3 and a CDR2 region comprising the amino acid sequence of SEQ ID NO: 4, and the light chain variable domain comprises a CDR1 region comprising the amino acid sequence of SEQ ID NO: 6 and a CDR2 region comprising the amino acid sequence of SEQ ID NO: 7. In a preferred aspect of this embodiment, the antibody may have CDR3 sequences as defined above

In another embodiment in accordance with the monoclonal antibody capable of binding to human GDF-15, or an antigen-binding portion thereof, the antigen-binding portion is a single-domain antibody (also referred to as "Nanobody^{™}"). In one aspect of this embodiment, the single-domain antibody comprises the CDR1, CDR2, and CDR3 amino acid sequences of SEQ ID NO: 3, SEQ ID NO: 4, and SEQ ID NO: 5, respectively. In another aspect of this embodiment, the single-domain antibody comprises the CDR1, CDR2, and CDR3 amino acid sequences of SEQ ID NO: 6, **ser-ala-ser,** and SEQ ID NO: 7, respectively. In a preferred aspect of this embodiment, the single-domain antibody is a humanized antibody.

Preferably, the antibodies capable of binding to human GDF-15 or the antigen-binding portions thereof have an equilibrium dissociation constant for human GDF-15 that is equal to or less than 100 nM, less than 20 nM, preferably less than 10 nM, more preferably less than 5 nM and most preferably between 0.1 nM and 2 nM.

In another embodiment in accordance with the above embodiments of the monoclonal antibody capable of binding to human GDF-15, or an antigen-binding portion thereof, the antibody capable of binding to human GDF-15 or the antigen-binding portion thereof binds to the same human GDF-15 epitope as the antibody to human GDF-15 obtainable from the cell line B1-23 deposited with the Deutsche Sammlung für Mikroorganismen und Zellkulturen GmbH (DMSZ) under the accession No. DSM ACC3142. As described herein, antibody binding to human GDF-15 in used in the methods of the present invention is preferably assessed by surface plasmon resonance measurements as a reference standard method, in accordance with the procedures described in Reference Example 1. Binding to the same epitope on human GDF-15 can be assessed similarly by surface plasmon resonance competitive binding experiments of the antibody to human GDF-15 obtainable from the cell line B1-23 and the antibody that is expected to bind to the same human GDF-15 epitope as the antibody to human GDF-15 obtainable from the cell line B1-23.

In a very preferred embodiment, the antibody capable of binding to human GDF-15 or the antigen-binding portion thereof is a monoclonal antibody capable of binding to human GDF-15, or an antigen-binding portion thereof, wherein the binding is binding to a conformational or discontinuous epitope on human GDF-15 comprised by the amino acid sequences of SEQ ID No: 25 and SEQ ID No: 26. In a preferred aspect of this embodiment, the antibody or antigen-binding portion thereof is an antibody or antigen-binding portion thereof as defined by the sequences of any one of the above embodiments.

In a further embodiment in accordance with the above embodiments, antibodies including the antibody capable of binding to human GDF-15 or the antigen-binding portion thereof can be modified, e.g. by a tag or a label.

A tag can, for instance, be a biotin tag or an amino acid tag. Non-limiting examples of such acid tag tags include Polyhistidin (His-) tags, FLAG-tag, Hemagglutinin (HA) tag, glycoprotein D (gD) tag, and c-myc tag. Tags may be used for various purposes. For instance, tags may be used to assist purification of the antibody capable of binding to human GDF-15 or the antigen-binding portion thereof. Preferably, such tags are present at the C-terminus or N-terminus of the antibody capable of binding to human GDF-15 or the antigen-binding portion thereof.

As used herein, the term "label" relates to any molecule or group of molecules which can facilitate detection of the antibody. For instance, labels may be enzymatic such as horseradish peroxidase (HRP), alkaline phosphatase (AP) or glucose oxidase. Enzymatically labelled antibodies may, for instance, be employed in enzyme-linked immunosorbent assays. Labels may also be radioactive isotopes, DNA sequences (which may, for instance, be used to detect the antibodies by polymerase chain reaction (PCR)), fluorogenic reporters and electrochemiluminescent groups (e.g. ruthenium complexes). As an alternative to labelling, antibodies used according to the invention, in particular an antibody capable of binding to human GDF-15 or the antigen-binding portion thereof, can be detected directly, e.g. by surface plasmon resonance measurements.

### Methods and Techniques

Generally, unless otherwise defined herein, the methods used in the present disclosure (e.g. cloning methods or methods relating to antibodies) are performed in accordance with procedures known in the art, e.g. the procedures described in Sambrook et al. ("Molecular Cloning: A Laboratory Manual.", 2nd Ed., Cold Spring Harbor Laboratory Press, Cold Spring Harbor, New York 1989), Ausubel et al. ("Current Protocols in Molecular Biology." Greene Publishing Associates and Wiley Interscience; New York 1992), and Harlow and Lane ("Antibodies: A Laboratory Manual" Cold Spring Harbor Laboratory Press, Cold Spring Harbor, New York 1988).

Binding of antibodies to their respective target proteins can be assessed by methods known in the art. The binding of monoclonal antibodies to their respective targets is preferably assessed by surface plasmon resonance measurements. These measurements are preferably carried out by using a Biorad ProteOn XPR36 system and Biorad GLC sensor chips, as exemplified for anti-human GDF-15 mAb-B1-23 in Reference Example 1.

Sequence Alignments of sequences according to the invention are performed by using the BLAST algorithm (see Altschul et al.(1990) "Basic local alignment search tool." Journal of Molecular Biology 215. p. 403-410.; Altschul et al.: (1997) Gapped BLAST and PSI-BLAST: a new generation of protein database search programs. Nucleic Acids Res. 25:3389-3402). Preferably, the following parameters are used: Max target sequences 10; Word size 3; BLOSUM 62 matrix; gap costs: existence 11, extension 1; conditional compositional score matrix adjustment. Thus, when used in connection with sequences, terms such as "identity" or "identical" refer to the identity value obtained by using the BLAST algorithm.

Monoclonal antibodies used in the methods of the invention can be produced by any method known in the art, including but not limited to the methods referred to in Siegel DL ("Recombinant monoclonal antibody technology." Transfus Clin Biol. 2002 Jan;9(1):15-22). In one embodiment, an antibody used in the methods of the invention is produced by the hybridoma cell line B1-23 deposited with the Deutsche Sammlung für Mikroorganismen und Zellkulturen GmbH (DSMZ) under the accession No. DSM ACC3142 under the Budapest treaty. The deposit was filed on September 29, 2011.

Levels of human GDF-15 (hGDF-15) can be measured by any method known in the art, including measurements of hGDF-15 protein levels by methods including (but not limited to) mass spectrometry for proteins or peptides derived from human GDF-15, Western Blotting using antibodies specific to human GDF-15, strip tests using antibodies specific to human GDF-15, or immunocytochemistry using antibodies specific to human GDF-15. A preferred method of measuring hGDF-15 serum levels is a measurement of hGDF-15 serum levels by Enzyme-Linked Immunosorbent Assay (ELISA) by using antibodies to GDF-15. Such ELISA methods are exemplified in Example 1. Alternatively, hGDF-15 serum levels may be determined by known electrochemiluminesence immunoassays using antibodies to hGDF-15. For instance, the Roche Elecsys^{®} technology can be used for such electrochemiluminesence immunoassays.

### Apparatuses

An apparatus can be an apparatus as defined in the appended claims which is configured to perform the methods of the invention.

As used herein, the term "configured to perform" means that the apparatus us specifically configured for the recited method steps. For instance, an apparatus configured to perform a method which uses a particular threshold level will be specifically configured to use that particular threshold.

The apparatus configured to perform the methods of the invention is an electrochemiluminescence analyzer such as Cobas^{®} analyzer. In this disclosure if LDH is measured, this may, for instance, be measured on an additional apparatus, which is not an electrochemiluminescence analyzer, and which is configured to perform LDH measurements such as enzymatic tests.

### Kits used in the invention

The invention also relates to uses of kits as defined in the appended claims.

The recombinant hGDF-15 contained in the kits may be present in a form which can conveniently be used for calibration purposes. For instance, it may be present in the form of stock solutions which cover several concentrations in the range of 0 to 15 ng/ml, e.g. at least one concentration in the range of 0-1 ng/ml, at least one concentration in the range of 1-3 ng/ml, at least one concentration in the range of 3-6 ng/ml, and preferably at least one further concentration in the range of 6-10 ng/ml, and more preferably further comprising at least one further concentration in the range of 10-15 ng/ml.

### Sequences

The amino acid sequences referred to in the present application are as follows (in an N-terminal to C-terminal order; represented in the one-letter amino acid code):
SEQ ID No: 1 (Region of the Heavy Chain Variable Domain comprising an FR1, a CDR1, an FR2, a CDR2 and an FR3 region from the Polypeptide Sequence of monoclonal anti-human GDF-15 mAb-B1-23): 
QVKLQQSGPGILQSSQTLSLTCSFSGFSLSTSGMGVSWIRQPSGKGLEWLAHIYWDDDKRYNPTLKSRLTISK
DPSRNQVFLKITSVDTADTATYYC
SEQ ID No: 2 (Region of the Light Chain Variable Domain comprising an FR1, a CDR1, an FR2, a CDR2 and an FR3 region from the Polypeptide Sequence of monoclonal anti-human GDF-15 mAb-B1-23): 
DIVLTQSPKFMSTSVGDRVSVTCKASQNVGTNVAWFLQKPGQSPKALIYSASYRYSGVPDRFTGSGSGTDFT
LTISNVQSEDLAEYFC
SEQ ID No: 3 (Heavy Chain CDR1 Region Peptide Sequence of monoclonal anti-human GDF-15 mAb-B1-23):
   GFSLSTSGMG
SEQ ID No: 4 (Heavy Chain CDR2 Region Peptide Sequence of monoclonal anti-human GDF-15 mAb-B1-23):
   IYWDDDK
SEQ ID No: 5 (Heavy Chain CDR3 Region Peptide Sequence of monoclonal anti-human GDF-15 mAb-B1-23):
   ARSSYGAMDY
SEQ ID No: 6 (Light Chain CDR1 Region Peptide Sequence of monoclonal anti-human GDF-15 mAb-B1-23):
   QNVGTN
Light Chain CDR2 Region Peptide Sequence of monoclonal anti-human GDF-15 mAb-B1-23:
   SAS
SEQ ID No: 7 (Light Chain CDR3 Region Peptide Sequence of monoclonal anti-human GDF-15 mAb-B1-23):
   QQYNNFPYT
SEQ ID No: 8 (recombinant mature human GDF-15 protein): 
GSARNGDHCPLGPGRCCRLHTVRASLEDLGWADWVLSPREVQVTMCIGACPSQFRAANMHAQIKTSLHRLK
PDTVPAPCCVPASYNPMVLIQKTDTGVSLQTYDDLLAKDCHCI
SEQ ID No: 9 (human GDF-15 precursor protein): 
MPGQELRTVNGSQMLLVLLVLSWLPHGGALSLAEASRASFPGPSELHSEDSRFRELRKRYEDLLTRLRANQS
WEDSNTDLVPAPAVRILTPEVRLGSGGHLHLRISRAALPEGLPEASRLHRALFRLSPTASRSWDVTRPLRRQL
SLARPQAPALHLRLSPPPSQSDQLLAESSSARPQLELHLRPQAARGRRRARARNGDHCPLGPGRCCRLHTV
RASLEDLGWADWVLSPREVQVTMCIGACPSQFRAANMHAQIKTSLHRLKPDTVPAPCCVPASYNPMVLIQKT
DTGVSLQTYDDLLAKDCHCI
SEQ ID No: 10 (human GDF-15 precursor protein + N-terminal and C-terminal GSGS linker): 
GSGSGSGMPGQELRTVNGSQMLLVLLVLSWLPHGGALSLAEASRASFPGPSELHSEDSRFRELRKRYEDLL
TRLRANQSWEDSNTDLVPAPAVRILTPEVRLGSGGHLHLRISRAALPEGLPEASRLHRALFRLSPTASRSWDV
TRPLRRQLSLARPQAPALHLRLSPPPSQSDQLLAESSSARPQLELHLRPQAARGRRRARARNGDHCPLGPG
RCCRLHTVRASLEDLGWADWVLSPREVQVTMCIGACPSQFRAANMHAQIKTSLHRLKPDTVPAPCCVPASY
NPMVLIQKTDTGVSLQTYDDLLAKDCHCIGSGSGSG
SEQ ID No: 11 (Flag peptide): DYKDDDDKGG
SEQ ID No: 12 (HA peptide): YPYDVPDYAG
SEQ ID No: 13 (peptide derived from human GDF-15): ELHLRPQAARGRR
SEQ ID No: 14 (peptide derived from human GDF-15): LHLRPQAARGRRR
SEQ ID No: 15 (peptide derived from human GDF-15): HLRPQAARGRRRA
SEQ ID No: 16 (peptide derived from human GDF-15): LRPQAARGRRRAR
SEQ ID No: 17 (peptide derived from human GDF-15): RPQAARGRRRARA
SEQ ID No: 18 (peptide derived from human GDF-15): PQAARGRRRARAR
SEQ ID No: 19 (peptide derived from human GDF-15): QAARGRRRARARN
SEQ ID No: 20 (peptide derived from human GDF-15): MHAQIKTSLHRLK
SEQ ID No: 25 (GDF-15 peptide comprising part of the GDF-15 Epitope that binds to B1-23):
   EVQVTMCIGACPSQFR
SEQ ID No: 26 (GDF-15 peptide comprising part of the GDF-15 Epitope that binds to B1-23):
   TDTGVSLQTYDDLLAKDCHCI

The nucleic acid sequences referred to in the present application are as follows (in a 5' to 3' order; represented in accordance with the standard nucleic acid code):
SEQ ID No: 21 (DNA nucleotide sequence encoding the amino acid sequence defined in SEQ ID No: 1): 
CAAGTGAAGCTGCAGCAGTCAGGCCCTGGGATATTGCAGTCCTCCCAGACCCTCAGTCTGACTTGTTCT
TTCTCTGGGTTTTCACTGAGTACTTCTGGTATGGGTGTGAGCTGGATTCGTCAGCCTTCAGGAAAGGGTC
TGGAGTGGCTGGCACACATTTACTGGGATGATGACAAGCGCTATAACCCAACCCTGAAGAGCCGGCTCA
CAATCTCCAAGGATCCCTCCAGAAACCAGGTATTCCTCAAGATCACCAGTGTGGACACTGCAGATACTGC
CACATACTACTGT
SEQ ID No: 22 (DNA nucleotide sequence encoding the amino acid sequence defined in SEQ ID No: 2): 
GACATTGTGCTCACCCAGTCTCCAAAATTCATGTCCACATCAGTAGGAGACAGGGTCAGCGTCACCTGCA
AGGCCAGTCAGAATGTGGGTACTAATGTGGCCTGGTTTCTACAGAAACCAGGGCAATCTCCTAAAGCACT
TATTTACTCGGCATCCTACCGGTACAGTGGAGTCCCTGATCGCTTCACAGGCAGTGGATCTGGGACAGA
TTTCACTCTCACCATCAGCAACGTGCAGTCTGAAGACTTGGCAGAGTATTTCTGT
SEQ ID No: 23 (DNA nucleotide sequence encoding the amino acid sequence defined in SEQ ID No: 5):
   GCTCGAAGTTCCTACGGGGCAATGGACTAC
SEQ ID No: 24 (DNA nucleotide sequence encoding the amino acid sequence defined in SEQ ID No: 7):
   CAGCAATATAACAACTTTCCGTACACG

### Examples

Reference Examples 1 to 3 exemplify an antibody to hGDF-15, which can be used in the methods, uses of kits, and in the apparatuses according to the invention. This hGDF-15 antibody is a monoclonal antibody which is known from WO 2014/049087.

### Reference Example 1: Generation and characterization of the GDF-15 Antibody B1-23

The antibody B1-23 was generated in a GDF-15 knock out mouse. Recombinant human GDF-15 (SEQ ID No: 8) was used as the immunogen.

The hybridoma cell line B1-23 producing mAb-B1-23 was deposited by the Julius-Maximilians-Universitat Würzburg, Sanderring 2, 97070 Würzburg, Germany, with the Deutsche Sammlung für Mikroorganismen und Zellkulturen GmbH (DMSZ) under the accession No. DSM ACC3142, in accordance with the Budapest Treaty.

By means of a commercially available test strip system, B1-23 was identified as an IgG2a (kappa chain) isotype. Using surface plasmon resonance measurements, the dissociation constant (Kd) was determined as follows:
Binding of the monoclonal anti-human-GDF-15 antibody anti-human GDF-15 mAb-B1-23 according to the invention was measured by employing surface plasmon resonance measurements using a Biorad ProteOn XPR36 system and Biorad GLC sensor chips:
For preparing the biosensors recombinant mature human GDF-15 protein was immobilized on flow cells 1 and 2. On one flow cell recombinant GDF-15 derived from Baculvirus-transfected insect cells (HighFive insect cells) and on the other recombinant protein derived from expression in E. coli was used. The GLC sensor chip was activated using Sulfo-NHS (N-Hydroxysulfosuccinimide) and EDC (1-Ethyl-3-[3-dimethylaminopropyl]carbodiimide hydrochloride) (Biorad ProteOn Amine Coupling Kit) according to the manufacturer's recommendation, the sensor surface was subsequently loaded with the proteins up to a density of about 600RU (1Ru = 1pg mm⁻²). The non-reacted coupling groups were then quenched by perfusion with 1M ethanolamine pH 8.5 and the biosensor was equilibrated by perfusing the chip with running buffer (10M HEPES, 150mM NaCl, 3.4mM EDTA, 0.005% Tween-20, pH 7.4, referred to as HBS150). As controls two flow cells were used, one empty with no protein coupled and one coupled with an non-physiological protein partner (human Interleukin-5), which was immobilized using the same coupling chemistry and the same coupling density. For interaction measurements anti-human GDF-15 mAb-B1-23 was dissolved in HBS150 and used in six different concentrations as analyte (concentration: 0.4, 0.8, 3, 12, 49 und 98 nM). The analyte was perfused over the biosensor using the one-shot kinetics setup to avoid intermittent regeneration, all measurements were performed at 25°C and using a flow rate of 100µl min⁻¹. For processing the bulk face effect and unspecific binding to the sensor matrix was removed by subtracting the SPR data of the empty flow cell (flow cell 3) from all other SPR data. The resulting sensogram was analyzed using the software ProteOn Manager version 3.0. For analysis of the binding kinetics a 1:1 Langmuir-type interaction was assumed. For the association rate constant a value of 5.4+0.06×10⁵ M⁻¹s⁻¹ (kₒₙ) and for the dissociation rate constant a value of 4.3+0.03×10⁻⁴ s⁻¹ (k_{off}) could be determined (values are for the interaction of anti-human GDF-15 mAb-B1-23 with GDF-15 derived from insect cell expression). The equilibrium dissociation constant was calculated using the equation K_{D} = k_{off}/kₒₙ to yield a value of about 790pM. Affinity values for the interaction of GDF-15 derived from E. coli expression and the anti-human GDF-15 mAb-B1-23 differ by less than a factor of 2, rate constants for GDF-15 derived from insect cells and E. coli deviate by about 45% and are thus within the accuracy of SPR measurements and likely do not reflect a real difference in affinity. Under the conditions used the anti-human GDF-15 mAb-B1-23 shows no binding to human interleukin-5 and thus confirms the specificity of the interaction data and the anti-human GDF-15 mAb-B1-23.

The amino acid sequence of recombinant human GDF-15 (as expressed in Baculovirus-transfected insect cells) is: 
GSARNGDHCP LGPGRCCRLH TVRASLEDLG WADWVLSPRE VQVTMCIGAC PSQFRAANMH
AQIKTSLHRL KPDTVPAPCC VPASYNPMVL IQKTDTGVSL QTYDDLLAKD CHCI
 (SEQ ID No: 8)

Thus, using surface plasmon resonance measurements, the dissociation constant (Kd) of 790pM was determined. As a comparison: the therapeutically used antibody Rituximab has a significantly lower affinity (Kd = 8nM).

It was previously shown that mAb B1-23 inhibits cancer cell proliferation *in vitro,* and that mAb B1-23 inhibits growth of tumors *in vivo* (WO2014/049087).

### Reference Example 2: mAb B1-23 recognizes a conformational or a discontinuous epitope of human GDF-15

Epitope Mapping: Monoclonal mouse antibody GDF-15 against 13mer linear peptides derived from GDF-15
Antigen: GDF-15:

GSGSGSGMPGQELRTVNGSQMLLVLLVLSWLPHGGALSLAEASRASFPGPSELHSEDSRFRELRKRYEDLL
TRLRANQSWEDSNTDLVPAPAVRILTPEVRLGSGGHLHLRISRAALPEGLPEASRLHRALFRLSPTASRSWDV
TRPLRRQLSLARPQAPALHLRLSPPPSQSDQLLAESSSARPQLELHLRPQAARGRRRARARNGDHCPLGPG

RCCRLHTVRASLEDLGWADWVLSPREVQVTMCIGACPSQFRAANMHAQIKTSLHRLKPDTVPAPCCVPASY
NPMVLIQKTDTGVSLQTYDDLLAKDCHCIGSGSGSG (322 amino acids with linker)(SEQ ID No: 10)

The protein sequence was translated into 13mer peptides with a shift of one amino acid. The C- and N-termini were elongated by a neutral GSGS linker to avoid truncated peptides (bold letters).

### Control Peptides:

Flag: DYKDDDDKGG (SEQ ID No:13), 78 spots; HA: YPYDVPDYAG (SEQ ID No:14), 78 spots (each array copy)
Peptide Chip Identifier:
   000264_01 (10/90, Ala2Asp linker)
Staining Conditions:
   Standard buffer: PBS, pH 7.4 + 0.05% Tween 20
   Blocking buffer: Rockland blocking buffer MB-070
   Incubation buffer: Standard buffer with 10% Rockland blocking buffer MB-070
   Primary sample: Monoclonal mouse antibody GDF-15 (1 µg/µl): Staining in incubation buffer for 16 h at 4°C at a dilution of 1:100 and slight shaking at 500 rpm
   Secondary antibody: Goat anti-mouse IgG (H+L) IRDye680, staining in incubation buffer with a dilution of 1:5000 for 30 min at room temperature (RT)
   Control antibodies: Monoclonal anti-HA (12CA5)-LL-Atto 680 (1:1000), monoclonal anti-FLAG(M2)-FluoProbes752 (1:1000); staining in incubation buffer for 1 h at RT
Scanner:
   Odyssey Imaging System, LI-COR Biosciences
   Settings: offset: 1mm; resolution: 21 µm; intensity green/red: 7/7
Results:
   After 30 min pre-swelling in standard buffer and 30 min in blocking buffer, the peptide array with 10, 12 and 15mer B7H3-derived linear peptides was incubated with secondary goat anti-mouse IgG (H+L) IRDye680 antibody only at a dilution of 1:5000 for 1h at room temperature to analyze background interactions of the secondary antibody. The PEPperCHIP^{®} was washed 2x1 min with standard buffer, rinsed with dist. water and dried in a stream of air. Read-out was done with Odyssey Imaging System at a resolution of 21 µm and green/red intensities of 7/7: We observed a weak interaction of arginine-rich peptides (ELHLRPQAARGRR (SEQ ID No:15), LHLRPQAARGRRR (SEQ ID No:16), HLRPQAARGRRRA (SEQ ID No:17), LRPQAARGRRRAR (SEQ ID No:18), RPQAARGRRRARA (SEQ ID No:19), PQAARGRRRARAR (SEQ ID No:20) and QAARGRRRARARN (SEQ ID No:21)) that are known as frequent binders, and with the basic peptide MHAQIKTSLHRLK (SEQ ID No:22) due to ionic interactions with the charged antibody dye.

After pre-swelling for 10 min in standard buffer, the peptide microarray was incubated overnight at 4 °C with monoclonal mouse antibody GDF-15 at a dilution of 1:100. Repeated washing in standard buffer (2x1 min) was followed by incubation for 30 min with the secondary antibody at a dilution of 1:5000 at room temperature. After 2x10 sec. washing in standard buffer and short rinsing with dist. water, the PEPperCHIP^{®} was dried in a stream of air. Read-out was done with Odyssey Imaging System at a resolution of 21 µm and green/red intensities of 7/7 before and after staining of control peptides by anti-HA and anti-FLAG(M2) antibodies.

It was shown that none of the linear 13mer peptides derived from GDF-15 interacted with monoclonal mouse antibody GDF-15 even at overregulated intensities. Staining of Flag and HA control peptides that frame the array, however, gave rise to good and homogeneous spot intensities.

### Summary:

The Epitope Mapping of monoclonal mouse GDF-15 antibody against GDF-15 did not reveal any linear epitope with the 13mer peptides derived from the antigen. According to this finding it is very likely that monoclonal mouse antibody GDF-15 recognizes a conformational or a discontinuous epitope with low affinity of partial epitopes. Due to the obvious absence of any GDF-15 signal above the background staining of the secondary antibody only, quantification of spot intensities with PepSlide^{®} Analyzer and subsequent peptide annotation were omitted.

### Reference Example 3: Structural identification of peptide ligand epitopes by mass spectrometric epitope excision and epitope extraction

The epitope of recombinant human GDF-15 which binds to the antibody B1-23 was identified by means of the epitope excision method and epitope extraction method (Suckau et al. Proc Natl Acad Sci USA. 1990 December; 87(24): 9848-9852.; R.Stefanescu et al., Eur.J.Mass Spectrom. 13, 69-75 (2007)).

For preparation of the antibody column, the antibody B1-23 was added to NHS-activated 6-aminohexanoic acid coupled sepharose. The sepharose-coupled antibody B1-23 was then loaded into a 0,8 ml microcolumn and washed with blocking and washing buffers.

### Epitope extraction experiment:

Recombinant human GDF-15 was digested with trypsin for 2h at 37°C (in solution), resulting in different peptides, according to the trypsin cleavage sites in the protein. After complete digestion, the peptides were loaded on the affinity column containing the immobilized antibody B1-23. Unbound as well as potentially bound peptides of GDF-15 were used for mass spectrometry analysis. An identification of peptides by means of mass spectrometry was not possible. This was a further indicator that the binding region of GDF-15 in the immune complex B1-23 comprises a discontinuous or conformational epitope. In case of a continuous linear epitope, the digested peptides should bind its interaction partner, unless there was a trypsin cleavage site in the epitope peptide. A discontinuous or conformational epitope could be confirmed by the epitope excision method described in the following part.

### Epitope excision experiment:

The immobilized antibody B1-23 on the affinity column was then incubated with recombinant GDF-15 for 2h. The formed immune complex on the affinity column was then incubated with trypsin for 2h at 37°C. The cleavage resulted in different peptides derived from the recombinant GDF-15. The immobilized antibody itself is proteolytically stable. The resulting peptides of the digested GDF-15 protein, which were shielded by the antibody and thus protected from proteolytic cleavage, were eluted under acidic conditions (TFA, pH2), collected and identified by mass spectrometry.

The epitope excision method using MS/MS identification resulted in the following peptides:

| Peptide | Position in sequence | Mass | Ion/Charge |
|---|---|---|---|
| EVQVTMCIGACPSQFR (SEQ ID No: 25) | 40-55 | 1769.91 | 590.50(3+) |
| TDTGVSLQTYDDLLAKDCHCI (SEQ ID No: 26) | 94-114 | 2310,96 | 771:33(3+) |

The part of human GDF-15, which binds the antibody B1-23, comprises a discontinuous or conformational epitope. Mass spectrometry identified 2 peptides in the GDF-15 protein, which are responsible for the formation of the immune complex. These peptides are restricted to the positions 40-55 (EVQVTMCIGACPSQFR) and 94-114 (TDTGVSLQTYDDLLAKDCHCI) in the GDF-15 amino acid sequence. Thus, these two peptides comprise an epitope of the GDF-15 protein that binds to the antibody B1-23.

### Example 1:

### Patients, Materials and Methods

### Patients

Patients from the Department of Dermatology, University of Tübingen, Germany, with histologically confirmed melanoma were identified in the Central Malignant Melanoma Registry (CMMR) database which prospectively records patients from more than 60 dermatological centers in Germany. 761 patients, with (a) archived serum samples taken between January 2008 and February 2012, (b) available follow-up data, and (c) history or presence of loco regional or distant metastasis at the time point of blood draw were selected. The aims and methods of data collection by the CMMR have previously been published in detail (Lasithiotakis, KG et al., Cancer / 107 / 1331-9. 2006). Data obtained for each patient included age, gender, the date of the last follow-up, and the date and cause of death, if applicable. All patients had given written informed consent to have clinical data recorded by the CMMR registry. The institutional ethics committee Tübingen has approved the study (ethic vote 125/2015BO2). Age, the pattern of distant metastasis (stage IV patients only), sub-stage (IIIA vs. IIIB vs. IIIC; stage III patients only) according to the AJCC classification (Balch, CM et al., J Clin Oncol / 27 / 6199-206. 2009), serum LDH and serum S100B (Elecsys S100 electrochemiluminescence immunoassay; Roche Diagnostics AG, Rotkreuz, Switzerland) were evaluated at the time of serum sampling. hGDF-15 serum concentrations were quantified in duplicates using a commercial ELISA kit according to the manufacturer's instructions (R&D systems, Wiesbaden, Germany):

### Analysis of hGDF-15 Serum Levels by Enzyme-Linked Immunosorbent Assay (ELISA):

Human GDF-15 serum levels were measured by Enzyme-Linked Immunosorbent Assay (ELISA).

Buffers and reagents:
Blocking solution: 1% BSA (fraction V pH 7.0, PAA) in PBS
Wash solution: PBS-Tween (0.05%)
Standard: human GDF-15 (stock concentration 120 µg/ml, from R&D Systems)
Capture antibody: Human GDF-15 MAb (Clone 147627) from R&D Systems, Mouse IgG2B (catalog #MAB957, from R&D Systems, stock concentration 360 µg/ml)
Detection antibody: Human GDF-15 Biotinylated Affinity Purified PAb, Goat IgG (catalog #BAF940, from R&D Systems, stock concentration 9 pl/ml)
Streptavidin-HRP (Catalog #DY998, from R&D Systems)
Substrate solution: 10 ml 0.1 M NaOAc pH6.0 + 100 µl TMB + 2 µl H₂O₂
Stop solution: 1 M H₂SO₄

Analysis Procedure:
1. Plate Preparation:
a. The capture antibody was diluted to the working concentration of 2 µg/ml in PBS. A 96-well microplate (Nunc maxisorp^{®}) was immediately coated with 50 µl per well of the diluted capture antibody excluding the outer rows (A and H). Rows A and H were filled with buffer to prevent evaporation of the samples during the experiment. The plate was gently tapped to ensure that the bottom of each well was thoroughly covered. The plate was placed in a humid chamber and incubated overnight at room temperature (RT).
b. Each well was aspirated and washed three times with PBS-Tween (0.05%).
c. 150 µl of blocking solution was added to each well, followed by incubation at RT for 1 hour.
d. Each well was aspirated and washed three times with PBS-Tween (0.05%).

2. Assay Procedure:
a. Standards were prepared. GDF-15 was diluted in buffered blocking solution to a final concentration of 1 ng/ml (4.17 µl GDF + 496 µl buffered blocking solution). 1:2 serial dilutions were made.
b. Duplicate samples 1:20 (6 µl + 114 µl buffered blocking solution) were prepared.
c. 50 µl of diluted samples or standards were added per well, followed by incubation for 1 hour at RT.

| | 1 | 2 | 3 | 4 | 5 | 6 | 7 | 8 | 9 | 10 | 11 | 12 |
|---|---|---|---|---|---|---|---|---|---|---|---|---|
| A | 0 | 0 | 0 | 0 | 0 | 0 | 0 | 0 | 0 | 0 | 0 | 0 |
| B | s1 | s2 | ... | | | | | | | | | s12 |
| C | s1 | s2 | ... | | | | | | | | | s12 |
| D | s13 | s14 | ... | | | | | | | | | s24 |
| E | s13 | s14 | ... | | | | | | | | | s24 |
| F | St | and | ard | | | | | dil | uti | on | s | |
| G | | | | | se | rial | | | | | | |
| H | 0 | 0 | 0 | 0 | 0 | 0 | 0 | 0 | 0 | 0 | 0 | 0 |

a. Each well was aspirated and washed three times with PBS-Tween (0.05%).
b. The detection antibody was diluted to a final concentration of 50 ng/ml (56 µl + 10 ml buffered blocking solution). 50 µl of the diluted detection antibody was added to each well, followed by incubation for 1 hour at RT.
c. Each well was aspirated and washed three times with PBS-Tween (0.05%).
d. Streptavidin-HRP was diluted 1:200 (50 µl + 10 ml blocking buffer). 50 µL of the working dilution of Streptavidin-HRP was added to each well, followed by incubation for 20 min at RT.
e. Each well was aspirated and washed three times with PBS-Tween (0.05%).
f. The substrate solution was prepared. 50 µL of substrate solution was added to each well, followed by incubation for 20 min at RT.
g. 50 µL of stop solution was added to each well.
h. The optical density of each well was determined immediately, using a microplate reader set to 450 nm.

3. Calculation of GDF-15 serum titer:
a. Each sample/GDF-15 standard dilution was applied in duplicate. To determine GDF-15 titer, the average of the duplicates was calculated and the background (sample without GDF-15) subtracted.
b. To create a standard curve, values from the linear range were plotted on an X-Y-diagram (X axis: GDF-15 concentration, Y axis: OD450), and a linear curve fit was applied. GDF-15 serum titer of the test samples was calculated by interpolating from the OD450 values of the standard dilutions with known concentration.
c. To calculate the final GDF-15 concentration of the samples, the distinct dilution factor was considered. Samples yielding OD values below or above the standard range were reanalyzed at appropriate dilutions.

### Statistical analysis

Follow-up time for survival analysis was defined from the date of blood sampling to the last follow-up or death. Cumulative survival probabilities according to Kaplan-Meier were calculated together with 95% confidence intervals (Cls) and compared using two-sided log-rank test statistics. For the analysis of OS, patients who were alive at the last follow-up were censored while patients who had died were considered an "event". To analyze the impact of sGDF-15 on OS, patients were randomly assigned to two cohorts using a 1:2 ratio (identification and validation cohort, respectively). In the identification cohort different cut-off points were applied to categorize patients according to sGDF-15 into two balanced groups comprising ≥25% of patients each. Differences in OS between patients with high vs. low sGDF-15 were analyzed for each cut-off point and the one resulting in the lowest log rank p-value was selected, similarly to optimization algorithms published earlier (Camp, RL et al., Clin Cancer Res / 10 / 7252-9. 2004). The optimal cut-off point as defined in the identification cohort was thereafter analyzed in 507 patients of validation cohort.

Cox proportional hazard regression analysis was used to calculate the relative effect considering additional prognostic factors in the entire patient cohort. Age was dichotomized according to the median age of patients. Serum S100B levels and sLDH were categorized as elevated vs. normal according to cut-off values as used in clinical routine (upper limit of normal 0.10 µg/l and 250 U/I, respectively). Patients with missing values were excluded from regression analysis. Results of the models were described by means of hazard ratios; p-values were based on the Wald test. All statistical analyses were carried out using the SPSS Version 22 (IBM SPSS, Chicago, Illinois, USA).

### Results

### Patients

Patients' characteristics are shown in Table 1. A total of 761 melanoma patients (52.0% male) was analyzed. The median age was 63 years. The median follow-up for patients who died was 10.3 months and 45.3 months for patients who were censored.

Stage IV patients (n=293) were assigned to the M-categories M1c (n=206; 70.3%), M1b (n=51; 17.4%), or M1a (n=36; 12.3%) based on the site of distant metastases and on serum LDH (sLDH) (Balch, CM et al., J Clin Oncol / 27 / 6199-206. 2009). The median survival estimate according to Kaplan Meier was 10.7 months. Survival probabilities were 46.4% at 1-year, 33.3% at 2-years, and 29.3% at 3-years.

A total of 468 stage III patients was included. Sub-stage was IIIA in 15.6%, IIIB in 37.2%, and IIIC in 47.2% of 422 patients with complete data for classification. Survival probabilities were 94.9% at 1-year, 85.0% at 2-years, and 72.8% at 5-years, respectively.

The median hGDF-15 serum concentration was 1.0 ng/mL considering all 761 patients (0.9 ng/mL for stage III vs. 1.5 ng/mL for stage IV patients). Mean sGDF-15 was 2.6 (1.1 ng/mL for stage III vs. 4.8 ng/mL for stage IV patients; p<0.001).

### Overall Survival according to hGDF-15 levels

Thirteen different cut-off points ranging from 0.7 ng/mL to 1.9 ng/mL at increments of 0.1 ng/mL were found to categorize patients of the identification cohort (n=254) according to sGDF-15 into two balanced groups (the smaller group had to comprise at least 25% of patients). The difference in prognosis was largest comparing 86 patients (33.9%) with hGDF-15 levels ≥1.5ng/mL and poor OS to 168 (66.1%) patients with lower levels and favorable OS (p<0.001; Figure 4A). The difference in OS applying this cut-off point for sGDF-15 was thereafter confirmed in the validation cohort (n=508; p<0.001; Figure 4B).

This inverse correlation between sGDF-15 and OS was observed in tumor-free stage III patients and in unresectable stage IV patients (Figure 1A; 1B) but not in tumor-free stage IV patients (Figure 1C) considering all patients (both cohorts combined).

Considering stage III patients of both cohorts, the 1-, 2- and 5 year survival probability was 96.1%, 87.8% and 75.7% for those with sGDF-15 below 1.5 ng/mL (n=369, 78.8% of all stage III patients) but only 90.4%, 74.2% and 61.5% for patients with higher sGDF-15 (n=99, 21.2%) (Table 2).

For patients of both cohorts with unresectable distant metastases and high hGDF-15 levels the probability to survive one year after analysis was only 14.3%, but 45.0% for patients with low sGDF-15. Similarly, the 2-year and 5-year survival was 6.3% and 2.6% compared to 19.9% and 5.2%, respectively. Median survival was 5.7 months versus 11.0 months for unresectable stage IV patients with high and low sGDF-15, respectively (Table 3).

**Table 1: Patient characteristics**

| **Factor** | **Category** | **Stage III (n=468)** | | **Stage IV tumor-free (n=87)** | | **Stage IV unresectable (n=206)** | | **Total (n=761)** | |
|---|---|---|---|---|---|---|---|---|---|
| | | N | (%) | N | (%) | N | (%) | N | (%) |
| Gender | Male | 228 | 48.7 | 47 | 54.0 | 121 | 58.7 | 396 | 52.0 |
| | Female | 240 | 51.3 | 40 | 46.0 | 85 | 41.3 | 365 | 48.0 |
| Age | ≤ 50 years | 120 | 25.6 | 16 | 18.4 | 57 | 27.7 | 193 | 25.4 |
| | 51 - 60 years | 82 | 17.5 | 16 | 18.4 | 56 | 27.2 | 154 | 20.2 |
| | 61 - 70 years | 117 | 25.0 | 28 | 32.2 | 38 | 18.4 | 183 | 24.0 |
| | ≥ 71 years | 149 | 31.8 | 27 | 31.0 | 55 | 26.7 | 231 | 30.4 |
| | Median age | 64 years | | 66 years | | 59 years | | 63 years | |
| Stage (AJCC 2009) | IIIA | 66 | 15.6 | | | | | 66 | 9.2 |
| | IIIB | 157 | 37.2 | | | | | 157 | 22.0 |
| | IIIC | 199 | 47.2 | | | | | 199 | 27.8 |
| | Stage III - unknown sub stage | 46 | | | | | | 46 | |
| | IV, M1a | | | 23 | 26.4 | 13 | 6.3 | 36 | 5.0 |
| | IV, M1b | | | 21 | 24.1 | 30 | 14.6 | 51 | 7.1 |
| | IV, M1c | | | 43 | 49.4 | 163 | 79.1 | 206 | 28.8 |
| S100B | Normal | 409 | 87.8 | 74 | 89.2 | 65 | 33.7 | 548 | 73.9 |
| | Elevated | 57 | 12.2 | 9 | 10.8 | 128 | 66.3 | 194 | 26.1 |
| | Unknown | 2 | | 4 | | 13 | | 19 | |
| LDH | Normal | 439 | 94.2 | 81 | 97.6 | 116 | 57.1 | 636 | 84.6 |
| | Elevated | 27 | 5.8 | 2 | 2.4 | 87 | 42.9 | 116 | 15.4 |
| | Unknown | 2 | | 4 | | 3 | | 9 | |
| Visceral involvement | Soft tissue only | | | 23 | 26.4 | 22 | 10.7 | 45 | 5.9 |
| | Lung | | | 21 | 24.1 | 36 | 17.5 | 57 | 7.5 |
| | Other organs | | | 43 | 49.4 | 148 | 71.8 | 191 | 25.1 |
| | None (stage III) | 468 | | | | | | 468 | 61.5 |

| | | | | | | | | | |
|---|---|---|---|---|---|---|---|---|---|
| AJCC: American Joint Committee on Cancer; LDH: lactate dehydrogenase | | | | | | | | | |

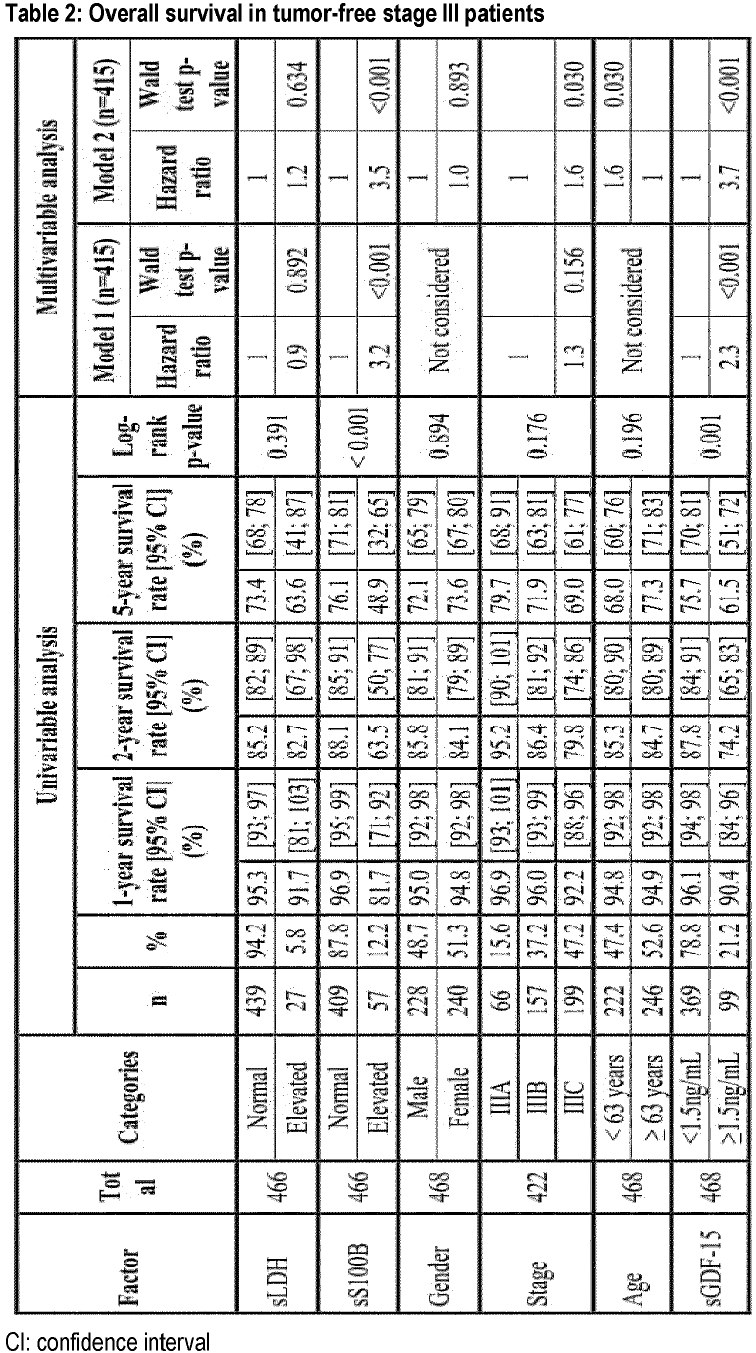

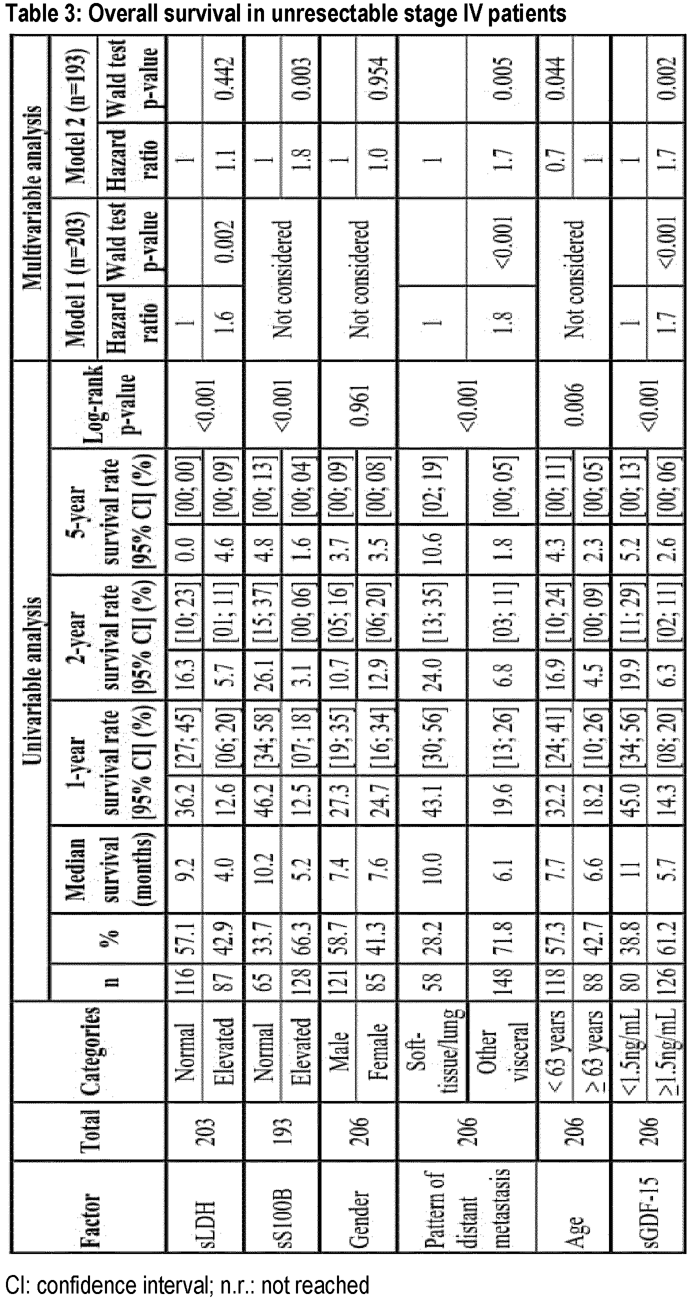

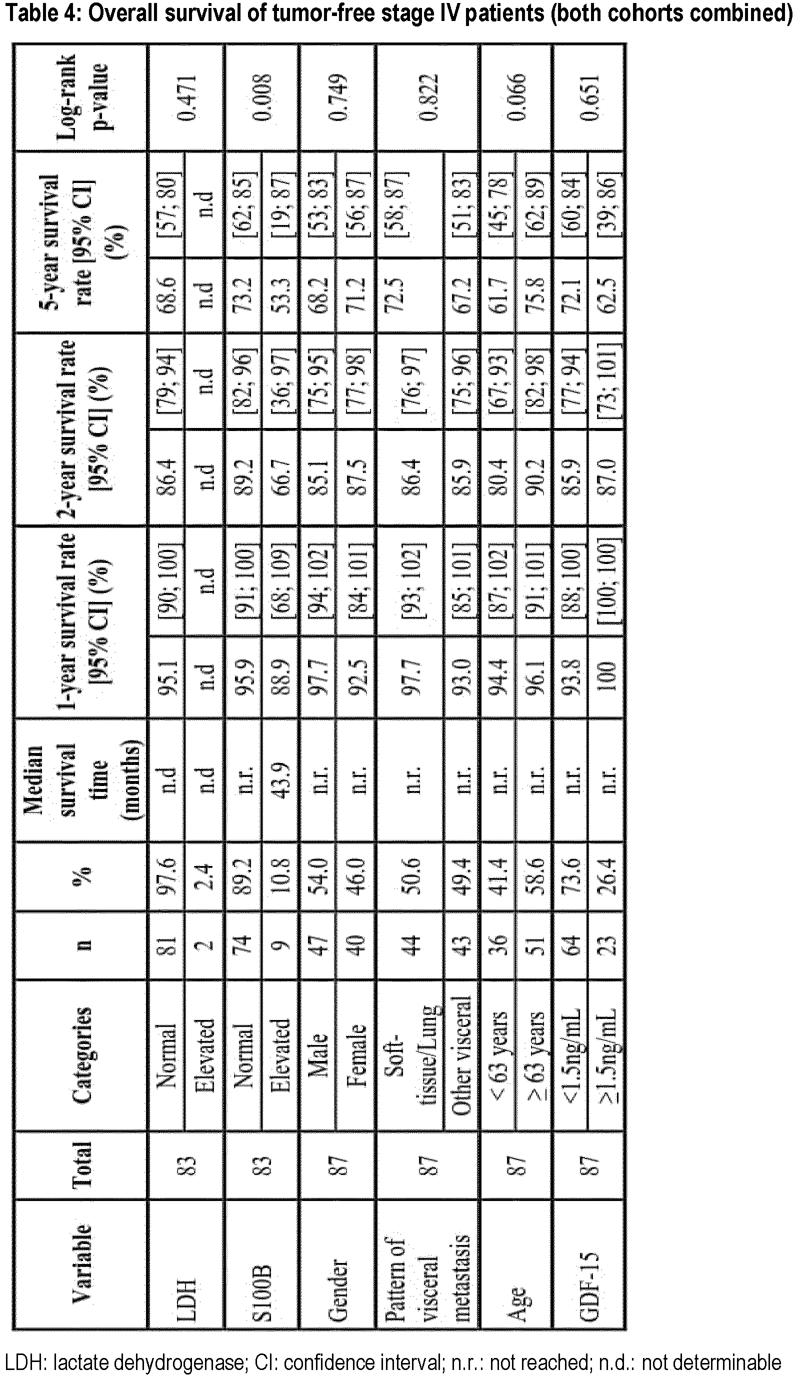

### The relative prognostic impact of sGDF-15 in stage III patients

Cox regression analyses of the entire cohort of stage III patients were performed to determine the relative impact of sGDF-15 compared to other prognostic factors (Table 3). In the first model the three biomarkers sGDF-15, sS100B, and sLDH were included to allow for direct comparison. Results were adjusted for sub-stage, as univariate analysis had revealed a trend towards better OS for sub-stages IIIA/B versus IIIC (p=0.088). In addition to elevated sGDF-15 (HR 2.3; p<0.001), elevated sS100B was strongly associated with poor OS (Figure 5A) and had independent negative impact on prognosis (HR 3.2; p<0.001) in multivariable analysis. One year survival rates were highest with 97.4% for patients with favorable results in both biomarkers in strong contrast to 56.2% for those with both markers elevated. The survival probabilities after one year of patients with either elevated sS100B or high sGDF-15 were 88.4% or 95.1%, respectively. In the second model age and gender were additionally considered. Here, stage IIIC and age>63 years had independent negative impact on prognosis in addition to sGDF15 and sS100B. The number of unfavorable values considering those 4 factors was strongly associated with survival (Figure 5). As expected for stage III melanoma, sLDH showed no correlation with outcome in neither model.

### The relative impact of hGDF-15 levels in stage IV patients

In stage IV patients without evidence of disease at the time point of blood sampling (n=87), no prognostic relevance was observed for sGDF-15. Neither the pattern of distant metastasis, nor sLDH were associated with OS (Table 4). Instead, sS100B was the only prognostic factor in this patient population (Figure 5C).

Looking at 203 thoroughly characterized stage IV melanoma patients with unresectable tumor burden, we applied Cox regression analysis to investigate the relative prognostic impact of sGDF-15 compared to other factors. In the first model, sGDF-15 was compared to the pattern of distant metastases and sLDH, which are both considered as prognostic factors in the AJCC classification (Table 3). Like in stage III melanoma, sGDF-15 had a strong independent impact on OS (HR 1.7; p<0.001) in conjunction with the pattern of distant metastases (HR 1.8; p<0.001) and sLDH (HR 1.6; p=0.002). The independent impact of sGDF-15 levels was evident both in M1a/b (Figure 3A) and in M1c patients (Figure 3B). The number of unfavorable values considering the three independent factors sLDH, sGDF-15 and the pattern of distant metastasis was strongly associated with OS (Figure 3C). Thereby, 47% of patients fell into a newly identified subgroup with an extremely poor (3.3%) probability to survive 1 year. The multivariate model 2 considered all analyzed variables (Table 3). Here, sS100B replaced sLDH as significant prognostic parameter and age had additional independent impact. Stratification according to the number of unfavorable factors considering sS100B, the M-category, hGDF-15, and age allowed identification of an 8% sub-group of patients with favorable prognosis and 1-year OS of 81.3%. In contrast, 16% of patients showing unfavorable values in all 4 independent factors had the poorest prognosis with a 1-year OS of 3.2% (Figure 7).

### Example 2: Alternative Evaluation of the Patient Samples described in Example 1

As an alternative Example in accordance with the invention, the same patient samples, which were already described in Example 1, were evaluated in an alternative manner, as described in the following:

### Patients, Materials and Methods:

### Patients

Patients from the Department of Dermatology, Tübingen, Germany, with histologically confirmed melanoma were identified in the Central Malignant Melanoma Registry (CMMR) database (Lasithiotakis et al., 2006). 761 patients, with (a) archived serum samples taken between January 2008 and February 2012, (b) available follow-up data, and either (c) history of loco-regional or (d) history or presence of distant metastasis at the time point of blood draw were selected. Serum used for analysis of sGDF-15 was sampled during routine blood draws for analysis of sS100B stage was defined according to the AJCC classification (Balch et al., 2009), serum LDH and serum S100B (Elecsys S100 electrochemiluminescence immunoassay; Roche Diagnostics, Rotkreuz, Switzerland) were categorized as elevated vs. normal according to cut-off values used in clinical routine (upper limits of normal 0.10 µg/l and 250 U/I, respectively). Distant soft tissue/lymph nodes, lung, brain, liver, bone, and other visceral organs were considered for the calculation of the number of involved distant sites. Thus the number could be between 1 and 6 for each stage IV patient. GDF-15 serum concentrations were quantified in duplicates using a commercial ELISA kit according to the manufacturer's instructions (R&D systems, Wiesbaden, Germany).

All patients had given written informed consent to have clinical data recorded by the CMMR registry. The institutional ethics committee Tübingen has approved the study (ethic vote 125/2015BO2).

### Statistical analysis

Follow-up time was defined from the date of blood sampling to the last follow-up or death. Survival probabilities according to Kaplan-Meier were calculated together with 95% confidence intervals and compared using two-sided log-rank tests. Patients who were either alive at the last follow-up or died from reasons other than melanoma were censored. Patients were randomly assigned to two cohorts using a 1:2 ratio. In the identification cohort, differences in OS between patients with high vs. low sGDF-15 were analyzed for cut-off points which yield two balanced groups comprising ≥25% of patients each. Then, the cut-off point resulting in the lowest log rank p-value was selected, similar to optimization algorithms published earlier (Camp et al., 2004) and thereafter analyzed in the validation cohort.

Cox regression analysis was used excluding patients with missing values. Results of the multivariable models were described by means of HRs; p-values were based on the Wald test. Combination models were developed using the nomogram function in the survival package for R. Differences in sGDF-15 according to prior systemic treatments were analyzed by Mann-Whitney U Testing. All statistical analyses were carried out using SPSS Version 22 (IBM SPSS, Chicago, Illinois, USA) and R 3.2.1 (R Foundation for Statistical Computing, Vienna Austria).

### Results:

### Patients

Patients' characteristics are shown in Table 5. A total of 761 melanoma patients was analyzed. The median follow-up for patients who died was 10.3 months and 45.3 months for patients who were alive at the time point of last follow-up.

Stage IV patients (n=293) were assigned to the M-categories M1c (n=206; 70.3%), M1b (n=51; 17.4%), or M1a (n=36; 12.3%). The median survival estimate according to Kaplan Meier was 10.7 months. Survival probabilities were 46.4% at 1 year, 33.3% at 2 years, and 29.3% at 3 years. Assessment for stage IV patients was within 12 weeks in 84 (28.7%), or within 12 months after first occurrence of distant metastasis in 96 (32.7%), or at later time points in 113 patients (38.6%). At the respective time-point 87 patients (29.7%) had no evidence of disease while 206 (70.3%) had unresectable tumor.

A total of 468 stage III patients was included. Sub-stage was IIIA in 15.6%, IIIB in 37.2%, and IIIC in 47.2% of 422 patients with complete data for classification. Survival probabilities were 94.9% at 1-year, 85.0% at 2-years, and 72.8% at 5-years, respectively. The time point of assessment was within 12 weeks for 55 patients (11.8%), within 12 months after first occurrence of loco regional metastasis for 100 (21.4%), or later for 313 patients (66.9%). None of the stage III patients had evidence of disease at the respective time point.

### GDF-15 serum levels according to stage, tumor burden and prior treatments

Median sGDF-15 was 1.0 ng/mL considering all 761 patients (0.9 ng/mL for stage III vs. 1.5 ng/mL for stage IV patients). Stage IV patients with clinical or radiologic evidence of tumor had higher median sGDF-15 (2.1 ng/mL) than tumor-free stage IV or tumor-free stage III patients (both 0.9 ng/mL; Figure 10A). Among tumor-free stage IV patients, median sGDF-15 was not different between 13 patients who had ongoing complete responses after systemic treatments and 74 patients who were tumor-free after metastasectomy of distant metastases (both 0.9 ng/mL). sGDF-15 correlated with sLDH and the number of involved distant sites in unresectable stage IV patients (Figure 10B and 10C). In general, median sGDF-15 was not different in patients who had received systemic treatment within the last 4 weeks or any time before blood sampling (Table 8). A separate analysis about the impact of pre-treatment with chemotherapy, ipilimumab, other immunotherapy, BRAF/MEK inhibitors, or other systemic treatments showed lower sGDF-15 after BRAF/MEK inhibitors and a trend towards higher levels after ipilimumab in unresectable stage IV patients. No significant impact of prior systemic treatments was observed in tumor-free stage IV patients. A small but significant difference in sGDF-15 was observed comparing tumor-free stage III patients who had prior adjuvant treatment with Interferon-a to those without (0.8 ng/mL vs. 0.9 ng/mL; Table 8).

### Overall survival according to GDF-15 levels

Thirteen different cut-off points of sGDF-15 ranging from 0.7 ng/mL to 1.9 ng/mL were tested in the identification cohort (n=254). The most significant difference in prognosis was observed when 86 patients (33.9%) with sGDF-15 ≥1.5 ng/mL and poor OS were compared to 168 (66.1%) patients with lower levels and favorable OS (p<0.001; Figure 11A). The difference in OS using this cut-off point was thereafter confirmed in the validation cohort (n=507; p<0.001; Figure 11B). A comparison of patient characteristics between the identification and the validation cohorts is provided in Table 9.

This inverse correlation between sGDF-15 and OS was observed in tumor-free stage III patients and in unresectable stage IV patients (Figure 1A; 1B) but not in tumor-free stage IV patients (Figure 1C) considering patients of both cohorts.

Among stage III patients, the 1-, 2- and 5-years OS probability was 96.1%, 87.8% and 75.7% for those with sGDF-15 <1.5 ng/mL but only 90.4%, 74.2% and 61.5% for patients with higher sGDF-15 (Table 6 and Table 10). The association with OS was significant for patients who had been tumor-free for up to 6 months before serum sampling, or for 6 to 24 months. No difference in OS was observed for patients, who had been tumor-free for more than 24 months (Figure 12).

For patients with unresectable distant metastases and sGDF-15 ≥1.5 ng/mL the 1-year OS probability was only 14.3%, but 45.0% for those with low sGDF-15. Similarly, the 2-year and 5-year survival was 6.3% and 2.6% compared to 19.9% and 5.2%, respectively (Table 7 and Table 11). The association with OS was significant for patients whose assessment was within 6 months and between 6 and 24 months after first diagnosis of distant metastasis but not for those, who had been in stage IV for more than 24 months (Figure 13).

### The relative prognostic impact of sGDF-15 in stage III patients

Cox regression analysis of all tumor-free stage III patients was performed to determine the relative impact of sGDF-15 compared to other prognostic factors. The hazard ratio (HR) was 2.2 (p<0.001) for patients with sGDF15 ≥1.5 ng/mL when adjusted for the sub-stage according to American Joint Committee on Cancer (Table 6; model 1). In model 2, which considered a broad spectrum of factors, elevated sS100B was strongly associated with poor OS (Figure 14A) and had independent negative impact on OS (HR 4.0; p<0.001) in addition to elevated sGDF-15 (HR 2.7; p<0.001) and the pattern of loco-regional metastasis (HR=4.1; p<0.001 for combined lymph-node and intransit/satellite involvement, HR=2.4; p=0.002 for lymph-node involvement only; Table 6). To obtain an individual risk score, a nomogram accounting for the relative impact of these three factors was developed (Figure 8A). Two years OS was 96.1% for patients without lymph node involvement, normal sS100B, and sGDF-15 <1.5 ng/mL (risk score 0), but only 40.2% for those with a risk score >175 (Figure 8B). No significant associations with OS were observed for age, gender, sLDH, sub-stage, ulceration, or tumor thickness. OS was not different between patients who received prior adjuvant systemic treatments compared to those without (Table 6). A similar impact of sGDF-15 on OS was observed, if the analysis was limited to stage III patients of the validation cohort (Table 12).

### The relative impact of GDF-15 levels in stage IV patients

sGDF-15 had independent impact on OS among the entire cohort of stage IV patients (n=293). As expected, a prominent impact of the disease status at the time-point of serum sampling was observed (unresectable disease HR=8.6; p<0.001 vs. tumor-free; Table 13). Thus, unresectable stage IV patients and those which were tumor-free after metastasectomy or complete responses upon prior systemic treatments were analyzed separately.

In tumor-free stage IV patients (n=87), no impact on OS was observed for sGDF-15 (Table 14). Instead, increased sS100B (Figure 14B), ≥2 involved distant sites, and no prior systemic treatments were associated with poor OS in univariate and multivariate analysis. None of 13 patients with ongoing complete responses following systemic treatments died during follow-up. If the analysis was limited to the subgroup of patients who were tumor-free after complete metastasectomy the same factors remained independently associated with OS (Table 15).

Looking at 206 unresectable stage IV patients (Table 7), elevated sGDF-15 had a strong independent negative impact on OS (HR 1.9; p<0.001) in addition to the M category (HR 1.6; p<0.001 for M1c). The association of sGDF-15 with OS was evident both in M1a/b (Figure 9A) and in M1c patients (Figure 9B). In more detailed multivariable model 2, elevated sGDF-15, elevated sS100B (Figure 14C), CNS involvement, and ≥4 involved distant sites were independently associated with poorer OS (Table 7). Strong differences in OS were observed according to the nomogram-based risk score accounting for the relative impact of these four factors. 31.1% of patients with a risk score <100 had a 1-year OS of 48.3%. In contrast, none of 21.2% of patients who had a risk score ≥250 survived the first year after serum sampling (Figures 9C, 9D). Despite being associated with OS in univariate analysis, sLDH and the pattern of distant metastasis had no additional impact on OS when considered together with the other factors. OS of patients who received prior systemic treatment was not different compared to those without (Table 7) and a similar independent impact of sGDF-15 on OS was observed, if the analysis was limited to unresectable patients who were treatment-naive (Table 16), or to those of the validation cohort only (Table 17). In patients with CNS-involvement GDF-15, sLDH and sS100B were associated with OS in univariate analysis but not independent factors when analyzed in combination (Table 18).

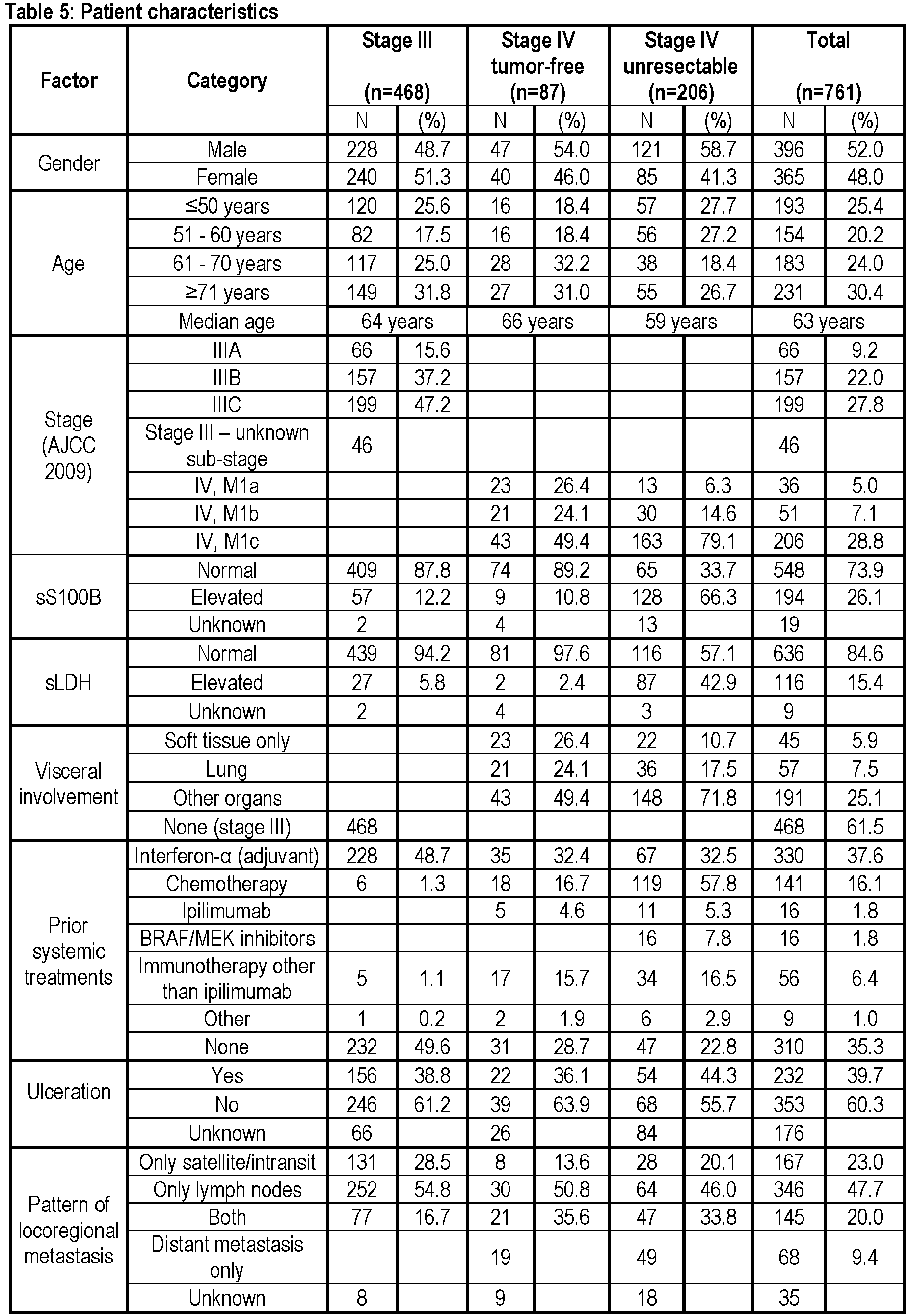

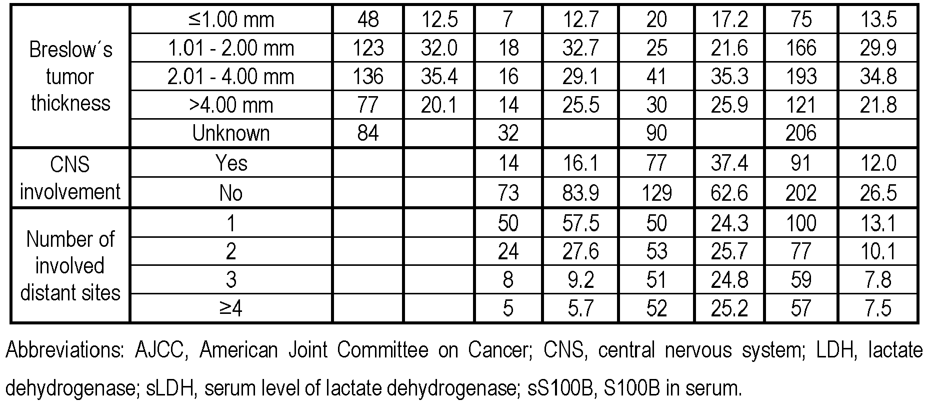

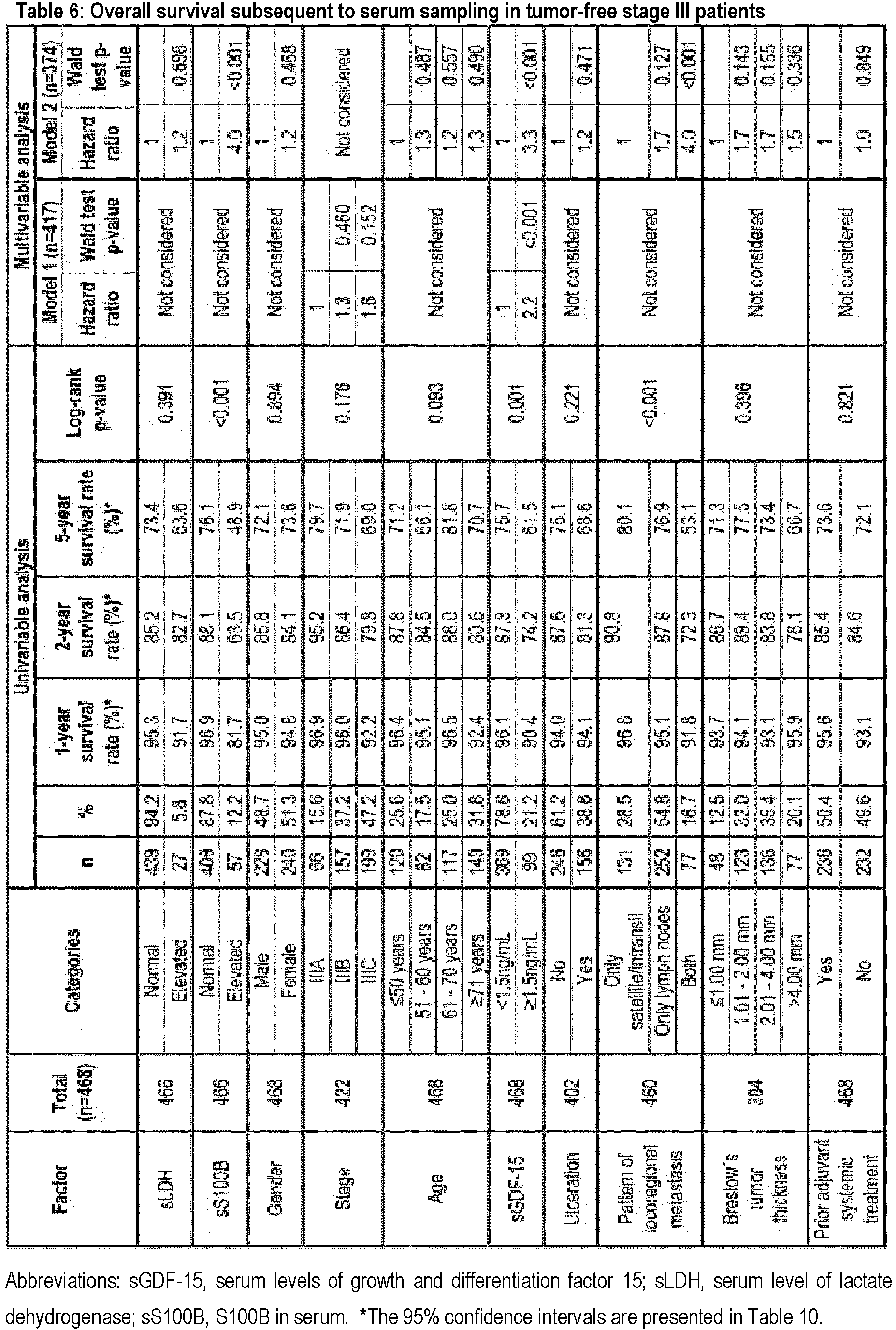

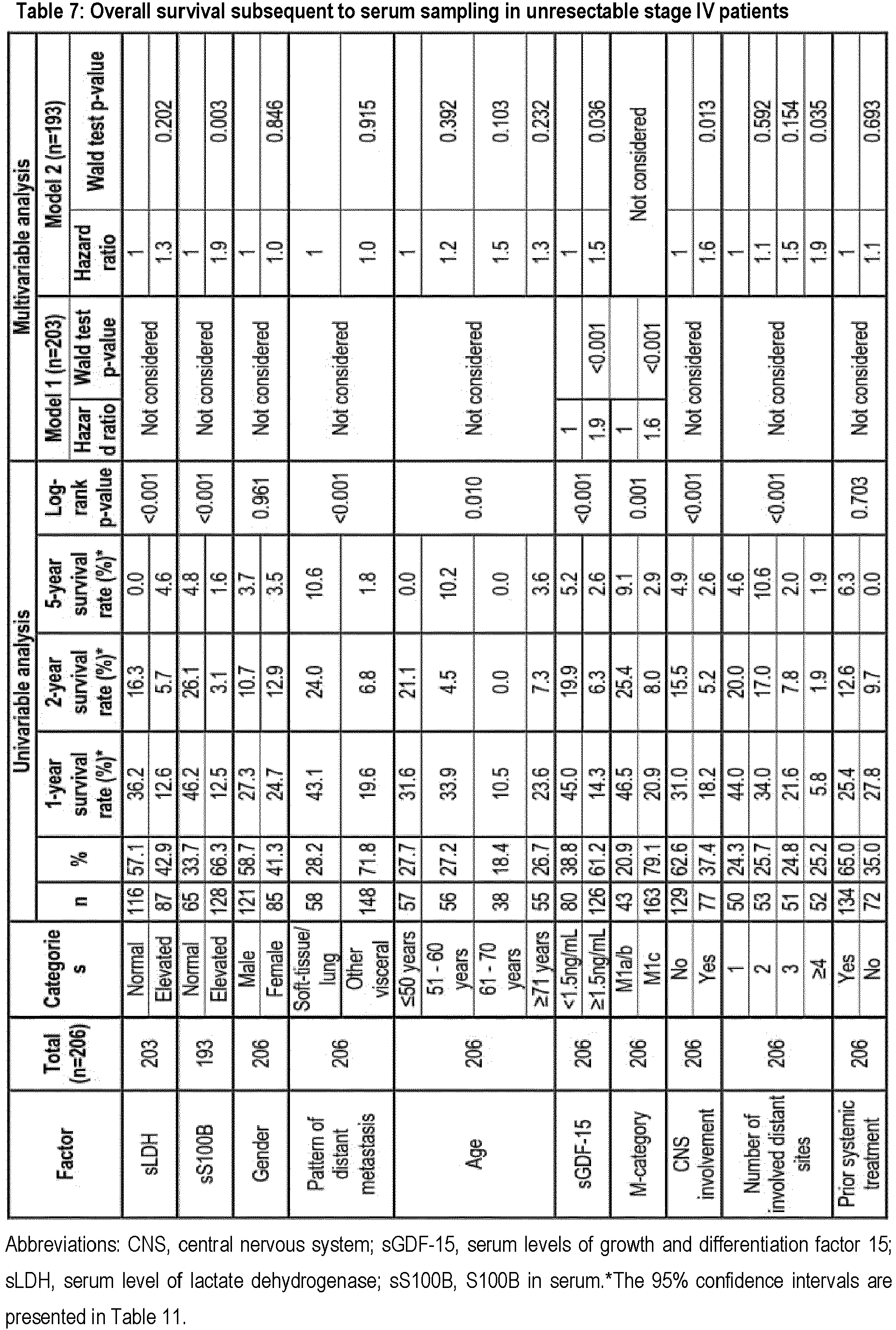

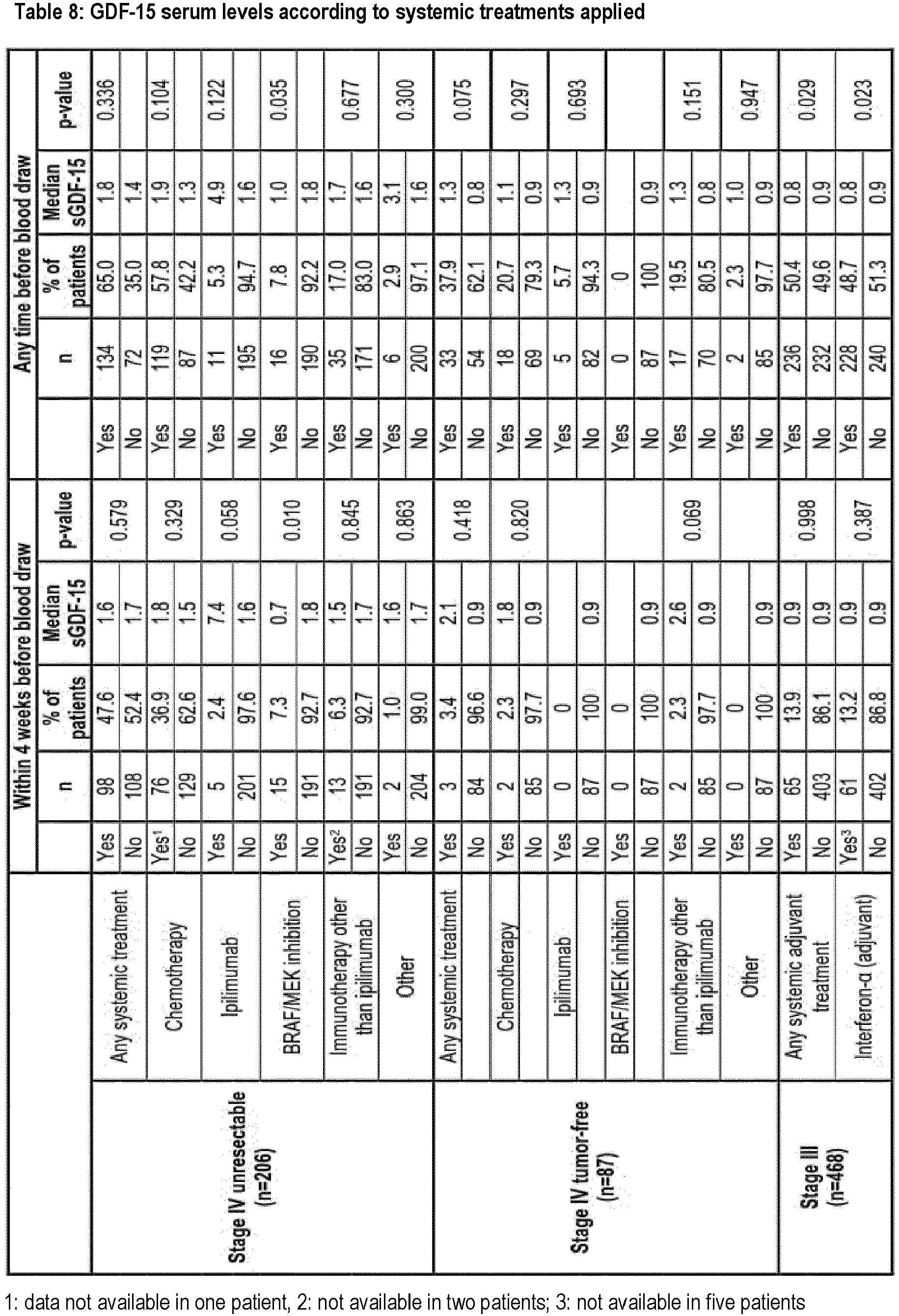

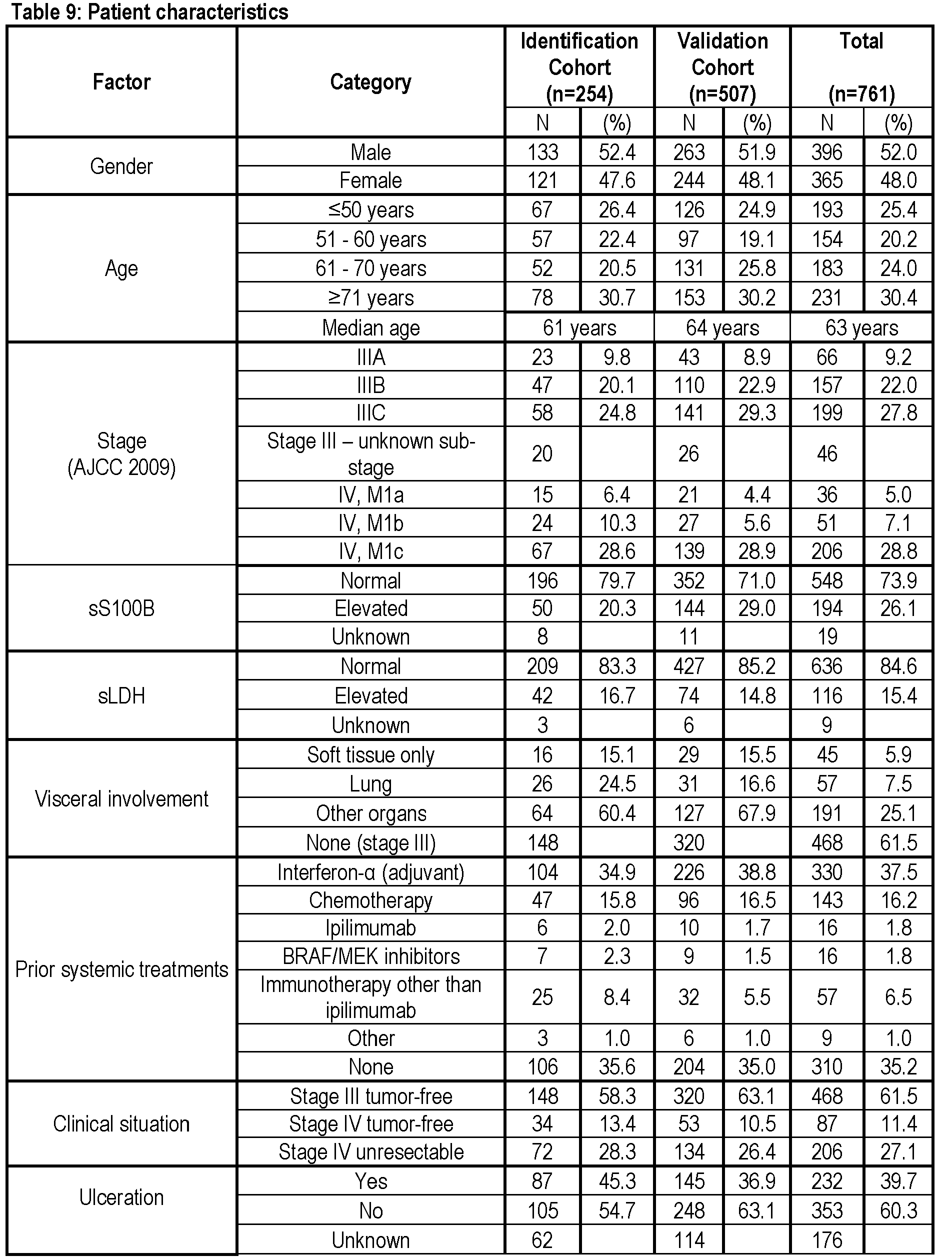

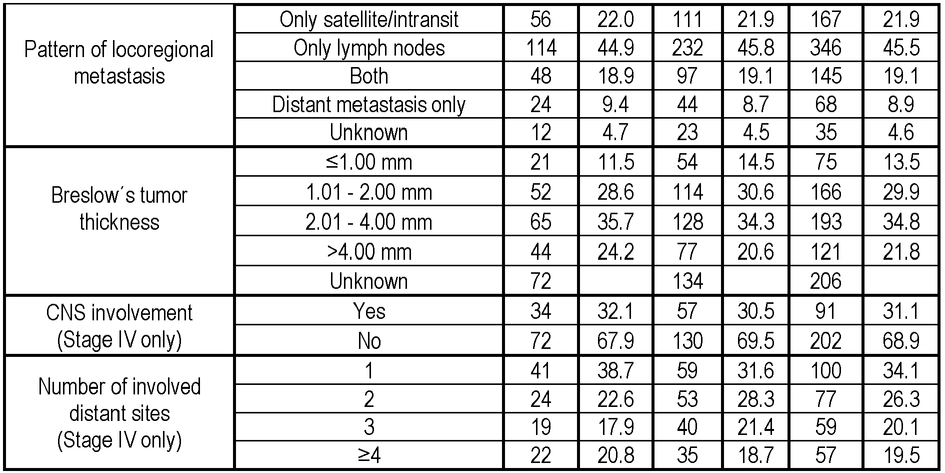

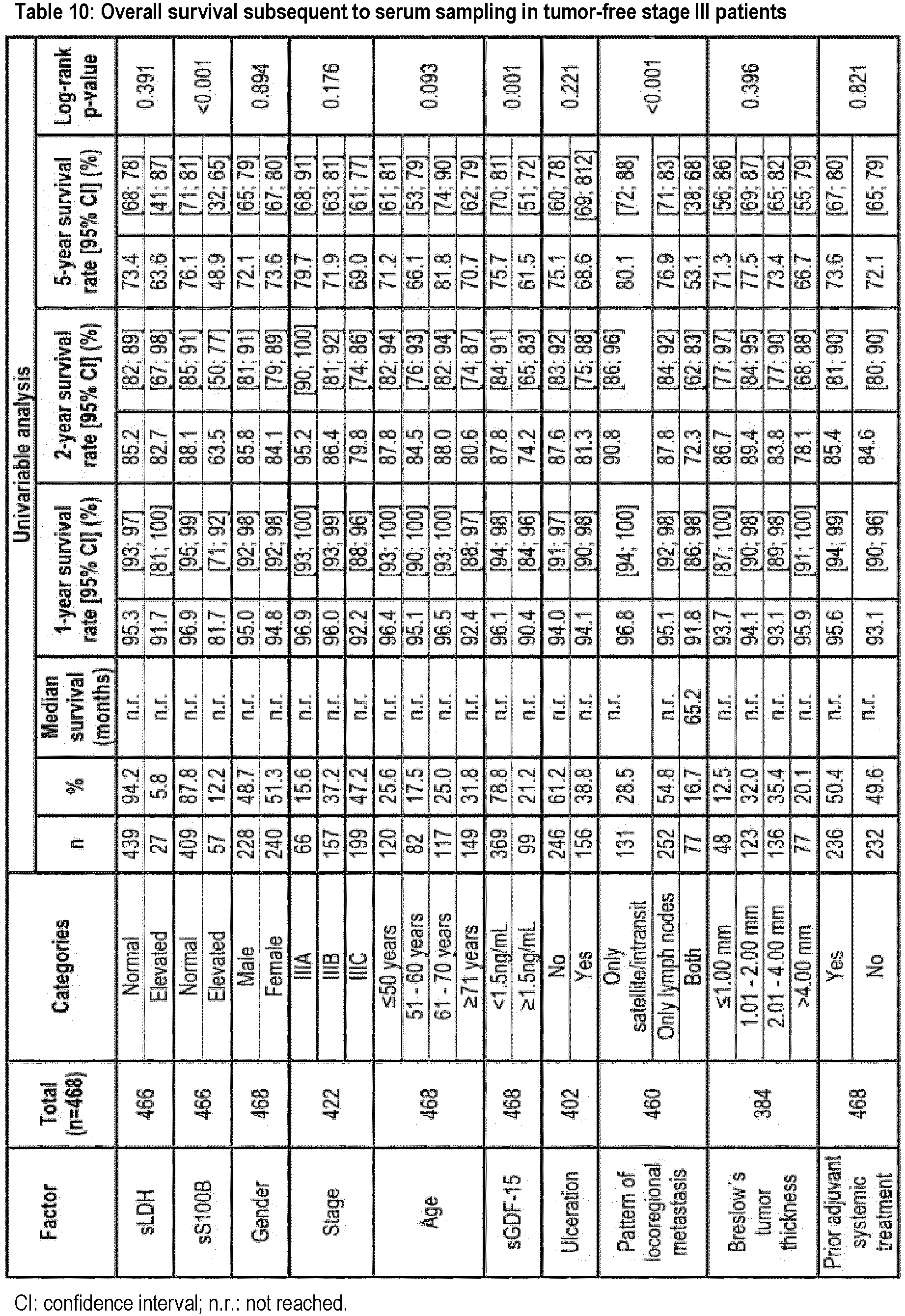

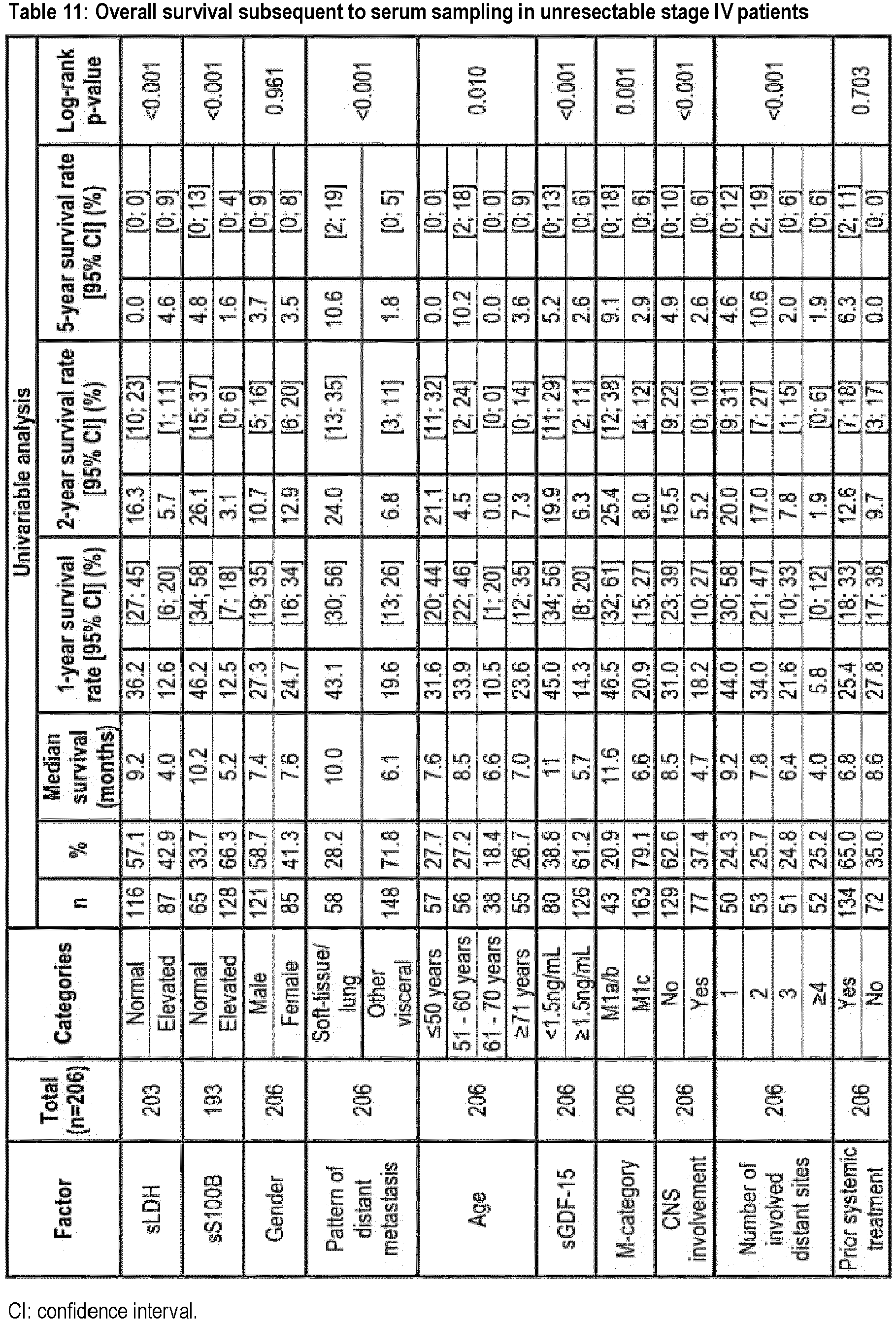

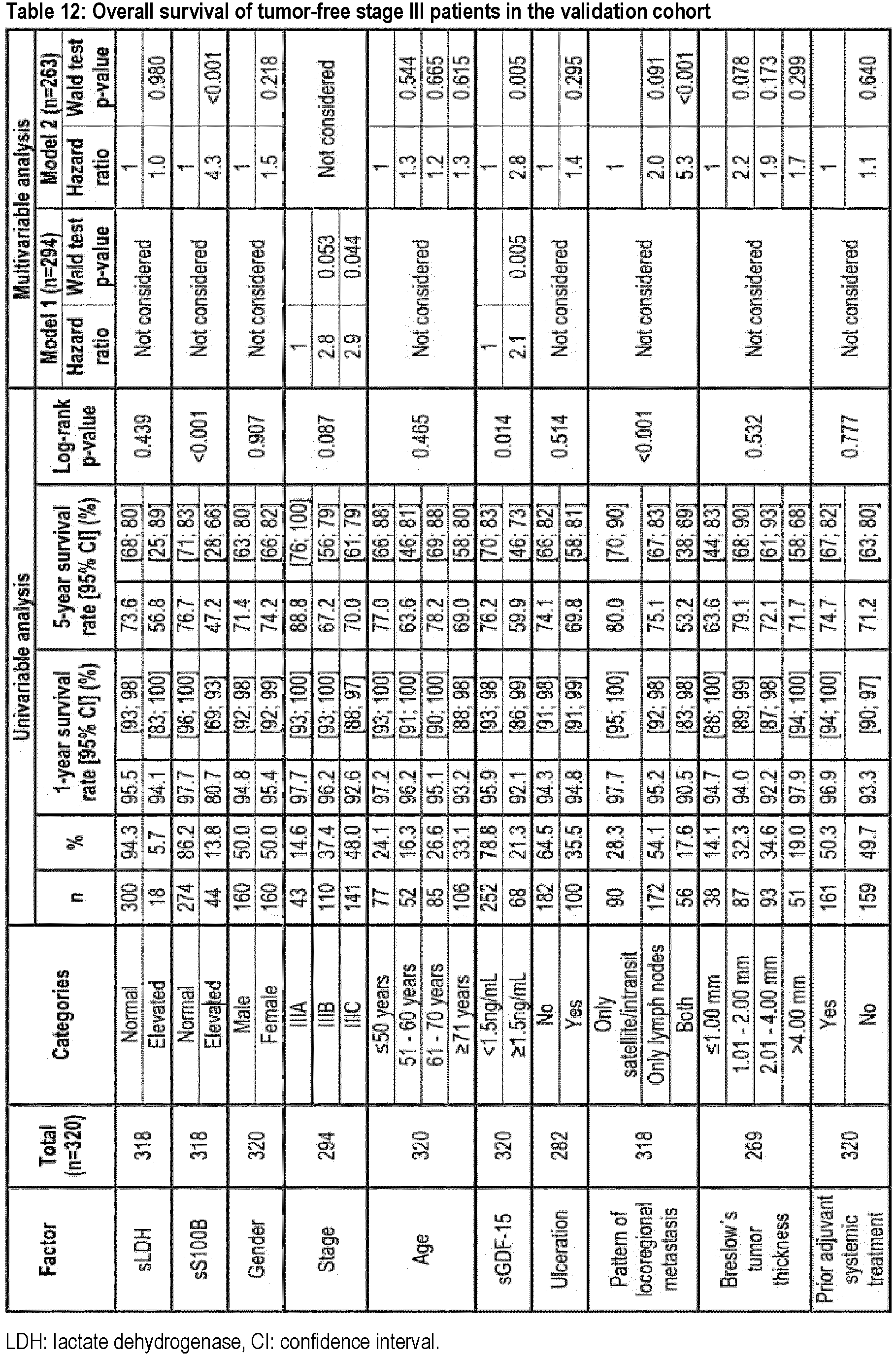

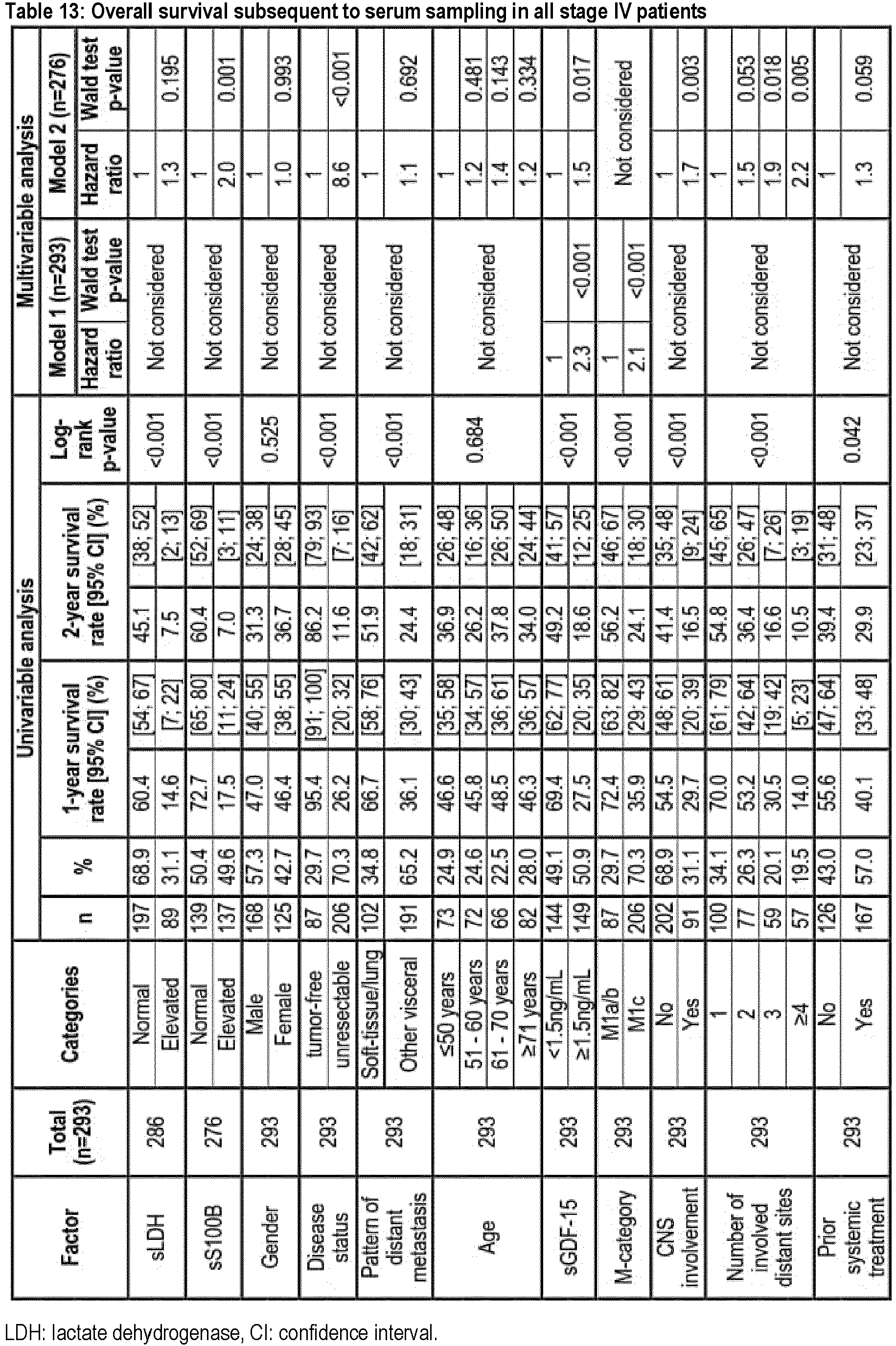

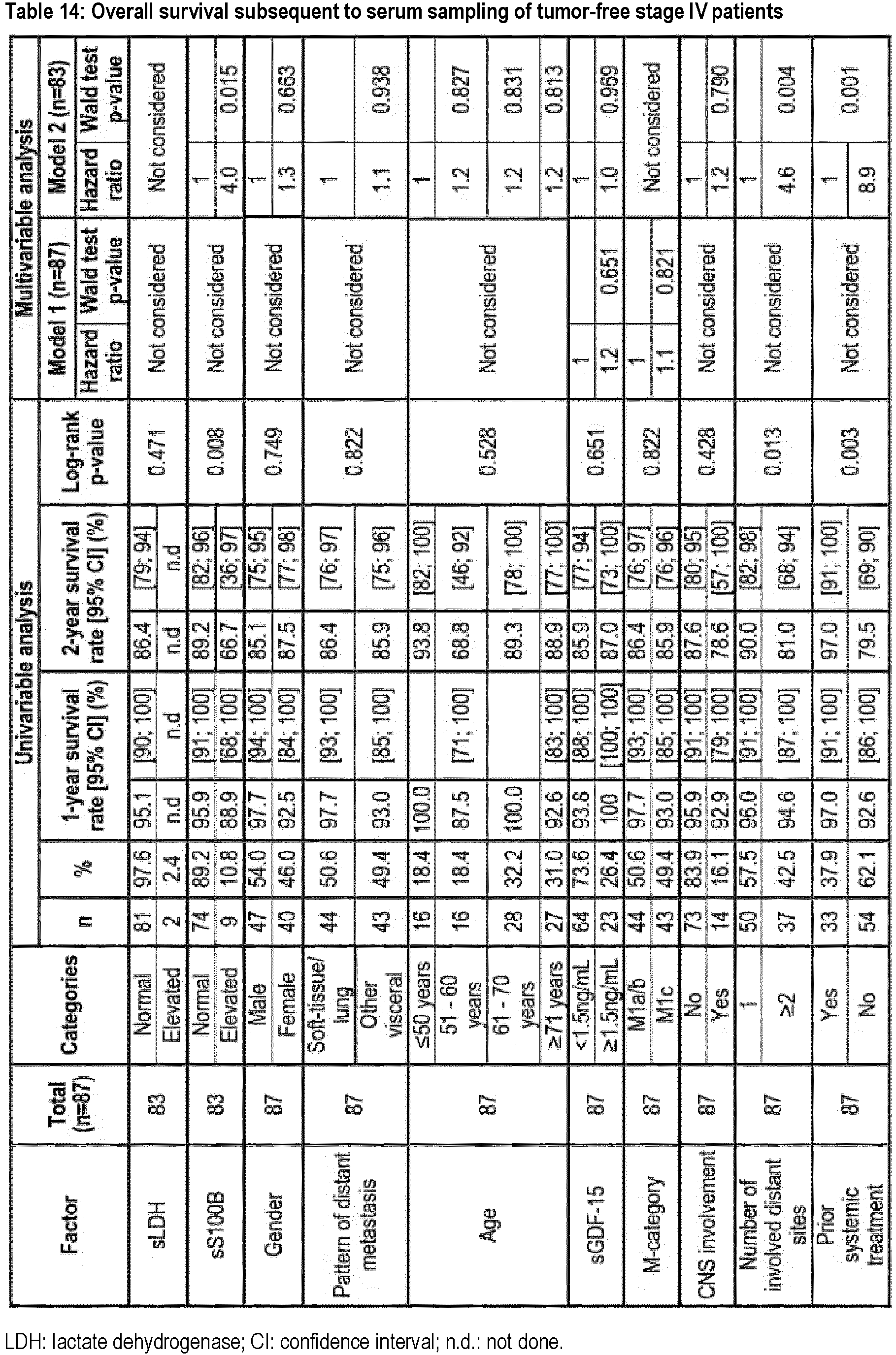

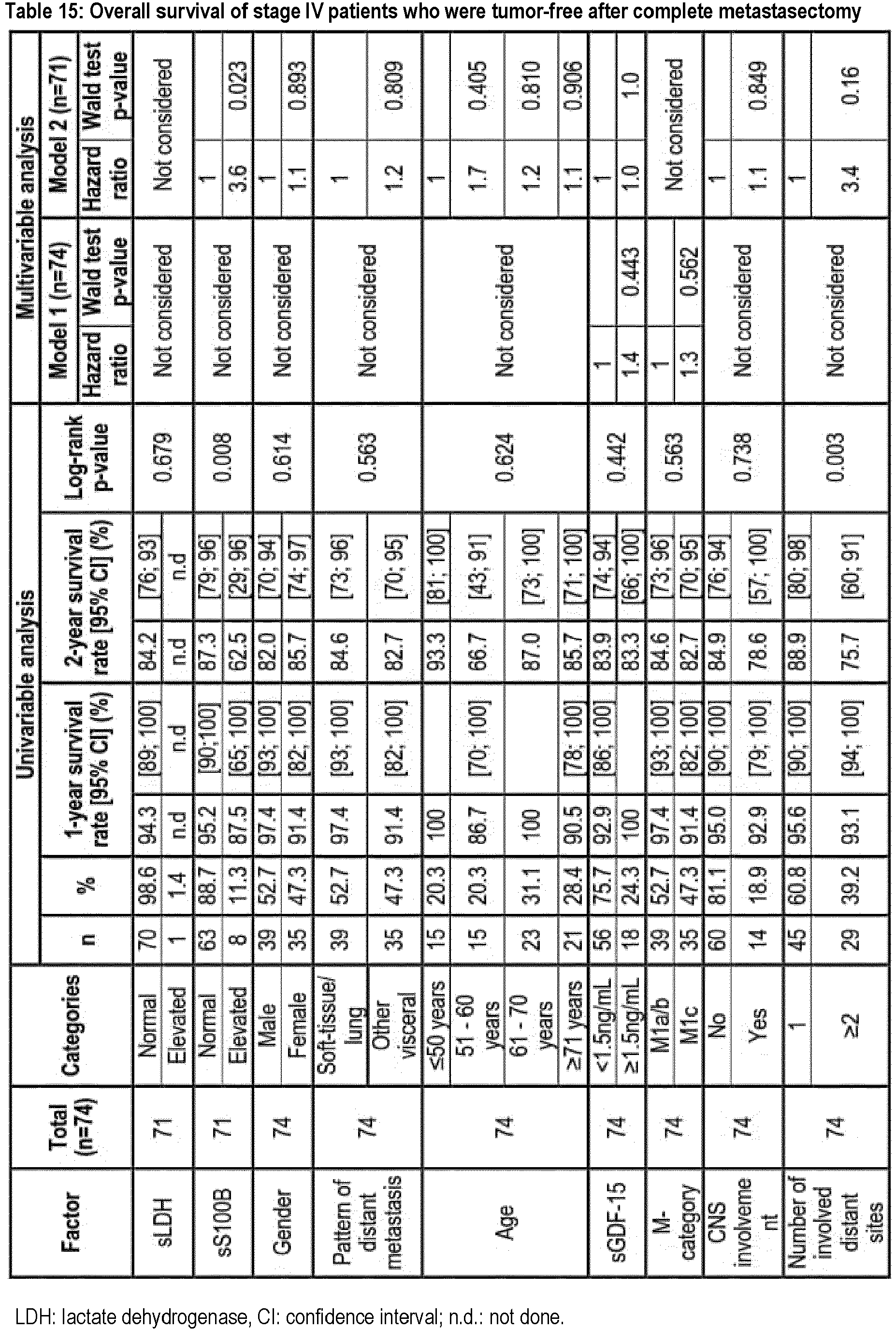

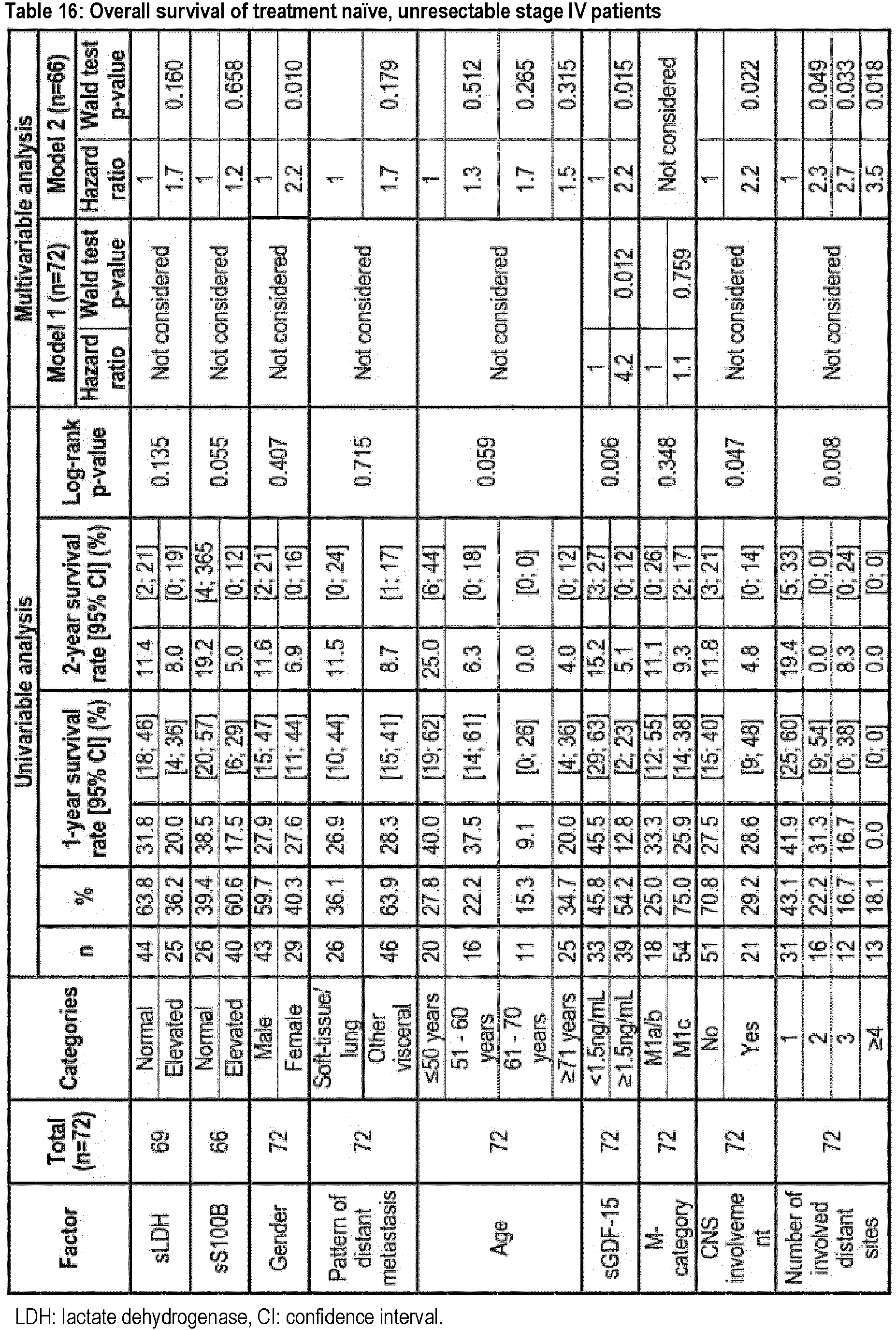

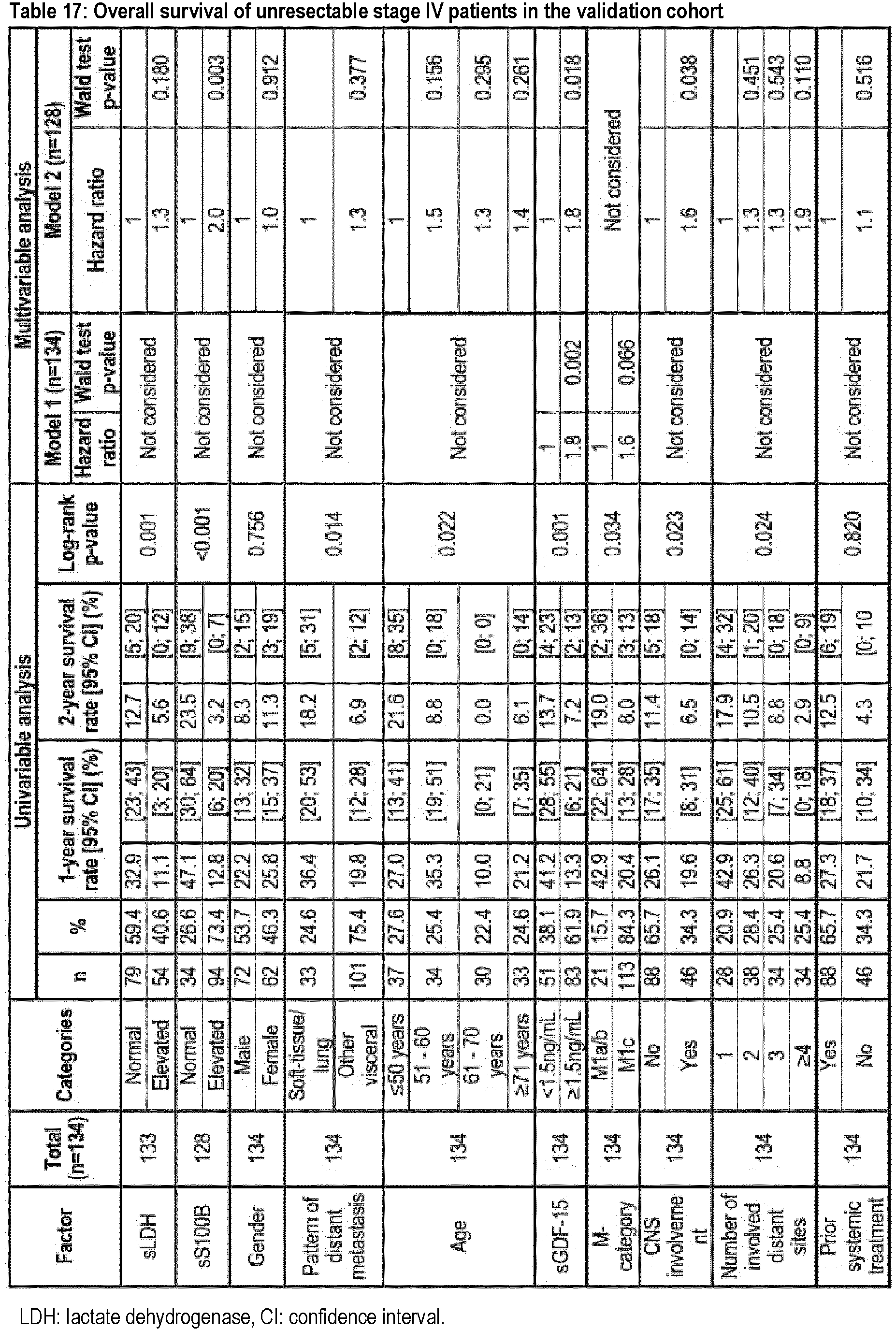

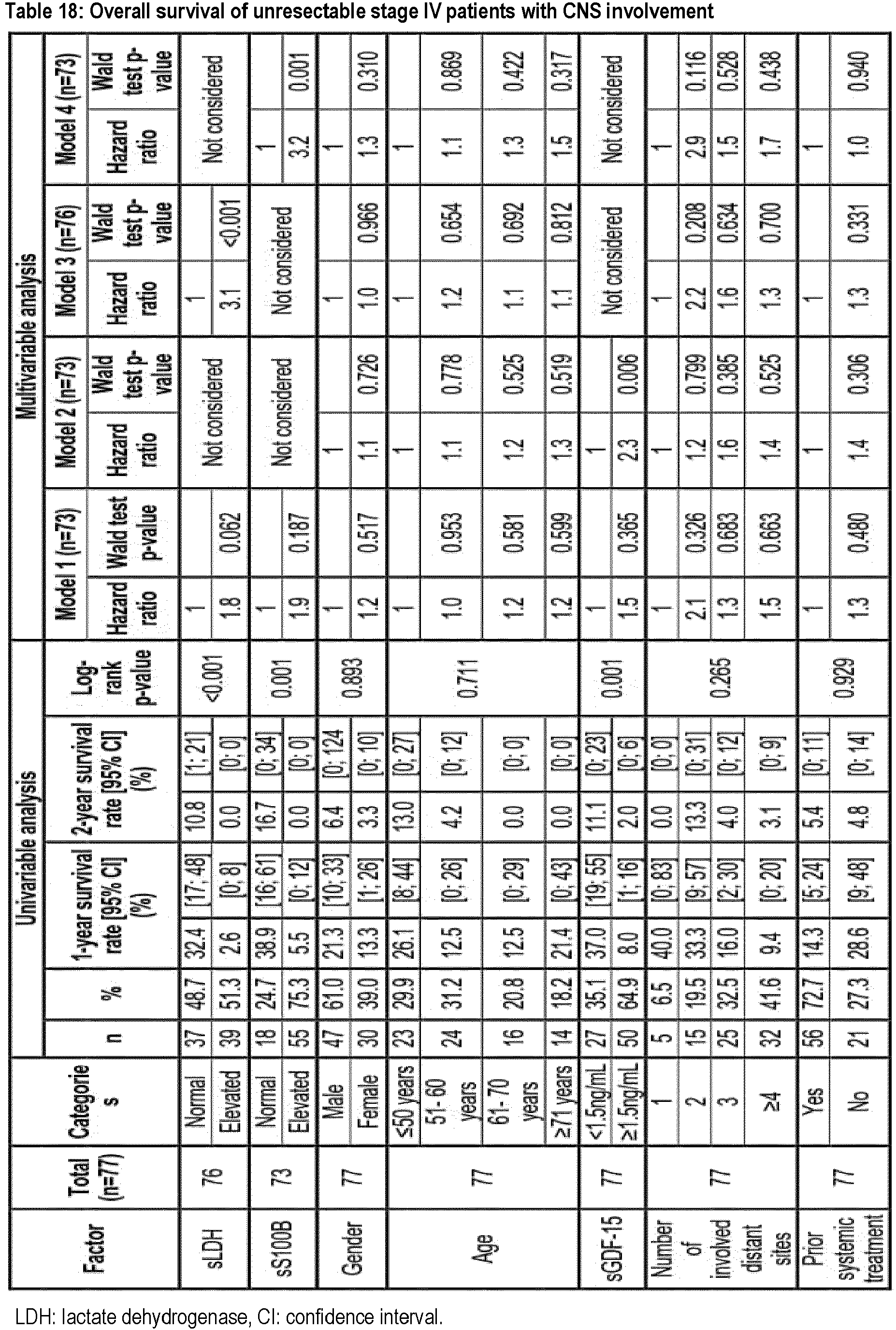

### Summary:

In the current study, the inventors surprisingly found that the serum concentration of hGDF-15 is a powerful prognostic biomarker for patients with metastatic melanoma.

For instance, the inventors found that hGDF-15 serum concentrations above 1.5 ng/mL most strongly predicted poor overall survival in a cohort of 761 patients with metastatic melanoma.

In tumor-free stage III patients, no world-wide accepted prognostic biomarkers are used in daily clinical routine. Estimation of the individual prognosis is mainly based on clinical and histopathological characteristics considered for the definition of the sub-stages IIIA, IIIB, or IIIC, respectively (Balch, CM et al., J Clin Oncol / 27 / 6199-206. 2009). Serum LDH does not harbor prognostic information in tumor-free patients after surgery of loco-regional metastases (Wevers, KP et al., Ann Surg Oncol / 20 / 2772-9. 2013). Serum levels of S100B are only analyzed for early detection of recurrences mainly in Europe (Pflugfelder, A et al., J Dtsch Dermatol Ges / 11 / 563-602. 2013), despite a large body of evidence of its prognostic impact in melanoma patients (Mocellin, S et al., Int J Cancer / 123/2370-6. 2008). In the current study, the inventors surprisingly found that sGDF15 and sS100B are both independent prognostic markers for these patients and are greatly superior to the clinical sub-stage for the identification of patients who will likely die from the disease.

The analysis of sGDF-15 alone allowed to identify 21% of all tumor-free stage III patients with high serum concentrations, who had a 2-fold increased risk to die within three years after blood draw compared to patients with low levels (33% vs 16%, respectively). The combined consideration of sGDF-15 and sS100B increased the proportion of patients at risk from 21% (sGDF-15 elevated irrespective of sS100B) to 31% (either one or both biomarkers elevated) and further enlarged the difference in OS between biomarker categories. In detail, the risk to die within 3 years with normal sS100B and low sGDF-15 was only 14% compared to 33% for patients with at least one biomarker elevated. The blood draw was taken at times without clinical or radiological evidence of disease in these patients thereby especially the combined analysis of both biomarkers may allow to identify patients which might profit from more intense surveillance or adjuvant therapies.

Thus, according to the invention, the use of hGDF-15 as a biomarker for the prediction of survival as defined in the appended claims, e.g. in combination with S100B as a further biomarker, is highly advantageous even for sub-groups of melanoma patients, for which no reliable prognosis of survival has yet been available.

In unresectable stage IV melanoma patients, the pattern of visceral metastasis and sLDH are regularly used to classify patients into prognostically different M-categories M1a, M1b, or M1c (Balch, CM et al., J Clin Oncol / 27 / 6199-206. 2009). In the present study, the consideration of sGDF-15 in combination with these two established prognostic factors significantly improved the estimation of prognosis for the individual patient (HR 1.7; p<0.001; pattern of visceral metastasis: HR 1.8; p<0.001; sLDH: HR 1.6; p=0.002) and allowed the identification of a relevant subgroup (comprising 30% of all patients with unresectable distant metastasis) with an extremely poor probability to survive 1 year (3.3%). In contrast, the worst biomarker category without consideration of sGDF-15 (visceral metastases other than lung and elevated sLDH; 35% of all unresectable stage IV patients) indicated a 1-year survival estimate of 8.3%. The additional consideration of sGDF-15 added prognostic information for M1a/b as well as for M1c patients. The gain in prognostic information based on the consideration of sGDF-15 is valuable for patient counselling and stratification within clinical trials, and might impact the individual risk/benefit assessment for therapeutic decisions. Considering the availability (and emergence) of various therapeutic options for advanced melanoma and the inevitable trade-off between efficacy and side effects, enhanced prognosis prediction most likely becomes instrumental for the further guidance of individualized therapy.

In conclusion, sGDF-15 is a powerful prognostic biomarker in patients with melanoma such as metastatic melanoma.

In tumor-free stage III patients the consideration of sGDF-15 alone or in combination with sS100B allows to identify individuals with increased risk to die from disease who might profit from more intense patient surveillance or adjuvant treatments. In patients with unresectable stage IV melanoma sGDF-15, sLDH and the pattern of visceral metastasis are independent prognostic factors. The combined consideration of these three factors improves the individual estimate of prognosis compared to the M-category alone and may influence individualized treatment decisions.

### References

Arbabi Ghahroudi M et al.: "Selection and identification of single domain antibody fragments from camel heavy-chain antibodies." FEBS Lett. 1997 Sep 15;414(3):521-6.
Ausubel et al.: "Current Protocols in Molecular Biology." Greene Publishing Associates and Wiley Interscience; New York 1992.
Baek, SJ; Okazaki, R; Lee, SH; Martinez, J; Kim, JS; Yamaguchi, K; Mishina, Y; Martin, DW; Shoieb, A; McEntee, MF; Eling, TE. Nonsteroidal anti-inflammatory drug-activated gene-1 over expression in transgenic mice suppresses intestinal neoplasia. Gastroenterology 1 131 / 1553-60. 2006
Baek, SJ; Kim, KS; Nixon, JB; Wilson, LC; Eling, TE. Cyclooxygenase inhibitors regulate the expression of a TGF-beta superfamily member that has proapoptotic and antitumorigenic activities. Mol Pharmacol / 59 / 901-8. 2001
Balch, CM; Gershenwald, JE; Soong, SJ; Thompson, JF; Atkins, MB; Byrd, DR; Buzaid, AC; Cochran, AJ; Coit, DG; Ding, S; Eggermont, AM; Flaherty, KT; Gimotty, PA; Kirkwood, JM; McMasters, KM; Mihm, MC, Jr.; Morton, DL; Ross, MI; Sober, AJ; Sondak, VK. Final version of 2009 AJCC melanoma staging and classification. J Clin Oncol / 27 / 6199-206. 2009
Balch, CM; Buzaid, AC; Soong, SJ; Atkins, MB; Cascinelli, N; Coit, DG; Fleming, ID; Gershenwald, JE; Houghton, A, Jr.; Kirkwood, JM; McMasters, KM; Mihm, MF; Morton, DL; Reintgen, DS; Ross, MI; Sober, A; Thompson, JA; Thompson, JF. Final version of the American Joint Committee on Cancer staging system for cutaneous melanoma. J Clin Oncol / 19 / 3635-48. 2001
Bauskin, AR; Brown, DA; Kuffner, T; Johnen, H; Luo, XW; Hunter, M; Breit, SN. Role of macrophage inhibitory cytokine-1 in tumorigenesis and diagnosis of cancer. Cancer Res / 66 / 4983-6. 2006
Blanco-Calvo, M; Tarrio, N; Reboredo, M; Haz-Conde, M; Garcia, J; Quindos, M; Figueroa, A; Anton-Aparicio, L; Calvo, L; Valladares-Ayerbes, M. Circulating levels of GDF15, MMP7 and miR-200c as a poor prognostic signature in gastric cancer. Future Oncol / 10 / 1187-202. 2014
Bock, AJ; Stavnes, HT; Kempf, T; Trope, CG; Berner, A; Davidson, B; Staff, AC. Expression and clinical role of growth differentiation factor-15 in ovarian carcinoma effusions. Int J Gynecol Cancer / 20 / 1448-55. 2010
Boyle, GM; Pedley, J; Martyn, AC; Banducci, KJ; Strutton, GM; Brown, DA; Breit, SN; Parsons, PG. Macrophage inhibitory cytokine-1 is overexpressed in malignant melanoma and is associated with tumorigenicity. J Invest Dermatol / 129 / 383-91. 2009
Brown, DA; Lindmark, F; Stattin, P; Balter, K; Adami, HO; Zheng, SL; Xu, J; Isaacs, WB; Gronberg, H; Breit, SN; Wiklund, FE. Macrophage inhibitory cytokine 1: a new prognostic marker in prostate cancer. Clin Cancer Res / 15 / 6658-64. 2009
Brown, DA; Ward, RL; Buckhaults, P; Liu, T; Romans, KE; Hawkins, NJ; Bauskin, AR; Kinzler, KW; Vogelstein, B; Breit, SN. MIC-1 serum level and genotype: associations with progress and prognosis of colorectal carcinoma. Clin Cancer Res / 9 / 2642-50. 2003
Camp, RL; Dolled-Filhart, M; Rimm, DL. X-tile: a new bio-informatics tool for biomarker assessment and outcome-based cut-point optimization. Clin Cancer Res / 10 / 7252-9. 2004
Chothia C et al.: Conformations of immunoglobulin hypervariable regions. Nature. 1989 Dec 21-28;342(6252):877-83.
Clackson T et al.: "Making antibody fragments using phage display libraries." Nature. 1991 Aug 15;352(6336):624-8.
Eisenhauer et al.: New response evaluation criteria in solid tumours: revised RECIST guideline (version 1.1). Eur. J. Cancer. 45, No. 2, January 2009, pp 228-47.
Eling, TE; Baek, SJ; Shim, M; Lee, CH. NSAID activated gene (NAG-1), a modulator of tumorigenesis. J Biochem Mol Biol / 39 / 649-55. 2006
Eton, O; Legha, SS; Moon, TE; Buzaid, AC; Papadopoulos, NE; Plager, C; Burgess, AM; Bedikian, AY; Ring, S; Dong, Q; Glassman, AB; Balch, CM; Benjamin, RS. Prognostic factors for survival of patients treated systemically for disseminated melanoma. J Clin Oncol / 16 / 1103-11. 1998
Giudicelli V et al.: IMGT/V-QUEST, an integrated software program for immunoglobulin and T cell receptor V-J and V-D-J rearrangement analysis. Nucleic Acids Res. 2004 Jul 1;32(Web Server issue):W435-40.
Gonçalves et al.: "Biological and methodological features of the measurement of S100B, a putative marker of brain injury." Clinical Biochemistry 41 (2008) 755-763).
Harlow and Lane: "Antibodies: A Laboratory Manual" Cold Spring Harbor Laboratory Press, Cold Spring Harbor, New York 1988.
Holliger P et al.: ""Diabodies": small bivalent and bispecific antibody fragments." Proc Natl Acad Sci USA. 1993 Jul 15;90(14):6444-8.
Holt LJ et al.: "Domain antibodies: proteins for therapy." Trends Biotechnol. 2003 Nov;21(11):484-90.
Huh, SJ; Chung, CY; Sharma, A; Robertson, GP. Macrophage inhibitory cytokine-1 regulates melanoma vascular development. Am J Pathol / 176 / 2948-57. 2010
Husaini, Y; Qiu, MR; Lockwood, GP; Luo, XW; Shang, P; Kuffner, T; Tsai, VW; Jiang, L; Russell, PJ; Brown, DA; Breit, SN. Macrophage inhibitory cytokine-1 (MIC-1/GDF15) slows cancer development but increases metastases in TRAMP prostate cancer prone mice. PLoS One / 7 / e43833. 2012
Johnen, H; Lin, S; Kuffner, T; Brown, DA; Tsai, VW; Bauskin, AR; Wu, L; Pankhurst, G; Jiang, L; Junankar, S; Hunter, M; Fairlie, WD; Lee, NJ; Enriquez, RF; Baldock, PA; Corey, E; Apple, FS; Murakami, MM; Lin, EJ; Wang, C; During, MJ; Sainsbury, A; Herzog, H; Breit, SN. Tumor-induced anorexia and weight loss are mediated by the TGF-beta superfamily cytokine MIC-1. Nat Med /13 / 1333-40. 2007
Jones PT et al.: "Replacing the complementarity-determining regions in a human antibody with those from a mouse." Nature. 1986 May 29-Jun 4;321 (6069):522-5.
Jones, MF; Ling Li, X; Subramanian, M; Shabalina, SA; Hara, T; Zhu, Y; Huang, J; Yang, Y; Wakefield, LM; Prasanth, KV; Lal, A. Growth differentiation factor-15 encodes a novel microRNA 3189 that functions as a potent regulator of cell death. Cell Death Differ / / 2015
Kabat et al.: Sequences of proteins of immunological interest, U.S. Dept. of Health and Human Services, Public Health Service, National Institutes of Health, Bethesda, Md. 1983.
Kluger, HM; Hoyt, K; Bacchiocchi, A; Mayer, T; Kirsch, J; Kluger, Y; Sznol, M; Ariyan, S; Molinaro, A; Halaban, R. Plasma markers for identifying patients with metastatic melanoma. ClinCancer Res / 17 / 2417-25. 2011
Kohler G and Milstein C: "Continuous cultures of fused cells secreting antibody of predefined specificity." Nature. 1975 Aug 7;256(5517):495-7.
Lasithiotakis, KG; Leiter, U; Gorkievicz, R; Eigentler, T; Breuninger, H; Metzler, G; Strobel, W; Garbe, C. The incidence and mortality of cutaneous melanoma in Southern Germany: trends by anatomic site and pathologic characteristics, 1976 to 2003. Cancer / 107 / 1331-9. 2006.
Liu T et al: "Macrophage inhibitory cytokine 1 reduces cell adhesion and induces apoptosis in prostate cancer cells." Cancer Res., vol. 63, no. 16, 1 August 2003, pp.5034-5040.
Ma J, Tang X, Sun WW, Liu Y, Tan YR, Ma HL, et al. (2016) Mutant GDF15 presents a poor prognostic outcome for patients with oral squamous cell carcinoma. Oncotarget 7:2113-22.
Manola, J; Atkins, M; lbrahim, J; Kirkwood, J. Prognostic factors in metastatic melanoma: a pooled analysis of Eastern Cooperative Oncology Group trials. J Clin Oncol / 18 / 3782-93. 2000
Marks JD et al.: "By-passing immunization. Human antibodies from V-gene libraries displayed on phage." J Mol Biol. 1991 Dec 5;222(3):581-97.
Martinez, JM; Sali, T; Okazaki, R; Anna, C; Hollingshead, M; Hose, C; Monks, A; Walker, NJ; Baek, SJ; Eling, TE. Drug-induced expression of nonsteroidal anti-inflammatory drug-activated gene/macrophage inhibitory cytokine-1/prostate-derived factor, a putative tumor suppressor, inhibits tumor growth. J Pharmacol Exp Ther / 318 / 899-906. 2006
Mauerer, A; Roesch, A; Hafner, C; Stempfl, T; Wild, P; Meyer, S; Landthaler, M; Vogt, T. Identification of new genes associated with melanoma. Exp Dermatol / 20 / 502-7. 2011
Mimeault, M; Johansson, SL; Batra, SK. Marked improvement of cytotoxic effects induced by docetaxel on highly metastatic and androgen-independent prostate cancer cells by downregulating macrophage inhibitory cytokine-1. Br J Cancer / 108/ 1079-91. 2013
Mimeault, M; Batra, SK. Divergent molecular mechanisms underlying the pleiotropic functions of macrophage inhibitory cytokine-1 in cancer. J Cell Physiol / 224 / 626-35. 2010
Min, KW; Liggett, JL; Silva, G; Wu, WW; Wang, R; Shen, RF; Eling, TE; Baek, SJ. NAG-1/GDF15 accumulates in the nucleus and modulates transcriptional regulation of the Smad pathway. Oncogene / / 2015
Mocellin, S; Zavagno, G; Nitti, D. The prognostic value of serum S100B in patients with cutaneous melanoma: a meta-analysis. Int J Cancer / 123 / 2370-6. 2008
Paul, W.E. (Ed.).: "Fundamental Immunology" 2nd Ed. Raven Press, Ltd., New York 1989.
Pflugfelder, A; Kochs, C; Blum, A; Capellaro, M; Czeschik, C; Dettenborn, T; Dill, D; Dippel, E; Eigentler, T; Feyer, P; Follmann, M; Frerich, B; Ganten, MK; Gartner, J; Gutzmer, R; Hassel, J; Hauschild, A; Hohenberger, P; Hubner, J; Kaatz, M; Kleeberg, UR; Kolbl, O; Kortmann, RD; Krause-Bergmann, A; Kurschat, P; Leiter, U; Link, H; Loquai, C; Loser, C; Mackensen, A; Meier, F; Mohr, P; Mohrle, M; Nashan, D; Reske, S; Rose, C; Sander, C; Satzger, I; Schiller, M; Schlemmer, HP; Strittmatter, G; Sunderkotter, C; Swoboda, L; Trefzer, U; Voltz, R; Vordermark, D; Weichenthal, M; Werner, A; Wesselmann, S; Weyergraf, AJ; Wick, W; Garbe, C; Schadendorf, D. Malignant melanoma S3-guideline "diagnosis, therapy and follow-up of melanoma". J Dtsch Dermatol Ges / 11 Suppl 6 / 1-116, 1-26. 2013
Pflugfelder, A; Kochs, C; Blum, A; Capellaro, M; Czeschik, C; Dettenborn, T; Dill, D; Dippel, E; Eigentler, T; Feyer, P; Follmann, M; Frerich, B; Ganten, MK; Gartner, J; Gutzmer, R; Hassel, J; Hauschild, A; Hohenberger, P; Hubner, J; Kaatz, M; Kleeberg, UR; Kolbl, O; Kortmann, RD; Krause-Bergmann, A; Kurschat, P; Leiter, U; Link, H; Loquai, C; Loser, C; Mackensen, A; Meier, F; Mohr, P; Mohrle, M; Nashan, D; Reske, S; Rose, C; Sander, C; Satzger, I; Schiller, M; Schlemmer, HP; Strittmatter, G; Sunderkotter, C; Swoboda, L; Trefzer, U; Voltz, R; Vordermark, D; Weichenthal, M; Werner, A; Wesselmann, S; Weyergraf, AJ; Wick, W; Garbe, C; Schadendorf, D. S3-guideline "diagnosis, therapy and follow-up of melanoma" -- short version. J Dtsch Dermatol Ges / 11 / 563-602. 2013
Remington's Pharmaceutical Sciences, Ed. AR Gennaro, 20th edition, 2000, Williams & Wilkins, PA, USA.
Riechmann L et al.: "Reshaping human antibodies for therapy." Nature. 1988 Mar 24;332(6162):323-7.
Riker, Al; Enkemann, SA; Fodstad, O; Liu, S; Ren, S; Morris, C; Xi, Y; Howell, P; Metge, B; Samant, RS; Shevde, LA; Li, W; Eschrich, S; Daud, A; Ju, J; Matta, J. The gene expression profiles of primary and metastatic melanoma yields a transition point of tumor progression and metastasis. BMC Med Genomics / 1 / 13. 2008
Roth, P; Junker, M; Tritschler, I; Mittelbronn, M; Dombrowski, Y; Breit, SN; Tabatabai, G; Wick, W; Weller, M; Wischhusen, J. GDF-15 contributes to proliferation and immune escape of malignant gliomas. Clin Cancer Res / 16/ 3851-9. 2010
Saerens D et al.: "Single-domain antibodies as building blocks for novel therapeutics." Curr Opin Pharmacol. 2008 Oct;8(5):600-8. Epub 2008 Aug 22.
Sambrook et al.: "Molecular Cloning: A Laboratory Manual.", 2nd Ed., Cold Spring Harbor Laboratory Press, Cold Spring Harbor, New York 1989.
Selander, KS; Brown, DA; Sequeiros, GB; Hunter, M; Desmond, R; Parpala, T; Risteli, J; Breit, SN; Jukkola-Vuorinen, A. Serum macrophage inhibitory cytokine-1 concentrations correlate with the presence of prostate cancer bone metastases. Cancer Epidemiol Biomarkers Prev / 16 / 532-7. 2007
Siegel DL: "Recombinant monoclonal antibody technology." Transfus Clin Biol. 2002 Jan;9(1):15-22.
Sirott, MN; Bajorin, DF; Wong, GY; Tao, Y; Chapman, PB; Templeton, MA; Houghton, AN. Prognostic factors in patients with metastatic malignant melanoma. A multivariate analysis. Cancer / 72 / 3091-8. 1993
Staff, AC; Bock, AJ; Becker, C; Kempf, T; Wollert, KC; Davidson, B. Growth differentiation factor-15 as a prognostic biomarker in ovarian cancer. Gynecol Oncol / 118 / 237-43. 2010
Stefanescu R. et al., Eur. J.Mass Spectrom. 13, 69-75 (2007)
Suckau et al. Proc Natl Acad Sci USA. 1990 December; 87(24): 9848-9852.
Suesskind, D; Schatz, A; Schnichels, S; Coupland, SE; Lake, SL; Wissinger, B; Bartz-Schmidt, KU; Henke-Fahle, S. GDF-15: a novel serum marker for metastases in uveal melanoma patients. Graefes Arch Clin Exp Ophthalmol / 250 / 887-95. 2012
Talantov, D; Mazumder, A; Yu, JX; Briggs, T; Jiang, Y; Backus, J; Atkins, D; Wang, Y. Novel genes associated with malignant melanoma but not benign melanocytic lesions. Clin Cancer Res / 11 / 7234-42. 2005
Wallin, U; Glimelius, B; Jirstrom, K; Darmanis, S; Nong, RY; Ponten, F; Johansson, C; Pahlman, L; Birgisson, H. Growth differentiation factor 15: a prognostic marker for recurrence in colorectal cancer. Br J Cancer /104 / 1619-27. 2011
Wang, X; Baek, SJ; Eling, TE. The diverse roles of nonsteroidal anti-inflammatory drug activated gene (NAG-1/GDF15) in cancer. Biochem Pharmacol / 85 / 597-606. 2013
Weide, B; Richter, S; Buttner, P; Leiter, U; Forschner, A; Bauer, J; Held, L; Eigentler, TK; Meier, F; Garbe, C. Serum S100B, lactate dehydrogenase and brain metastasis are prognostic factors in patients with distant melanoma metastasis and systemic therapy. PLoS One / 8/ e81624. 2013
Weide, B; Elsasser, M; Buttner, P; Pflugfelder, A; Leiter, U; Eigentler, TK; Bauer, J; Witte, M; Meier, F; Garbe, C. Serum markers lactate dehydrogenase and S100B predict independently disease outcome in melanoma patients with distant metastasis. Br J Cancer / 107 / 422-8. 2012
Weinberg R. et al.: The Biology of Cancer. Garland Science: New York 2006. 850p.
Wevers, KP; Kruijff, S; Speijers, MJ; Bastiaannet, E; Muller Kobold, AC; Hoekstra, HJ. S-100B: a stronger prognostic biomarker than LDH in stage IIIB-C melanoma. Ann Surg Oncol / 20 / 2772-9. 2013
Zhang, J., Yao, Y.-H., Li, B.-G., Yang, Q., Zhang, P.-Y., and Wang, H.-T. (2015). Prognostic value of pretreatment serum lactate dehydrogenase level in patients with solid tumors: a systematic review and meta-analysis. Scientific Reports 5, 9800.
Zhang, L; Yang, X; Pan, HY; Zhou, XJ; Li, J; Chen, WT; Zhong, LP; Zhang, ZY. Expression of growth differentiation factor 15 is positively correlated with histopathological malignant grade and in vitro cell proliferation in oral squamous cell carcinoma. Oral Oncol / 451 627-32. 2009
Zhou, Z; Li, W; Song, Y; Wang, L; Zhang, K; Yang, J; Zhang, W; Su, H; Zhang, Y. Growth differentiation factor-15 suppresses maturation and function of dendritic cells and inhibits tumor-specific immune response. PLoS One / 8 / e78618. 2013
Zimmers, TA; Gutierrez, JC; Koniaris, LG. Loss of GDF-15 abolishes sulindac chemoprevention in the ApcMin/+ mouse model of intestinal cancer. J Cancer Res Clin Oncol / 136 / 571-6. 2010
WO 2005/099746
WO 2009/021293
WO 2014/049087
PCT/EP2015/056654

## Claims

1. A method for predicting the probability of survival of a human melanoma patient, wherein the method comprises the steps of:
a) determining the level of hGDF-15 in a human blood sample obtained from said patient; and
b) predicting said probability of survival based on the determined level of hGDF-15 in said human blood sample; wherein a decreased level of hGDF-15 in said human blood sample indicates an increased probability of survival,
wherein the human melanoma patient is a tumor-free stage III melanoma patient or an unresectable stage IV melanoma patient,
wherein step b) comprises comparing said level of hGDF-15 determined in step a) with a hGDF-15 threshold level, wherein said probability is predicted based on the comparison of said level of hGDF-15 determined in step a) with said hGDF-15 threshold level; and wherein a level of hGDF-15 in said human blood sample which is decreased compared to said hGDF-15 threshold level indicates that the probability of survival is increased compared to a probability of survival at or above said hGDF-15 threshold level,
wherein the human blood sample is a human serum sample, and
wherein the hGDF-15 threshold level is a level selected from the range of between 1.1 ng/ml and 2.2 ng/ml.

2. The method of claim 1, wherein the hGDF-15 threshold level is a hGDF-15 level selected from the range of between 1.2 ng/ml and 2.0 ng/ml.

3. The method of claim 1, wherein the hGDF-15 threshold level is a hGDF-15 level selected from the range of between 1.3 ng/ml and 1.8 ng/ml.

4. The method of claim 1, wherein the hGDF-15 threshold level is a hGDF-15 level selected from the range of between 1.4 ng/ml and 1.6 ng/ml.

5. The method of claim 1, wherein the hGDF-15 threshold level is 1.5 ng/ml.

6. The method according to any one of the preceding claims,
wherein step a) further comprises determining the level of lactate dehydrogenase in said human blood sample, and
wherein in step b), said probability of survival is also predicted based on the determined level of lactate dehydrogenase in said human blood sample; and wherein a decreased level of lactate dehydrogenase in said human blood sample indicates an increased probability of survival;
wherein optionally, step b) comprises comparing said level of lactate dehydrogenase determined in step a) with a lactate dehydrogenase threshold level, wherein said probability is also predicted based on the comparison of said level of lactate dehydrogenase determined in step a) with said lactate dehydrogenase threshold level; and wherein a level of lactate dehydrogenase in said human blood sample which is decreased compared to said lactate dehydrogenase threshold level or is at said lactate dehydrogenase threshold level indicates that the probability of survival is increased compared to a probability of survival above said lactate dehydrogenase threshold level;
wherein optionally, step a) further comprises determining the level of S100B in said human blood sample, and wherein in step b), said probability of survival is also predicted based on the determined level of S100B in said human blood sample; and wherein a decreased level of S100B in said human blood sample indicates an increased probability of survival,
preferably wherein step b) comprises comparing said level of S100B determined in step a) with a S100B threshold level, wherein said probability is predicted based on the comparison of said level of S100B determined in step a) with said S100B threshold level; and wherein a level of S100B in said human blood sample which is decreased compared to said S100B threshold level or is at said S100B threshold level indicates that the probability of survival is increased compared to a probability of survival above said S100B threshold level.

7. The method according to any of the preceding claims, wherein in step b), said probability of survival is also predicted based on the age of said patient; and wherein an increased age indicates a decreased probability of survival,
preferably wherein step b) comprises comparing said age of said patient to a threshold age, wherein said probability is predicted based on the comparison of said age of said patient with said threshold age; and wherein an age of said patient which is equal to or increased compared to said threshold age indicates that the probability of survival is decreased compared to a probability of survival below said threshold age,
more preferably wherein said threshold age is selected from the range of 60 to 65 years,
most preferably wherein said threshold age is 63 years.

8. The method according to any one of the preceding claims, wherein in step b), said probability of survival is also predicted based on metastasis; and wherein the presence of metastases in visceral organs other than lung indicates that the probability of survival is decreased as compared to the probability of survival when metastases are absent from visceral organs other than lung.

9. The method according to any of the preceding claims, wherein step a) comprises determining the level of hGDF-15 by using one or more antibodies capable of binding to hGDF-15 or an antigen-binding portion thereof,
preferably wherein the one or more antibodies capable of binding to hGDF-15 or the antigen-binding portion thereof form a complex with hGDF-15,
and/or wherein the one or more antibodies comprise at least one polyclonal antibody,
and/or wherein the one or more antibodies or the antigen-binding portion comprise at least one monoclonal antibody or an antigen-binding portion thereof, and wherein the binding is preferably binding to a conformational or discontinuous epitope on hGDF-15, and wherein the conformational or discontinuous epitope is comprised by the amino acid sequences of SEQ ID No: 25 and SEQ ID No: 26, and wherein preferably, the antibody or antigen-binding portion thereof comprises a heavy chain variable domain which comprises a CDR1 region comprising the amino acid sequence of SEQ ID NO: 3, a CDR2 region comprising the amino acid sequence of SEQ ID NO: 4 and a CDR3 region comprising the amino acid sequence of SEQ ID NO: 5, and the antibody or antigen-binding portion thereof comprises a light chain variable domain which comprises a CDR1 region comprising the amino acid sequence of SEQ ID NO: 6, a CDR2 region comprising the amino acid sequence ser-ala-ser and a CDR3 region comprising the amino acid sequence of SEQ ID NO: 7.

10. The method according to any one of claims 1 to 9, wherein in step a), the level of hGDF-15 is determined by capturing hGDF-15 with a monoclonal antibody or antigen-binding fragment thereof according to claim 9 and by detecting hGDF-15 with a polyclonal antibody, or by detecting hGDF-15 with a monoclonal antibody or antigen-binding fragment thereof which binds to a different epitope than the antibody which captures hGDF-15.

11. The method according to any one of the preceding claims, wherein the method is an *in vitro* method.

12. The method according to any one of the preceding claims,
wherein
(i) in step a), the level of hGDF-15 in the human blood sample is determined by an enzyme linked immunosorbent assay, or
(ii) in step a), the level of hGDF-15 in the human blood sample is determined by an electrochemiluminescence assay, and wherein the electrochemiluminescence assay is preferably a sandwich detection method comprising a step of forming a detection complex between
(A) streptavidin-coated beads;
(B) a biotinylated first antibody or antigen-binding portion thereof capable of binding to hGDF-15;
(C) hGDF-15 from the sample; and
(D) a ruthenium complex-labelled second antibody or antigen-binding portion thereof capable of binding to hGDF-15;
wherein said detection complex has the structure (A)-(B)-(C)-(D), and wherein the biotinylated first antibody or antigen-binding portion thereof binds to a first hGDF-15 epitope and the ruthenium complex-labelled second antibody or antigen-binding portion thereof binds to a second hGDF-15 epitope which is different from said first hGDF-15 epitope,
wherein the method further comprises a step of detecting the detection complex by measuring electrochemiluminescence,
and wherein the level of hGDF-15 in the human blood sample is determined based on the electrochemiluminescence measurement.

13. An apparatus configured to perform the method according to any one of claims 1-12, wherein the apparatus is an electrochemiluminescence analyzer configured to perform the method according to claim 12 wherein in step a), the level of hGDF-15 in the human blood sample is determined by an electrochemiluminescence assay.

14. Use of a detection kit in a method according to any one of claims 1-12, the detection kit comprising:
(i) streptavidin-coated beads;
(ii) a biotinylated first antibody or antigen-binding portion thereof capable of binding to hGDF-15;
(iii) recombinant hGDF-15, preferably in form of a buffered solution comprising recombinant hGDF-15, the buffered solution having a pH in the range of 4 to 7;
(iv) a ruthenium complex-labelled second antibody or antigen-binding portion thereof capable of binding to hGDF-15; and optionally
(v) instructions for use, preferably instructions for use in a method according to claims 1-12; and preferably
(vi) a container containing said recombinant hGDF-15, wherein the surface of the container which is in contact with recombinant hGDF-15 is coated with a non-adhesive material,
wherein the biotinylated first antibody or antigen-binding portion thereof is capable of binding to a first hGDF-15 epitope and the ruthenium complex-labelled second antibody or antigen-binding portion thereof is capable of binding to a second hGDF-15 epitope which is different from said first hGDF-15 epitope,
preferably wherein one of the first antibody or antigen-binding portion thereof capable of binding to hGDF-15 and second antibody or antigen-binding portion thereof capable of binding to hGDF-15 is a monoclonal antibody or antigen-binding portion thereof, wherein the binding is binding to a conformational or discontinuous epitope on hGDF-15 which is comprised by the amino acid sequences of SEQ ID No: 25 and SEQ ID No: 26, and wherein preferably,
the antibody or antigen-binding portion thereof comprises a heavy chain variable domain which comprises a CDR1 region comprising the amino acid sequence of SEQ ID NO: 3, a CDR2 region comprising the amino acid sequence of SEQ ID NO: 4 and a CDR3 region comprising the amino acid sequence of SEQ ID NO: 5, and the antibody or antigen-binding portion thereof comprises a light chain variable domain which comprises a CDR1 region comprising the amino acid sequence of SEQ ID NO: 6, a CDR2 region comprising the amino acid sequence ser-ala-ser and a CDR3 region comprising the amino acid sequence of SEQ ID NO: 7.

## Patentansprüche

1. Verfahren zur Vorhersage der Überlebenswahrscheinlichkeit eines menschlichen Melanompatienten, wobei das Verfahren die folgenden Schritte umfasst:
a) Bestimmung des Spiegels von hGDF-15 in einer menschlichen Blutprobe, die von dem Patienten erhalten wurde; und
b) Vorhersage der Überlebenswahrscheinlichkeit auf Grundlage des bestimmten Spiegels von hGDF-15 in der menschlichen Blutprobe; wobei ein verringerter Spiegel von hGDF-15 in der menschlichen Blutprobe eine erhöhte Überlebenswahrscheinlichkeit anzeigt,
wobei der menschliche Melanompatient ein tumorfreies Stadium III-Melanompatient oder ein inoperables Stadium IV-Melanompatient ist,
wobei Schritt b) das Vergleichen des in Schritt a) bestimmten Spiegels von hGDF-15 mit einem hGDF-15-Schwellenwert umfasst, wobei die Wahrscheinlichkeit auf Grundlage des Vergleichs des in Schritt a) bestimmten Spiegels von hGDF-15 mit dem hGDF-15-Schwellenwert vorhergesagt wird; und wobei ein Spiegel von hGDF-15 in der menschlichen Blutprobe, der im Vergleich zu dem hGDF-15-Schwellenwert verringert ist, anzeigt, dass die Überlebenswahrscheinlichkeit im Vergleich zu einer Überlebenswahrscheinlichkeit bei dem oder oberhalb des hGDF-15-Schwellenwerts erhöht ist,
wobei die menschliche Blutprobe eine menschliche Serumprobe ist, und
wobei der hGDF-15-Schwellenwert ein Spiegel ist, ausgewählt aus dem Bereich zwischen 1,1 ng/ml und 2,2 ng/ml.

2. Verfahren gemäß Anspruch 1, wobei der hGDF-15-Schwellenwert ein hGDF-15-Spiegel ist, ausgewählt aus dem Bereich zwischen 1,2 ng/ml und 2,0 ng/ml.

3. Verfahren gemäß Anspruch 1, wobei der hGDF-15-Schwellenwert ein hGDF-15-Spiegel ist, ausgewählt aus dem Bereich zwischen 1,3 ng/ml und 1,8 ng/ml.

4. Verfahren gemäß Anspruch 1, wobei der hGDF-15-Schwellenwert ein hGDF-15-Spiegel ist, ausgewählt aus dem Bereich zwischen 1,4 ng/ml und 1,6 ng/ml.

5. Verfahren gemäß Anspruch 1, wobei der hGDF-15-Schwellenwert 1,5 ng/ml beträgt.

6. Verfahren gemäß irgendeinem der vorherigen Ansprüche, wobei Schritt a) ferner die Bestimmung des Spiegels von Laktatdehydrogenase in der menschlichen Blutprobe umfasst, und
wobei in Schritt b) die Überlebenswahrscheinlichkeit auch auf Grundlage des bestimmten Spiegels von Laktatdehydrogenase in der menschlichen Blutprobe vorhergesagt wird; und wobei ein verringerter Spiegel von Laktatdehydrogenase in der menschlichen Blutprobe eine erhöhte Überlebenswahrscheinlichkeit anzeigt;
wobei optional Schritt b)das Vergleichen des in Schritt a) bestimmten Spiegels von Laktatdehydrogenase mit einem Laktatdehydrogenase-Schwellenwert umfasst, wobei die Wahrscheinlichkeit auch auf Grundlage des Vergleichs des in Schritt a) bestimmten Spiegels von Laktatdehydrogenase mit dem Laktatdehydrogenase-Schwellenwert vorhergesagt wird; und wobei ein Spiegel von Laktatdehydrogenase in der menschlichen Blutprobe, der im Vergleich zu dem Laktatdehydrogenase-Schwellenwert verringert ist oder bei dem Laktatdehydrogenase-Schwellenwert liegt, anzeigt, dass die Überlebenswahrscheinlichkeit im Vergleich zu einer Überlebenswahrscheinlichkeit oberhalb des Laktatdehydrogenase-Schwellenwerts erhöht ist;
wobei optional Schritt a) ferner die Bestimmung des Spiegels von S100B in der menschlichen Blutprobe umfasst, und wobei in Schritt b) die Überlebenswahrscheinlichkeit auch auf Grundlage des bestimmten Spiegels von S100B in der menschlichen Blutprobe vorhergesagt wird; und wobei ein verringerter Spiegel von S100B in der menschlichen Blutprobe eine erhöhte Überlebenswahrscheinlichkeit anzeigt,
wobei vorzugsweise Schritt b) das Vergleichen des in Schritt a) bestimmten Spiegels von S100B mit einem S100B-Schwellenwert umfasst, wobei die Wahrscheinlichkeit auf Grundlage des Vergleichs des in Schritt a) bestimmten Spiegel von S100B mit dem S100B-Schwellenwert vorhergesagt wird; und wobei ein Spiegel von S100B in der menschlichen Blutprobe, der im Vergleich zu dem S100B-Schwellenwert verringert ist oder bei dem S100B-Schwellenwert liegt, anzeigt, dass die Überlebenswahrscheinlichkeit im Vergleich zu einer Überlebenswahrscheinlichkeit oberhalb des S100B-Schwellenwerts erhöht ist.

7. Verfahren gemäß irgendeinem der vorherigen Ansprüche, wobei in Schritt b) die Überlebenswahrscheinlichkeit auch auf Grundlage des Alters des Patienten vorhergesagt wird; und wobei ein erhöhtes Alter eine verringerte Überlebenswahrscheinlichkeit anzeigt,
wobei vorzugsweise Schritt b) das Vergleichen des Alters des Patienten mit einem Schwellenalter umfasst, wobei die Wahrscheinlichkeit auf Grundlage des Vergleichs des Alters des Patienten mit dem Schwellenalter vorhergesagt wird; und wobei ein Alter des Patienten, das gleich oder erhöht ist im Vergleich zu dem Schwellenalter, anzeigt, dass die Überlebenswahrscheinlichkeit im Vergleich zu einer Überlebenswahrscheinlichkeit unterhalb des Schwellenalters verringert ist,
noch bevorzugter wobei das Schwellenalter aus dem Bereich von 60 bis 65 Jahren ausgewählt ist,
am meisten bevorzugt wobei das Schwellenalter 63 Jahre beträgt.

8. Verfahren gemäß irgendeinem der vorherigen Ansprüche, wobei in Schritt b) die Überlebenswahrscheinlichkeit auch auf Grundlage von Metastasierung vorhergesagt wird; und wobei das Vorhandensein von Metastaseierung in anderen viszeralen Organen als der Lunge anzeigt, dass die Überlebenswahrscheinlichkeit im Vergleich zu der Überlebenswahrscheinlichkeit verringert ist, wenn Metastasen in anderen viszeralen Organen als der Lunge abwesend sind.

9. Verfahren gemäß irgendeinem der vorherigen Ansprüche, wobei Schritt a) die Bestimmung des Spiegels von hGDF-15 durch Verwendung eines oder mehrerer Antikörper oder eines Antigen-bindenden Teils davon umfasst, der/die in der Lage ist/sind, an hGDF-15 zu binden,
wobei vorzugsweise der eine oder die mehreren Antikörper oder der Antigen-bindende Teil davon, der/die in der Lage ist/sind, an hGDF-15 zu binden, einen Komplex mit hGDF-15 bildet/bilden,
und/oder wobei der eine oder die mehreren Antikörper mindestens einen polyklonalen Antikörper umfassen,
und/oder wobei der eine oder die mehreren Antikörper oder der Antigen-bindende Teil mindestens einen monoklonalen Antikörper oder einen Antigen-bindenden Teil davon umfasst/umfassen, und wobei die Bindung vorzugsweise eine Bindung an ein konformationelles oder diskontinuierliches Epitop ("*conformational or discontinuous epitope*") auf hGDF-15 ist, und wobei das konformationelle oder diskontinuierliche Epitop von den Aminosäuresequenzen von SEQ ID NO: 25 und SEQ ID NO: 26 umfasst wird, und wobei vorzugsweise der Antikörper oder Antigen-bindende Teil davon eine variable Domäne der schweren Kette umfasst, die eine CDR1-Region, umfassend die Aminosäuresequenz von SEQ ID NO: 3, eine CDR2-Region, umfassend die Aminosäuresequenz von SEQ ID NO: 4, und eine CDR3-Region, umfassend die Aminosäuresequenz von SEQ ID NO: 5, umfasst, und der Antikörper oder Antigen-bindende Teil davon eine variable Domäne der leichten Kette umfasst, die eine CDR1-Region, umfassend die Aminosäuresequenz von SEQ ID NO: 6, eine CDR2-Region, umfassend die Aminosäuresequenz ser-ala-ser, und eine CDR3-Region, umfassend die Aminosäuresequenz von SEQ ID NO: 7, umfasst.

10. Verfahren gemäß irgendeinem der Ansprüche 1 bis 9, wobei in Schritt a) der Spiegel von hGDF-15 durch Einfangen von hGDF-15 mit einem monoklonalen Antikörper oder Antigen-bindenden Fragment davon gemäß Anspruch 9, und durch Detektieren von hGDF-15 mit einem polyklonalen Antikörper, oder durch Detektieren von hGDF-15 mit einem monoklonalen Antikörper oder Antigen-bindenden Fragment davon, der an ein anderes Epitop bindet als der Antikörper, der hGDF-15 einfängt, bestimmt wird.

11. Verfahren gemäß irgendeinem der vorhergehenden Ansprüche, wobei das Verfahren ein In-vitro-Verfahren ist.

12. Das Verfahren gemäß irgendeinem der vorhergehenden Ansprüche, wobei
(i) in Schritt a) der Spiegel von hGDF-15 in der menschlichen Blutprobe durch einen enzymgekoppelten Immunadsorptionstest bestimmt wird, oder
(ii) in Schritt a) der Spiegel von hGDF-15 in der menschlichen Blutprobe durch einen Elektrochemilumineszenz-Test bestimmt wird, und wobei der Elektrochemilumineszenz- Test vorzugsweise ein Sandwich-Detektionsverfahren ist, das einen Schritt der Bildung eines Detektionskomplexes zwischen
(A) Streptavidin-beschichteten Kügelchen;
(B) einem biotinylierten ersten Antikörper oder Antigen-bindenden Teil davon, der in der Lage ist, an hGDF-15 zu binden;
(C) hGDF-15 aus der Probe; und
(D) einem Rutheniumkomplex-markierten zweiten Antikörper oder Antigen-bindenden Teil davon, der in der Lage ist, an hGDF-15 zu binden;
umfasst;
wobei der Detektionskomplex die Struktur (A)-(B)-(C)-(D) aufweist, und wobei der biotinylierte erste Antikörper oder Antigen-bindende Teil davon an ein erstes hGDF-15-Epitop bindet und der Rutheniumkomplex-markierte zweite Antikörper oder Antigen-bindende Teil davon an ein zweites hGDF-15-Epitop bindet, das anders ist als das erste hGDF-15-Epitop,
wobei das Verfahren ferner einen Schritt des Detektierens des Detektionskomplexes durch Messung von Elektrochemilumineszenz umfasst,
und wobei der Spiegel von hGDF-15 in der menschlichen Blutprobe auf Grundlage der Elektrochemilumineszenzmessung bestimmt wird.

13. Vorrichtung, die zur Durchführung des Verfahrens gemäß irgendeinem der Ansprüche 1-12 konfiguriert ist, wobei die Vorrichtung ein Elektrochemilumineszenz-Analysator ist, der zur Durchführung des Verfahrens gemäß Anspruch 12 konfiguriert ist, wobei in Schritt a) der Spiegel von hGDF-15 in der menschlichen Blutprobe durch einen Elektrochemilumineszenz-Test bestimmt wird.

14. Verwendung eines Detektionskits in einem Verfahren gemäß irgendeinem der Ansprüche 1-12, wobei das Detektionskit umfasst:
(i) Streptavidin-beschichtete Kügelchen;
(ii) einen biotinylierten ersten Antikörper oder Antigen-bindenden Teil davon, der in der Lage ist, an hGDF-15 zu binden;
(iii) rekombinantes hGDF-15, vorzugsweise in Form einer gepufferten Lösung, umfassend rekombinantes hGDF- 15, wobei die gepufferte Lösung einen pH-Wert im Bereich von 4 bis 7 aufweist;
(iv) einen Rutheniumkomplex-markierten zweiten Antikörper oder Antigen-bindenden Teil davon, der in der Lage ist, an hGDF-15 zu binden; und gegebenenfalls
(v) Gebrauchsanweisungen, vorzugsweise Gebrauchsanweisungen zur Verwendung in einem Verfahren gemäß den Ansprüchen 1-12; und optional
(vi) einen Behälter, der das rekombinante hGDF-15 enthält, wobei die Oberfläche des Behälters, die in Kontakt mit rekombinantem hGDF-15 ist, mit einem nichthaftenden Material beschichtet ist,
wobei der biotinylierte erste Antikörper oder Antigen-bindende Teil davon in der Lage ist, an ein erstes hGDF-15-Epitop zu binden, und der Rutheniumkomplex-markierte zweite Antikörper oder Antigen-bindende Teil davon in der Lage ist, an ein zweites hGDF-15-Epitop zu binden, das anders ist als das erste hGDF-15-Epitop,
vorzugsweise wobei einer von dem ersten Antikörper oder Antigen-bindenden Teil davon, der in der Lage ist, an hGDF-15 zu binden, und dem zweiten Antikörper oder Antigen-bindenden Teil davon, der in der Lage ist, an hGDF-15 zu binden, ein monoklonaler Antikörper oder Antigen-bindender Teil davon ist, wobei die Bindung eine Bindung an ein konformationelles oder diskontinuierliches Epitop ("*conformational or discontinuous epitope*") auf hGDF-15 ist, das von den Aminosäuresequenzen von SEQ ID NO: 25 und SEQ ID NO: 26 umfasst wird, und wobei vorzugsweise
der Antikörper oder Antigen-bindende Teil davon eine variable Domäne der schweren Kette umfasst, die eine CDR1-Region, umfassend die Aminosäuresequenz von SEQ ID NO: 3, eine CDR2-Region, umfassend die Aminosäuresequenz von SEQ ID NO: 4, und eine CDR3-Region, umfassend die Aminosäuresequenz von SEQ ID NO: 5, umfasst, und der Antikörper oder Antigen-bindende Teil davon eine variable Domäne der leichten Kette umfasst, die eine CDR1-Region, umfassend die Aminosäuresequenz von SEQ ID NO: 6, eine CDR2-Region, umfassend die Aminosäuresequenz ser-ala-ser, und eine CDR3-Region, umfassend die Aminosäuresequenz von SEQ ID NO: 7, umfasst.

## Revendications

1. Procédé de prédiction de la probabilité de survie d'un patient humain atteint d'un mélanome, dans lequel le procédé comprend les étapes consistant à :
a) déterminer le niveau de hGDF-15 dans un échantillon de sang humain obtenu à partir dudit patient ; et
b) prédire ladite probabilité de survie sur la base du niveau déterminé de hGDF-15 dans ledit échantillon de sang humain ; dans lequel un niveau diminué de hGDF-15 dans ledit échantillon de sang humain indique une probabilité de survie augmentée,
dans lequel le patient humain atteint d'un mélanome est un patient atteint d'un mélanome de stade III sans tumeur ou un patient atteint d'un mélanome de stade IV non résécable,
dans lequel l'étape b) comprend une comparaison dudit niveau de hGDF-15 déterminé à l'étape a) à un niveau seuil de hGDF-15, dans lequel ladite probabilité est prédite sur la base de la comparaison dudit niveau de hGDF-15 déterminé à l'étape a) audit niveau seuil de hGDF-15 ; et dans lequel un niveau de hGDF-15 dans ledit échantillon de sang humain qui est diminué par rapport audit niveau seuil de hGDF-15 indique que la probabilité de survie est augmentée par rapport à une probabilité de survie à ou au-dessus dudit niveau seuil de hGDF-15,
dans lequel l'échantillon de sang humain est un échantillon de sérum humain, et
dans lequel le niveau seuil de hGDF-15 est un niveau sélectionné à partir de la plage comprise entre 1,1 ng/ml et 2,2 ng/ml.

2. Procédé selon la revendication 1, dans lequel le niveau seuil de hGDF-15 est un niveau de hGDF-15 sélectionné à partir de la plage comprise entre 1,2 ng/ml et 2,0 ng/ml.

3. Procédé selon la revendication 1, dans lequel le niveau seuil de hGDF-15 est un niveau de hGDF-15 sélectionné à partir de la plage comprise entre 1,3 ng/ml et 1,8 ng/ml.

4. Procédé selon la revendication 1, dans lequel le niveau seuil de hGDF-15 est un niveau de hGDF-15 sélectionné à partir de la plage comprise entre 1,4 ng/ml et 1,6 ng/ml.

5. Procédé selon la revendication 1, dans lequel le niveau seuil de hGDF-15 est de 1,5 ng/ml.

6. Procédé selon l'une quelconque des revendications précédentes,
dans lequel l'étape a) comprend en outre une détermination du niveau de lactate déshydrogénase dans ledit échantillon de sang humain, et
dans lequel à l'étape b), ladite probabilité de survie est également prédite sur la base du niveau déterminé de lactate déshydrogénase dans ledit échantillon de sang humain ; et dans lequel un niveau diminué de lactate déshydrogénase dans ledit échantillon de sang humain indique une probabilité de survie augmentée ;
dans lequel facultativement, l'étape b) comprend une comparaison dudit niveau de lactate déshydrogénase déterminé à l'étape a) à un niveau seuil de lactate déshydrogénase, dans lequel ladite probabilité est également prédite sur la base de la comparaison dudit niveau de lactate déshydrogénase déterminé à l'étape a) audit niveau seuil de lactate déshydrogénase ; et dans lequel un niveau de lactate déshydrogénase dans ledit échantillon de sang humain qui est diminué par rapport audit niveau seuil de lactate déshydrogénase ou est audit niveau seuil de lactate déshydrogénase indique que la probabilité de survie est augmentée par rapport à une probabilité de survie au-dessus dudit niveau seuil de lactate déshydrogénase ;
dans lequel facultativement, l'étape a) comprend en outre une détermination du niveau de S100B dans ledit échantillon de sang humain, et dans lequel à l'étape b), ladite probabilité de survie est également prédite sur la base du niveau déterminé de S100B dans ledit échantillon de sang humain ; et dans lequel un niveau diminué de S100B dans ledit échantillon de sang humain indique une probabilité augmentée de survie,
de préférence dans lequel l'étape b) comprend une comparaison dudit niveau de S100B déterminé à l'étape a) à un niveau seuil de S100B, dans lequel ladite probabilité est prédite sur la base de la comparaison dudit niveau de S100B déterminé à l'étape a) audit niveau seuil de S100B ; et dans lequel un niveau de S100B dans ledit échantillon de sang humain qui est diminué par rapport audit niveau seuil de S100B ou est audit niveau seuil de S100B indique que la probabilité de survie est augmentée par rapport à une probabilité de survie au-dessus dudit niveau seuil de S100B.

7. Procédé selon l'une quelconque des revendications précédentes, dans lequel à l'étape b), ladite probabilité de survie est également prédite sur la base de l'âge dudit patient ; et dans lequel un âge augmenté indique une probabilité de survie diminuée,
de préférence dans lequel l'étape b) comprend une comparaison dudit âge dudit patient à un âge seuil, dans lequel ladite probabilité est prédite sur la base de la comparaison dudit âge dudit patient audit âge seuil ; et dans lequel un âge dudit patient qui est égal ou augmenté par rapport audit âge seuil indique que la probabilité de survie est diminuée par rapport à une probabilité de survie en-dessous dudit âge seuil,
plus préférentiellement dans lequel ledit âge seuil est sélectionné à partir de la plage de 60 à 65 ans, le plus préférentiellement dans lequel ledit âge seuil est de 63 ans.

8. Procédé selon l'une quelconque des revendications précédentes, dans lequel à l'étape b), ladite probabilité de survie est également prédite sur la base d'une métastase ; et dans lequel la présence de métastases dans des organes viscéraux autres que le poumon indique que la probabilité de survie est diminuée par rapport à la probabilité de survie lorsque des métastases sont absentes des organes viscéraux autres que le poumon.

9. Procédé selon l'une quelconque des revendications précédentes, dans lequel l'étape a) comprend une détermination du niveau de hGDF-15 en utilisant un ou plusieurs anticorps aptes à se lier au hGDF-15 ou à une partie de liaison à un antigène de celui-ci,
de préférence dans lequel les un ou plusieurs anticorps aptes à se lier au hGDF-15 ou à la partie de liaison à un antigène de celui-ci forment un complexe avec le hGDF-15,
et/ou dans lequel les un ou plusieurs anticorps comprennent au moins un anticorps polyclonal,
et/ou dans lequel les un ou plusieurs anticorps ou la partie de liaison à un antigène comprennent au moins un anticorps monoclonal ou une partie de liaison à un antigène de celui-ci, et dans lequel la liaison est de préférence une liaison à un épitope conformationnel ou discontinu sur le hGDF-15, et dans lequel l'épitope conformationnel ou discontinu est constitué par les séquences d'acides aminés de SEQ ID No: 25 et de SEQ ID No: 26, et dans lequel de préférence, l'anticorps ou la partie de liaison à un antigène de celui-ci comprend un domaine variable à chaîne lourde qui comprend une région CDR1 comprenant la séquence d'acides aminés de SEQ ID NO: 3, une région CDR2 comprenant la séquence d'acides aminés de SEQ ID NO: 4, et une région CDR3 comprenant la séquence d'acides aminés SEQ ID NO: 5, et l'anticorps ou la partie de liaison à un antigène de celui-ci comprend un domaine variable à chaîne légère qui comprend une région CDR1 comprenant la séquence d'acides aminés de SEQ ID NO: 6, une région CDR2 comprenant la séquence d'acides aminés ser-ala-ser et une région CDR3 comprenant la séquence d'acides aminés SEQ ID NO: 7.

10. Procédé selon l'une quelconque des revendications 1 à 9, dans lequel à l'étape a), le niveau de hGDF-15 est déterminé en capturant le hGDF-15 avec un anticorps monoclonal ou un fragment de liaison à un antigène de celui-ci selon la revendication 9 et en détectant le hGDF-15 avec un anticorps polyclonal, ou en détectant le hGDF-15 avec un anticorps monoclonal ou un fragment de liaison à un antigène de celui-ci qui se lie à un épitope différent de l'anticorps qui capture le hGDF-15.

11. Procédé selon l'une quelconque des revendications précédentes, dans lequel le procédé est un procédé *in vitro.*

12. Procédé selon l'une quelconque des revendications précédentes,
dans lequel
(i) à l'étape a), le niveau de hGDF-15 dans l'échantillon de sang humain est déterminé par une analyse par immuno-absorption enzymatique, ou
(ii) à l'étape a), le niveau de hGDF-15 dans l'échantillon de sang humain est déterminé par une analyse d'électrochimiluminescence, et dans lequel l'analyse d'électrochimiluminescence est de préférence un procédé de détection en sandwich comprenant une étape consistant à former un complexe de détection entre
(A) des perles enrobées de streptavidine ;
(B) un premier anticorps biotinylé ou une partie de liaison à un antigène de celui-ci apte à se lier au hGDF-15 ;
(C) du hGDF-15 issu de l'échantillon ; et
(D) un deuxième anticorps étiqueté complexe de ruthénium ou une partie de liaison à un antigène de celui-ci apte à se lier au hGDF-15 ;
dans lequel ledit complexe de détection présente la structure (A)-(B)-(C)-(D), et dans lequel le premier anticorps biotinylé ou la partie de liaison à un antigène de celui-ci se lie à un premier épitope de hGDF-15 et le deuxième anticorps étiqueté complexe de ruthénium ou la partie de liaison à un antigène de celui-ci se lie à un deuxième épitope de hGDF-15 qui est différent dudit premier épitope de hGDF-15, dans lequel le procédé comprend en outre une étape consistant à détecter le complexe de détection en mesurant l'électrochimiluminescence,
et dans lequel le niveau de hGDF-15 dans l'échantillon de sang humain est déterminé sur la base de la mesure d'électrochimiluminescence.

13. Appareil configuré pour mettre en oeuvre le procédé selon l'une quelconque des revendications 1 à 12, dans lequel l'appareil est un analyseur d'électrochimiluminescence configuré pour mettre en oeuvre le procédé selon la revendication 12 dans lequel à l'étape a), le niveau de hGDF-15 dans l'échantillon de sang humain est déterminé par une analyse d'électrochimiluminescence.

14. Utilisation d'un kit de détection dans un procédé selon l'une quelconque des revendications 1 à 12, le kit de détection comprenant :
(i) des perles enrobées de streptavidine ;
(ii) un premier anticorps biotinylé ou une partie de liaison à un antigène de celui-ci apte à se lier au hGDF-15 ;
(iii) un hGDF-15 recombinant, de préférence sous forme d'une solution tamponnée comprenant du hGDF-15 recombinant, la solution tamponnée présentant un pH dans la plage de 4 à 7 ;
(iv) un deuxième anticorps étiqueté complexe de ruthénium ou une partie de liaison à un antigène de celui-ci apte à se lier au hGDF-15 ; et facultativement
(v) des instructions d'utilisation, de préférence des instructions pour utilisation dans un procédé selon les revendications 1 à 12 ; et de préférence
(vi) un récipient contenant ledit hGDF-15 recombinant, dans lequel la surface du récipient qui est en contact avec le hGDF-15 recombinant est enrobée d'un matériau non adhésif,
dans lequel le premier anticorps biotinylé ou la partie de liaison à un antigène de celui-ci est apte à se lier à un premier épitope de hGDF-15 et le deuxième anticorps étiqueté complexe de ruthénium ou la partie de liaison à un antigène de celui-ci est apte à se lier à un deuxième épitope de hGDF-15 qui est différent dudit premier épitope de hGDF-15,
de préférence dans lequel l'un parmi le premier anticorps ou la partie de liaison à un antigène de celui-ci apte à se lier au hGDF-15 et le deuxième anticorps ou la partie de liaison à un antigène de celui-ci apte à se lier au hGDF-15 est un anticorps monoclonal ou une partie de liaison à un antigène de celui-ci, dans lequel la liaison est une liaison à un épitope conformationnel ou discontinu sur le hGDF-15 qui est constitué par la séquence d'acides aminés de SEQ ID No: 25 et de SEQ ID No: 26, et dans lequel de préférence,
l'anticorps ou la partie de liaison à un antigène de celui-ci comprend un domaine variable à chaîne lourde qui comprend une région CDR1 comprenant la séquence d'acides aminés de SEQ ID NO: 3, une région CDR2 comprenant la séquence d'acides aminés de SEQ ID NO: 4, et une région CDR3 comprenant la séquence d'acides aminés SEQ ID NO: 5, et l'anticorps ou la partie de liaison à un antigène de celui-ci comprend un domaine variable à chaîne légère qui comprend une région CDR1 comprenant la séquence d'acides aminés de SEQ ID NO: 6, une région CDR2 comprenant la séquence d'acides aminés ser-ala-ser, et une région CDR3 comprenant la séquence d'acides aminés SEQ ID NO: 7.
